# EUROPEAN PATENT SPECIFICATION

(11) **EP 1 960 546 B1**
(45) Date of publication and mention of the grant of the patent: **19.10.2011**
(21) Application number: 06840081.1
(22) Date of filing: 30.11.2006
(51) Int. Cl.: C12Q 1/68

(54) **METHODS OF IDENTIFYING AND TREATING INDIVIDUALS EXHIBITING COMPLEX KARYOTYPES**
VERFAHREN ZUR IDENTIFIZIERUNG UND BEHANDLUNG VON KOMPLEXE KARYOTYPEN ZEIGENDEN INDIVIDUEN
METHODES D'IDENTIFICATION ET DE TRAITEMENT D'INDIVIDUS PRESENTANT DES KARYOTYPES COMPLEXES

(30) Priority: 01.12.2005 US 741280 P
(43) Date of publication of application: 27.08.2008
(73) Proprietor: Bristol-Myers Squibb Company, Princeton, NJ 08543-4000 (US)
(72) Inventor: SAWYERS, Charles, Los Angeles, CA 90095 (US); LEE, Francis, Y., Princeton, NJ 08543 (US); SHAH, Neil, Pravin, Los Angeles, CA 91367 (US)
(74) Representative: Reitstötter - Kinzebach
(86) International application number: PCT/US2006/061399
(87) International publication number: WO 2007/065124

(56) References cited:
- WO-A-2006/074149
- WO-A-2007/014250
- MANLEY ET AL: "Advances in the structural biology, design and clinical development of Bcr-Abl kinase inhibitors for the treatment of chronic myeloid leukaemia" BIOCHIMICA ET BIOPHYSICA ACTA (BBA) - PROTEINS & PROTEOMICS, ELSEVIER, vol. 1754, no. 1-2, 8 September 2005 (2005-09-08), pages 3-13, XP005214189 ISSN: 1570-9639
- CORBIN AMIE S ET AL: "Analysis of the structural basis of specificity of inhibition of the Abl kinase by STI571" JOURNAL OF BIOLOGICAL CHEMISTRY, vol. 277, no. 35, 30 August 2002 (2002-08-30), pages 32214-32219, XP002429304 ISSN: 0021-9258
- DATABASE BIOSIS [Online] BIOSCIENCES INFORMATION SERVICE, PHILADELPHIA, PA, US; November 2005 (2005-11), JABBOUR ELIAS ET AL: "Outcome of salvage therapy in patients (pts) with chronic myeloid leukemia (CML) who failed imatinib after developing BCR-ABL kinase mutation." XP002431032 Database accession no. PREV200600183462 & BLOOD, vol. 106, no. 11, Part 1, November 2005 (2005-11), page 318A, 47TH ANNUAL MEETING OF THE AMERICAN-SOCIETY-OF-HEMATOLOGY; ATLANTA, GA, USA; DECEMBER 10 -13, 2005 ISSN: 0006-4971
- DATABASE BIOSIS [Online] BIOSCIENCES INFORMATION SERVICE, PHILADELPHIA, PA, US; 16 November 2000 (2000-11-16), CORBIN AMIE S ET AL: "Analysis of the structural basis of specificity of inhibition of the Abl kinase by STI571" XP002431033 Database accession no. PREV200100330629 & BLOOD, vol. 96, no. 11 Part 1, 16 November 2000 (2000-11-16), page 470a, 42ND ANNUAL MEETING OF THE AMERICAN SOCIETY OF HEMATOLOGY; SAN FRANCISCO, CALIFORNIA, USA; DECEMBER 01-05, 2000 ISSN: 0006-4971
- TALPAZ M ET AL: "Hematologic and cytogenetic responses in imatinib-resistant accelerated and blast phase chronic myeloid leukemia (CML) patients treated with the dual SRC/ABL kinase inhibitor BMS-354825: Results from a phase I dose escalation study" BLOOD, W.B.SAUNDERS COMPANY, ORLANDO, FL, US, vol. 104, no. 11, Part 1, November 2004 (2004-11), page 10A, XP002402758 ISSN: 0006-4971
- DATABASE CLINICALTRIALS [Online] clinical trials; June 2005 (2005-06), "Advanced Chronic Myelogenous Leukemia (CML) - Follow On: Study of BMS-354825 in Subjects with CML" XP002431034 retrieved from WWW.CLINICALTRIALS.GOV Database accession no. NTC00123487
- DATABASE BIOSIS [Online] BIOSCIENCES INFORMATION SERVICE, PHILADELPHIA, PA, US; November 2004 (2004-11), CORTES JORGE ET AL: "Phase I study of lonafarnib (SCH66336) in combination with imatinib for patients (pts) with chronic myeloid leukemia (CML) after failure to imatinib." XP002431035 Database accession no. PREV200510268981 & BLOOD, vol. 104, no. 11, Part 1, November 2004 (2004-11), page 288A, 46TH ANNUAL MEETING OF THE AMERICAN-SOCIETY-OF-HEMATOLOGY; SAN DIEGO, CA, USA; DECEMBER 04 -07, 2004 ISSN: 0006-4971
- BRADEEN HEATHER A ET AL: "Comparison of imatinib, mesylate, dasatinib (BMS-354825), and nilotinib (AMN107) in an N-ethyl-N-nitrosourea (ENU)-based mutagenesis screen: high efficacy of drug combinations" BLOOD, vol. 108, no. 7, October 2006 (2006-10), pages 2332-2338, XP009082064 ISSN: 0006-4971
- NICOLINI F E ET AL: "Mutation status and clinical outcome of 89 imatinib mesylate-resistant chronic myelogenous leukemia patients: a retrospective analysis from the French intergroup of CML (Fi(phi)-LMC GROUP)" LEUKEMIA (BASINGSTOKE), vol. 20, no. 6, June 2006 (2006-06), pages 1061-1066, XP002429305 ISSN: 0887-6924
- GIEHL ET AL: "Centrosome aberrations in chronic myeloid leukemia correlate with stage of diesase and chromosomal instability", LEUKEMIA, vol. 19, 2005, pages 1192-1197,
- OUDAT ET AL: "A unique, complex variant philadelphia chromosome translocation in a patient with typical chronic myelogenous leukemia", ARCH PATHOL LAB MED, vol. 125, 2001, pages 47-49,
- HELLER ET AL: "Breakpoint differentiation in chromosomal aberrations of hematological malignancies: identiication of 33 previous unrecorded breakpoints", INTERNATIONAL JOURNAL OF ONCOLOGY, vol. 24, 2004, pages 127-136,
- ESPINOZA ET AL: "A complex translocation (9;22;16)(q34;q11.2;p13) in chronic myelocytic leukemia", CANCER GENETICS AND CYTOGENETICS, vol. 157, 2005, pages 175-177,
- GIEHL ET AL: "Centrosome aberrations in chronic myeloid leukemia correlate with stage of diesase and chromosomal instability" LEUKEMIA, vol. 19, 2005, pages 1192-1197,
- OUDAT ET AL: "A unique, complex variant philadelphia chromosome translocation in a patient with typical chronic myelogenous leukemia" ARCH PATHOL LAB MED, vol. 125, 2001, pages 47-49,
- HELLER ET AL: "Breakpoint differentiation in chromosomal aberrations of hematological malignancies: identiication of 33 previous unrecorded breakpoints" INTERNATIONAL JOURNAL OF ONCOLOGY, vol. 24, 2004, pages 127-136,
- ESPINOZA ET AL: "A complex translocation (9;22;16)(q34;q11.2;p13) in chronic myelocytic leukemia" CANCER GENETICS AND CYTOGENETICS, vol. 157, 2005, pages 175-177,

## Description

### FIELD OF THE INVENTION

The invention described herein relates to diagnostic methods and compositions useful in the management of disorders, for example cancers, involving cells that harbor complex karyotypes.

### BACKGROUND OF THE INVENTION

The advanced stages of chronic myeloid leukemia (CML) - the accelerated phase (AP) and blast crisis (BC) - are the most refractory to available treatment options. BC patients fall into two categories: those with myeloid blast crisis (MBC) and those with lymphoid blast crisis (LBC) (1). The constitutively activated tyrosine kinase BCR-ABL remains central to the underlying pathophysiology of the disease in advanced CML, and in Philadelphia-chromosome positive acute lymphoid leukemia (Ph+ ALL). Imatinib has substantial clinical activity in advanced phase CML patients and those with Ph+ ALL; however, responses are often short-lived (2-5). At 4 years of follow-up, 75% of AP CML patients and 95% of BC patients developed resistance to imatinib (6). Similar to chronic phase CML, mutations in *BCR-ABL* are the most common reason for this treatment resistance. Other mechanisms, such as *BCR-ABL* gene amplification and activation of SRC kinases have also been implicated (7-10).

Dasatinib is a novel, orally active kinase inhibitor, which targets several oncogenic pathways, including BCR-ABL and SRC family kinases (11). Dasatinib is several hundred-fold more potent than imatinib in cells that express wild-type BCR-ABL and is active against all but one of the known imatinib-resistant BCR-ABL mutations (11-13). Phase I clinical studies comprising treatment of CML patients with dasatinib are described in Manley et al., BIOCHIMICA ET BIOPHYSICA ACTA (BBA) - PROTEINS & PROTEOMICS, vol. 1754, 3-13; Talpaz et al., BLOOD, vol. 104, no. 11, Part 1, 2004, page 10A; and DATABASE CLINICAL TRIALS [Online] clinical trials; June 2005 (2005-06), "Advanced Chronic Myelogenous Leukemia (CML) - Follow On: Study of BMS-354825 in Subjects with CML" XP002431034 retrieved from WWW.CLINICALTRIALS.GOV Database accession no. NTC00123487. Additionally, an *in vitro* mutagenesis study demonstrated that, compared with imatinib, a much more limited spectrum of *BCR-ABL* mutations is capable of conferring resistance to dasatinib (14), likely due to the less stringent conformational requirements for dasatinib versus imatinib binding to BCR-ABL (15, 16). Dasatinib is also highly active against the SRC family kinase LYN which has been found to be overexpressed and activated in some leukemic cell lines rendered resistant to imatinib (8). These data suggest that dasatinib could circumvent multiple mechanisms of imatinib resistance, and therefore may be of particular value in such patients with advanced CML and Ph+ ALL.

The Philadelphia chromosome is a specific genetic, chromosomal abnormality that is associated with chronic myelogenous leukemia (CML) and involves a chromosomal translocation between chromosomes 9 and 22 which is readily observable in the patients karyotype. The translocation that gives rise to the Philadelphia chromosome also results in the formation of the constitutively active tyrosine kinase BCR-ABL. 95% of patients with CML show this abnormality, with the remainder harboring either a cryptic translocation that is invisible on G-banded chromosome preparations or a more complex translocation involving another chromosome or chromosomes as well as chromosomes 9 and 22. 25-30% of patients with acute lymphoblastic leukemia (ALL) also are Ph chromosome positive. Patients having additional chromosomal aberrations aside from merely the Philadelphia chromosome are considered to have a "complex" karyotype.

The inventors describe for the first time herein data demonstrating that patients with complex karyotypes have an increased likelihood of being at least partially resistant to a protein tyrosine kinase inhibitor. Accordingly, there is a need for diagnostic and therapeutic procedures and compositions tailored to identify and address such resistance. Particularly there is a need for a treatment for cancer, mastocytosis and related disorders involving patients harboring a complex karyotype. The invention provided herein satisfies this need.

### SUMMARY OF THE INVENTION

According to the present invention, a complex karyotyp is a karyotype that includes chromosomal abnormalities in addition to the presence of a Philadelphia chromosome.

The present invention relates to a method for determining the responsiveness of an individual with a BCR-ABL associated disorder to treatment with N-(2-chloro-6-methylphenyl)-2-[[6-[4-(2-hydroxyethyl)-1-piperazinyl]-2-methyl-4-pyrimidinyl]amino]-5-thiazolecarboxamide, or a pharmaceutically acceptable salt, solvate or hydrate thereof, comprising screening a biological sample from said individual for the presence of a complex karyotype; wherein the presence of said karyotype is indicative of the individual being at least partially resistant to N-(2-chloro-6-methylphenyl)-2-[[6-[4-(2-hydroxyethyl)-1-piperazinyl]-2-methyl-4-pyrimidinyl]amino]-5-thiazolecarboxamide or a pharmaceutically acceptable salt, solvate or hydrate thereof, therapy.

The present invention further relates to a method of determining a treatment regimen for an individual suffering from a BCR-ABL-associated disorder comprising:
determining whether a biological sample obtained from the individual has a complex karyotype, wherein the presence of said karyotype is indicative of the patient being at least partially resistant to N-(2-chloro-6-methylphenyl)-2-[[6-[4-(2-hydroxyethyl)-1-piperazinyl]-2-methyl-4-pyrimidinyl] amino]-5-thiazolecarboxamide, or a pharmaceutically acceptable salt, solvate or hydrate thereof, therapy; and
recommending the administering a therapeutically effective amount of N-(2-chloro-6-methylphenyl)-2-[[6-[4-(2-hydroxyethyl)- I -piperazinyl]-2-methyl-4-pyrimidinyl] amino]-5-thiazolecarboxamide, or a pharmaceutically acceptable salt, solvate or hydrate thereof, to the individual.

The present invention also relates to a kit for use in treating an individual with a BCR-ABL associated disorder, comprising:
a means for determining whether a biological sample obtained from said individual has a complex karyotype; and
a therapeutically effective amount of N-(2-chloro-6-methylphenyl)-2-[[6-[4-(2-hydroxyethyl)-1-piperazinyl]-2-methyl-4-pyrimidinyl]amino]-5-thiazolecarboxamide, or a pharmaceutically acceptable salt, solvate or hydrate thereof.

The present invention further relates to the use of a kit in determining a treatment strategy for an individual with a BCR-ABL-associated disorder, the kit comprising:
a means for determining whether a biological sample obtained from said individual has a complex karyotype,
wherein said treatment strategy comprises administration of a therapeutically effective amount of N-(2-chloro-6-methylphenyl)-2-[[6-[4-(2-hydroxyethyl)-1-piperazinyl]-2-methyl-4-pyrimidinyl]amino]-5-thiazolccarboxamide, or a pharmaceutically acceptable salt, solvate or hydrate thereof

The present invention provides a method of screening a biological sample, for example cells that do not respond, or that have stopped responding, or that have a diminished response, to kinase inhibitors used to inhibit proliferation of said cells. For example, the present invention provides a method of screening cells from an individual suffering from cancer who either imatinib naïve or is being treated with imatinib, and whose cells do not respond or have stopped responding or that have a diminished response to imatinib, for the presence of a complex chromosome karyotype, wherein the presence of said complex chromosome karyotype, either alone or in conjunction with the detection of a protein tyrosine kinase inhibitor resistant BCR-ABL mutation, is indicative of said individual requiring administration of either a therapeutically acceptable amount of dasatinib and/or a more aggressive dosing regimen of a therapeutically acceptable amount of dasatinib, alone or in combination with other agents to inhibit proliferation of said cells.

The present invention also provides a method of screening a biological sample, for example cells that harbor a complex chromosome karyotype, whereby the presence of a complex chromosome karyotype is indicative of said individual requiring administration of either a therapeutically acceptable amount of dasatinib and/or administration of a more aggressive dosing regimen of a therapeutically acceptable amount of dasatinib, alone or in combination with other agents to inhibit proliferation of said cells, wherein said other agent is imatinib.

The present disclosure also provides a method of screening a biological sample, for example cells that harbor a complex chromosome karyotype, whereby the presence of a complex chromosome karyotype is indicative of said individual requiring administration of either a therapeutically acceptable amount of dasatinib and/or administration of a more aggressive dosing regimen of a therapeutically acceptable amount of dasatinib, alone or in combination with other agents to inhibit proliferation of said cells, wherein said other agent is a farnysyl transferase inhibitor and/or Rab-GGTase inhibitor.

The present invention also provides a method of screening a biological sample, for example cells that harbor a complex chromosome karyotype, whereby the presence of a complex chromosome karyotype is indicative of said individual requiring administration of either a therapeutically acceptable amount of dasatinib and/or administration of a more aggressive dosing regimen of a therapeutically acceptable amount of dasatinib, alone or in combination with other agents to inhibit proliferation of said cells, wherein said other agent is (R)-2,3,4,5-tetrahydro-1-(1H-imidazol-4-ylmethyl)-3-(phenylmethyl)-4-(2-thienylsulfonyl)-1H-1,4-benzodiazepine-7-carbonitrile, hydrochloride salt.

The present invention also provides a method of screening a biological sample, for example cells that harbor a complex chromosome karyotype, whereby the presence of a complex chromosome karyotype is indicative of said individual requiring administration of either a therapeutically acceptable amount of dasatinib and/or administration of a more aggressive dosing regimen of a therapeutically acceptable amount of dasatinib, alone or in combination with other agents to inhibit proliferation of said cells, wherein said other agent is a protein tyrosine kinase inhibitor.

The present invention also provides a method of screening a biological sample, for example cells that harbor a complex chromosome karyotype, whereby the presence of a complex chromosome karyotype is indicative of said individual requiring administration of either a therapeutically acceptable amount of dasatinib and/or administration of a more aggressive dosing regimen of a therapeutically acceptable amount of dasatinib, alone or in combination with other agents to inhibit proliferation of said cells, wherein said other agent is imatinib.

The present invention also provides a method of screening a biological sample, for example cells that harbor a complex chromosome karyotype, whereby the presence of a complex chromosome karyotype is indicative of said individual requiring administration of either a therapeutically acceptable amount of dasatinib and/or administration of a more aggressive dosing regimen of a therapeutically acceptable amount of dasatinib, alone or in combination with other agents to inhibit proliferation of said cells, wherein said other agent is an mTOR inhibitor.

The present invention also provides a method of screening a biological sample, for example cells that harbor a complex chromosome karyotype, whereby the presence of a complex chromosome karyotype is indicative of said individual requiring administration of either a therapeutically acceptable amount of dasatinib and/or administration of a more aggressive dosing regimen of a therapeutically acceptable amount of dasatinib, alone or in combination with other agents to inhibit proliferation of said cells, wherein said other agent is rapamycin.

The present invention also provides a method of screening a biological sample, for example cells that harbor a complex chromosome karyotype, whereby the presence of a complex chromosome karyotype is indicative of said individual requiring administration of either a therapeutically acceptable amount of dasatinib and/or administration of a more aggressive dosing regimen of a therapeutically acceptable amount of dasatinib, alone or in combination with other agents to inhibit proliferation of said cells, wherein said other agent is a tubulin stabilizing agent.

The present invention also provides a method of screening a biological sample, for example cells that harbor a complex chromosome karyotype, whereby the presence of a complex chromosome karyotype is indicative of said individual requiring administration of either a therapeutically acceptable amount of dasatinib and/or administration of a more aggressive dosing regimen of a therapeutically acceptable amount of dasatinib, alone or in combination with other agents to inhibit proliferation of said cells, wherein said other agent is pacitaxol.

The present invention also provides a method of screening a biological sample, for example cells that harbor a complex chromosome karyotype, whereby the presence of a complex chromosome karyotype is indicative of said individual requiring administration of either a therapeutically acceptable amount of dasatinib and/or administration of a more aggressive dosing regimen of a therapeutically acceptable amount of dasatinib, alone or in combination with other agents to inhibit proliferation of said cells, wherein said other agent is an epothilone.

The present invention also provides a method of screening a biological sample, for example cells that harbor a complex chromosome karyotype, whereby the presence of a complex chromosome karyotype is indicative of said individual requiring administration of either a therapeutically acceptable amount of dasatinib and/or administration of a more aggressive dosing regimen of a therapeutically acceptable amount of dasatinib, alone or in combination with other agents to inhibit proliferation of said cells, wherein said other agent is a taxane.

The present invention also provides a method of screening a biological sample, for example cells harbor a complex chromosome karyotype, whereby the presence of said complex chromosome karyotype is indicative that a patient requires administration of either a therapeutically acceptable amount of dasatinib and/or a more aggressive dosing regimen, or increased dosing frequency, of a therapeutically acceptable amount of dasatinib in order to inhibit proliferation of said cells, wherein said increased level is 10, 20, 30, 40, 50, 60, 70, 80, 90, or 95% more than the prescribed dasatinib dose, or 1.5x, 2x, 2.5x, 3x, 3.5x, 4x, 4.5x, or 5x more dasatinib than the prescribed dose, and alternatively wherein said increased dosing frequency is in combination with another agent.

The present invention also provides a method of screening a biological sample, for example cells harbor a complex chromosome karyotype, whereby the presence of said complex chromosome karyotype is indicative that a patient requires administration of either a therapeutically acceptable amount of dasatinib and/or more aggressive dosing regimen, or increased dosing frequency, of a therapeutically acceptable amount of dasatinib in order to inhibit proliferation of said cells, wherein said increased level is 10, 20, 30, 40, 50, 60, 70, 80, 90, or 95% more than the prescribed dasatinib dose, or 1.5x, 2x, 2.5x, 3x, 3.5x, 4x, 4.5x, or 5x more dasatinib than the prescribed dose, and alternatively wherein said increased dosing frequency is in combination with another agent, wherein said agent is imatinib; a tubulin stabilizing agent (e.g., pacitaxol, epothilone, taxane, etc.); a farnysyl transferase inhibitor (e.g., (R)-2,3,4,5-tetrahydro-1-(1H-imidazol-4-ylmethyl)-3-(phenylmethyl)-4-(2-thienylsulfonyl)-1H-1,4-benzodiazepine-7-carbonitrile, hydrochloride salt); another protein tyrosine kinase inhibitor, especially a BCR-ABL inhibitor such as imatinib as indicated herein, or AMN107; or any other combination or dosing regimen comprising *N*-(2-chloro-6-methylphenyl)-2-((6-(4-(2-hydroxyethyl)-1-piperazinyl)-2-methyl-4-pyrimidinyl)amino)-1,3-thiazole-5-carboxamide disclosed herein. Additional combinations comprising *N*-(2-chloro-6-methylphenyl)-2-((6-(4-(2-hydroxyethyl)-1-piperazinyl)-2-methyl-4-pyrimidinyl)amino)-1,3-thiazole-5-carboxamide that may be useful to practice the methods of the present invention.

The present invention also describes a method of treating an individual suffering from a BCR-ABL-associated disorder comprising the step of determining whether a biological sample obtained from the individual has a complex karyotype, wherein the presence of said karyotype is indicative of the patient being at least partially resistant to N-(2-chloro-6-methylphenyl)-2-[[6-[4-(2-hydroxyethyl)-1-piperazinyl]-2-methyl-4-pyrimidinyl] amino]-5-thiazolecarboxamide, or a pharmaceutically acceptable salt, solvate, or hydrate thereof, therapy; and administering a therapeutically effective amount of N-(2-chloro-6-methylphenyl)-2-[[6-[4-(2-hydroxyethyl)-1-piperazinyl]-2-methyl-4-pyrimidinyl]amino]-5-thiazolecarboxamide, or a pharmaceutically acceptable salt, solvate, or hydrate thereof, to the individual; or wherein said thiazolecarboxamide is administered at a higher dosage or dosing frequency if it is determined that the biological sample has a complex karyotype; or wherein said thiazolecarboxamide is administered in combination with another compound disclosed herein.

The present invention also provides a kit for use in determining a treatment strategy for an individual with a BCR-ABL-associated disorder, comprising a means for determining whether a biological sample obtained from said individual has a complex karyotype; and optionally instructions for use and interpretation of the kit results, wherein said treatment strategy comprises administration of a therapeutically effective amount of N-(2-chloro-6-methylphenyl)-2-[[6-[4-(2-hydroxyethyl)-1-piperazinyl]-2-methyl-4-pyrimidinyl]amino]-5-thiazolecarboxamide, or a pharmaceutically acceptable salt, hydrate or solvate thereof.

The present inventors have discovered that mutations to the BCR-ABL polypeptide appear in certain individuals treated with N-(2-chloro-6-methylphenyl)-2-[[6-[4-(2-hydroxyethyl)-1-piperazinyl]-2-methyl-4-pyrimidinyl]amino]-5-thiazolecarboxamide and that these mutations can render the polypeptide at least partially resistant to therapy with N-(2-chloro-6-methylphenyl)-2-[[6-[4-(2-hydroxyethyl)-1-piperazinyl]-2-methyl-4-pyrimidinyl] amino]-5-thiazolecarboxamide.

The present invention describes methods of identifying subjects that have mutant BCR-ABL polypeptides, and in particular, BCR-ABL polypeptides having a mutation at position 49, 53, 100, 126, 138, 146, 242, 271, 292, 324, 338, 351 and/or 458. In particular, the present invention describes methods of identifying subjects that have a N49S, N53S, C100R, S126P, E138G, N146S, I242T, K271R, E292V, L324Q, V338M, M351A, M458T mutation. In certain aspects, the present invention describes methods of identifying subjects that have a N49S, N53S, C100R, S126P, E138G, N146S, I242T, K271R, E292V, L324Q, V338M, M351A, M458T mutation. The invention further describes methods of identifying subject that have, not only the N49S, N53S, C100R, S126P, E138G, N146S, I242T, K271R, E292V, L324Q, V338M, M351A, M458T mutation, but any number of additional mutations that are associated with at least partial resistant to drug therapy, including therapy with imatinib and/or N-(2-chloro-6-methylphenyl)-2-[[6-[4-(2-hydroxyethyl)-1-piperazinyl]-2-methyl-4-pyrimidinyl] amino]-5-thiazolecarboxamide or a pharmaceutically acceptable salt, hydrate, or solvate thereof.

The present invention also describes the treatment of such subjects by tailoring their treatment regimen depending on whether or not they harbor mutant BCR-ABL polypeptides, and in particular, BCR-ABL polypeptides having at least a N49S, N53S, C100R, S126P, E138G, N146S, I242T, K271R, E292V, L324Q, V338M, M351A, M458T mutation.

The present invention also describes the treatment of such subjects by tailoring their treatment regimen depending on whether or not they harbor mutant BCR-ABL polypeptides having a N49S, N53S, C100R, S126P, E138G, N146S, I242T, K271R, E292V, L324Q, V338M, M351A, and/or M458T mutation.

The present invention also describes mutant BCR-ABL polypeptides having at least a N49S, N53S, C100R, S126P, E138G, N146S, I242T, K271R, E292V, L324Q, V338M, M351A, and/or M458T mutation and polynucleotides encoding such polypeptides. The present invention further describes mutant BCR-ABL polypeptides having not only the N49S, N53S, C100R, S126P, E138G, N146S, I242T, K271R, E292V, L324Q, V338M, M351A, and/or M458T mutations but any number of additional mutations that are associated with at least partial resistance to drug therapy, including therapy with imatinib and/or N-(2-chloro-6-methylphenyl)-2-[[6-[4-(2-hydroxyethyl)-1-piperazinyl]-2-methyl-4-pyrimidinyl] amino]-5-thiazolecarboxamide or a pharmaceutically acceptable salt, hydrate, or solvate thereof.

The invention describes methods of establishing a treatment regimen for an individual having a BCR-ABL related disorder. The treatment regimen can comprise the administration of N-(2-chloro-6-methylphenyl)-2-[[6-[4-(2-hydroxyethyl)-1-piperazinyl]-2-methyl-4-pyrimidinyl]amino]-5-thiazolecarboxamide, or a pharmaceutically acceptable salt, hydrate, or solvate thereof, at a higher dose or dosing frequency than recommended for an individual having a non-mutated BCR-ABL or a BCR-ABL polypeptide lacking the N49S, N53S, C100R, S 126P, E138G, N146S, I242T, K271R, E292V, L324Q, V338M, M351A, and/or M458T mutation. Alternatively, the treatment regiment can comprise combination therapy with N-(2-chloro-6-methylphenyl)-2-[[6-[4-(2-hydroxyethyl)-1-piperazinyl]-2-methyl-4-pyrimidinyl]amino]-5-thiazolecarboxamide and any other agent that works to inhibit proliferation of cancerous cells or induce apoptosis of cancerous cells, including, for example, a tubulin stabilizing agent, a farnysyl transferase inhibitor, a BCR-ABL T3151 inhibitor and/or another protein tyrosine kinase inhibitor. Preferred other agents include imatinib, AMN107, PD180970, CGP76030, AP23464, SKI 606, NS-187, or AZD0530. The treatment regimen can include administration of a higher dose of N-(2-chloro-6-methylphenyl)-2-[[6-[4-(2-hydroxyethyl)-1-piperazinyl]-2-methyl-4-pyrimidinyl]amino]-5-thiazolecarboxamide with a second therapeutic agent , a reduced dose of N-(2-chloro-6-methylphenyl)-2-[[6-[4-(2-hydroxyethyl)-1-piperazinyl]-2-methyl-4-pyrimidinyl]amino]-5-thiazolecarboxamide with a second therapeutic agent, or an unchanged dose of N-(2-chloro-6-methylphenyl)-2-[[6-[4-(2-hydroxyethyl)-1-piperazinyl]-2-methyl-4-pyrimidinyl]amino]-5-thiazolecarboxamide with a second therapeutic agent.

The present invention provides a kit for use in determining treatment strategy for an individual with a protein tyrosine kinase-associated disorder, comprising a means for detecting a mutant BCR-ABL kinase, including but not limited to, N49S, N53S, C100R, S126P, E138G, N146S, I242T, K271R, E292V, L324Q, V338M, M351A, and/or M458T, in a biological sample from said patient; and optionally instructions for use and interpretation of the kit results. The kit can also comprise, for example, a means for obtaining a biological sample from an individual. The treatment strategy can comprise, for example, the administration of N-(2-chloro-6-methylphenyl)-2-[[6-[4-(2-hydroxyethyl)-1-piperazinyl]-2-methyl-4-pyrimidinyl]amino]-5-thiazolecarboxamide or a pharmaceutically acceptable salt, hydrate, or solvate thereof.

### BRIEF DESCRIPTION OF THE FIGURES/DRAWINGS

The file of this patent application contains at least one Figure executed in color. Copies of this patent with color Figure(s) will be provided by the Patent and Trademark Office upon request and payment of the necessary fee.

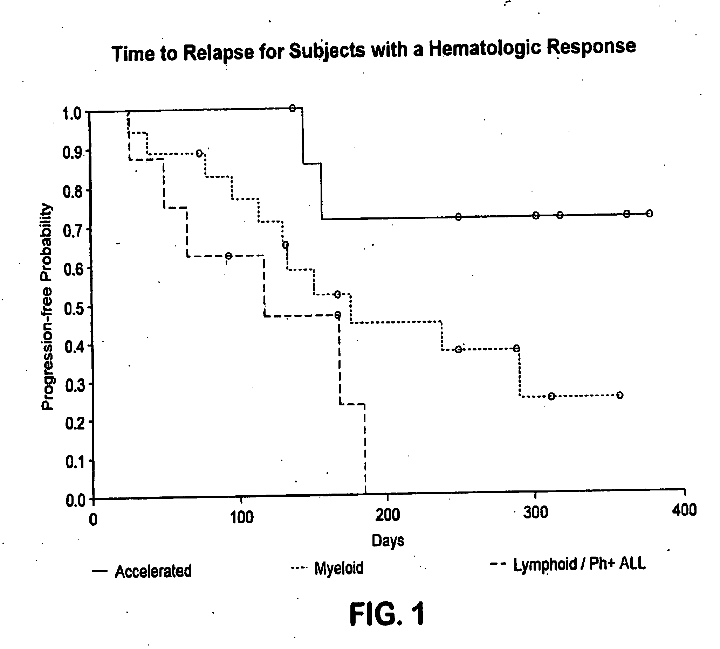

**Figure 1****. Time to Progression in Responding Patients. Kaplan-Meier** plot of time to progression in the 35 patients who met the criteria for minor or major hematologic response are shown, by disease cohort. One patient in the LBC/Ph+ALL cohort remains on study at 167 days.

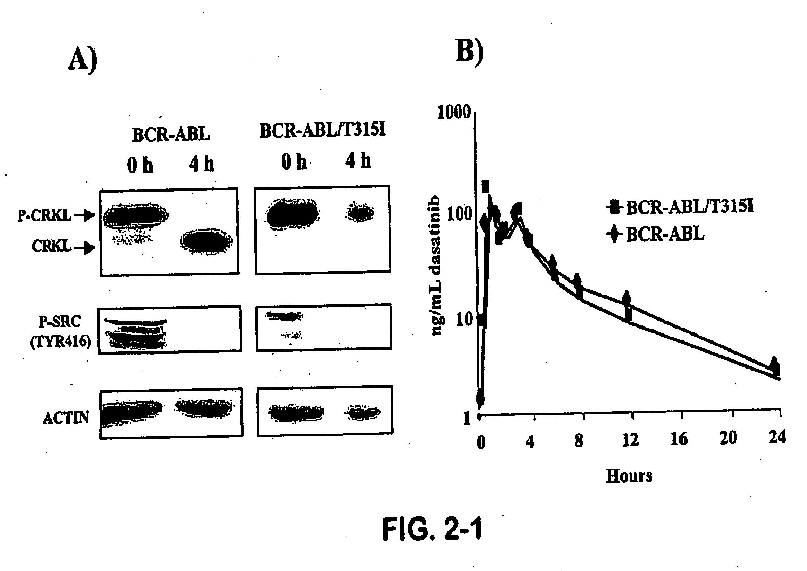

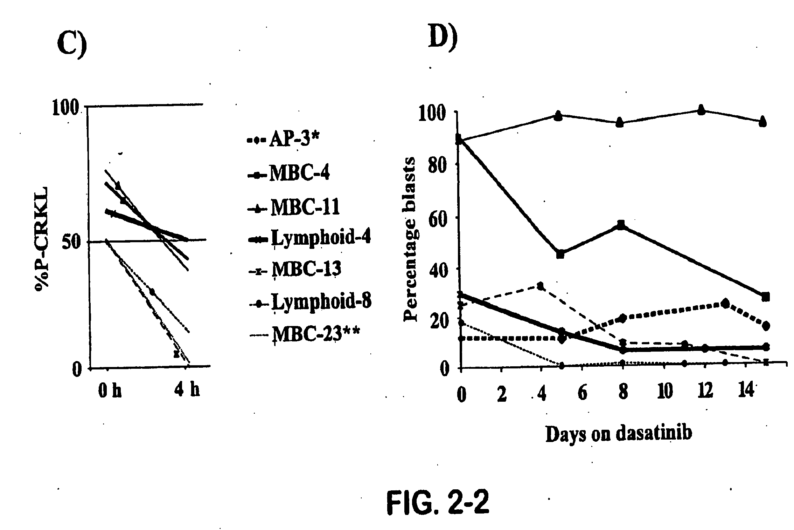

**Figure 2****. Molecular correlates of response to dasatinib. A)** Effect of dasatinib on the kinase activities of BCR-ABL and SRC in patients harboring wild-type or T315I *BCR-ABL* mutation. Protein lysates prepared from patients AP-7 (left) and AP-8 (right) were prepared from PBMC isolated immediately prior to (O h) and four hours after (4 h) ingesting the first dose (90 mg) of dasatinib. Results of Western immunoblotting with α-CRKL antibody or α-P-SRC are shown. Anti-actin antibody was used as a loading control. **B)** Pharmacokinetic analysis of dasatinib in patient AP-7 (blue) and AP-8 (pink). Plasma concentrations of dasatinib at the indicated times following initial dose ingestion of dasatinib are depicted. **C)** Quantification of the ratio of [P-CRKL/total CRKL] immediately prior to (0 h), and four hours after (4 h) initial dasatinib dose ingestion in patients without T315I *BCR-ABL* mutation who failed to achieve an objective hematologic or cytogenetic response. **D)** Effect of dasatinib on peripheral blood blast percentage in patients without T315I *BCR-ABL* mutation who failed to achieve an objective hematologic or cytogenetic response. Number of days after initial dose of dasatinib is shown.

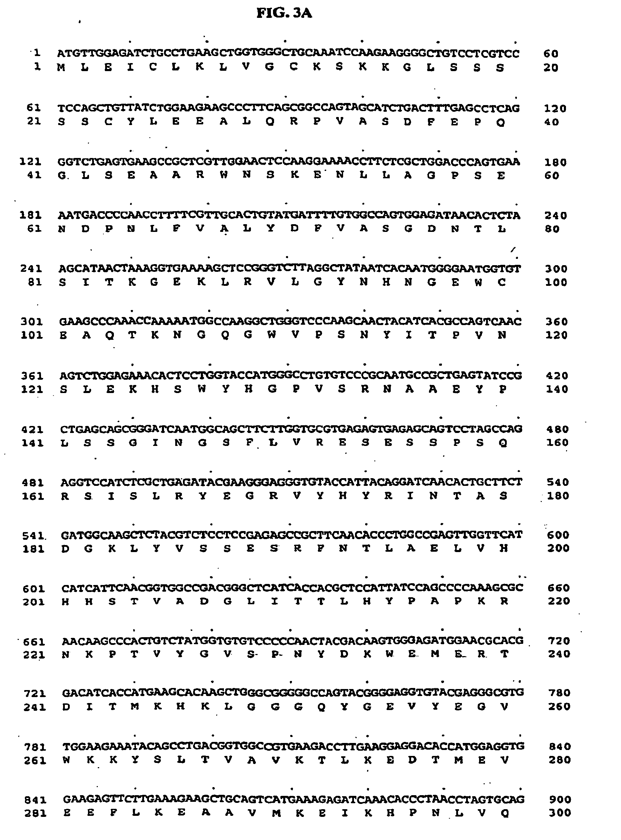

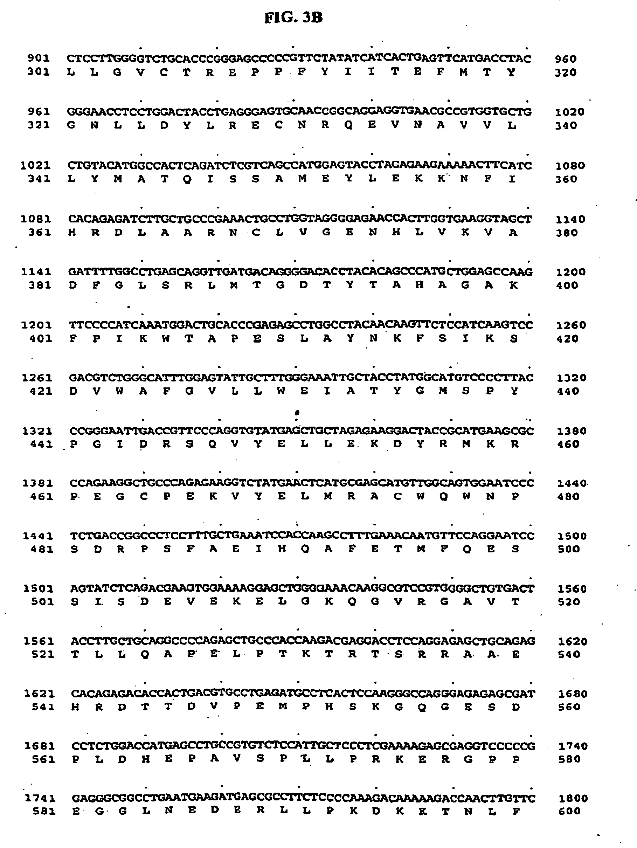

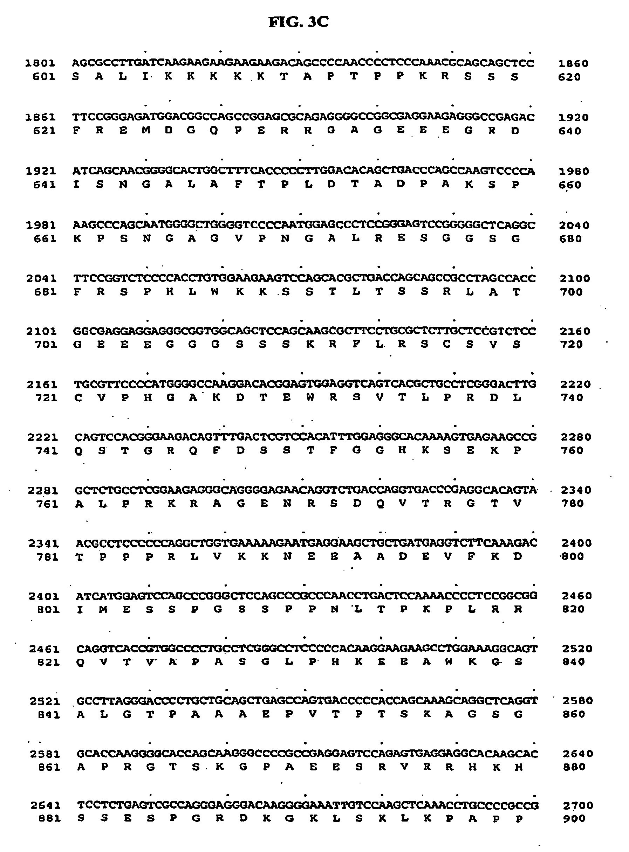

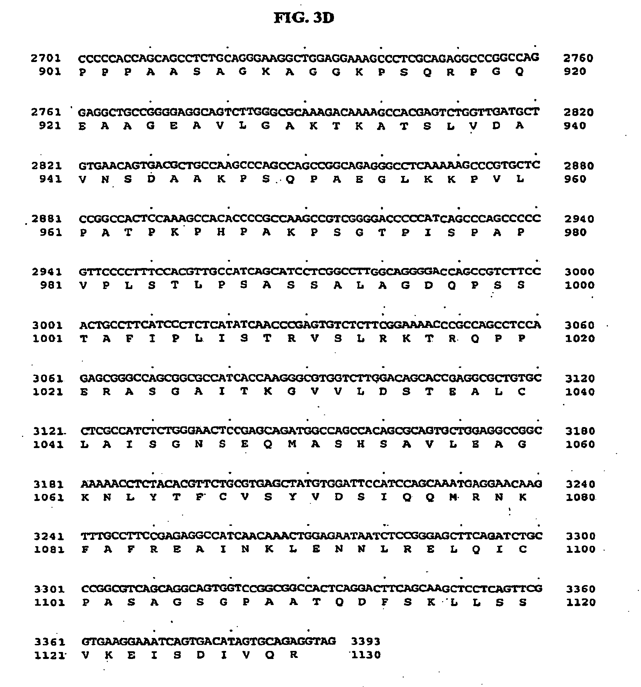

**Figures 3A****-E** show the polynucleotide sequence (SEQ ID NO:1) and deduced amino acid sequence (SEQ ID NO:2) of the wild-type BCR-ABL polypeptide. The standard one-letter abbreviation for amino acids is used to illustrate the deduced amino acid sequence. The polynucleotide sequence contains a sequence of 3393 nucleotides (SEQ ID NO:1; gi|177942; gi|NP_005148.1 and gi|M14752.1), encoding a polypeptide of 1130 amino acids (SEQ ID NO:2; gi|177943; gi|NP_005148.1 and gi|M14752.1).

### DETAILED DESCRIPTION OF THE INVENTION

Imatinib is a small-molecule inhibitor of the BCR/ABL tyrosine kinase that produces clinical remissions in CML patients with minimal toxicity relative to older treatment modalities. Imatinib is now frontline therapy for CML but resistance is increasingly encountered. According to one study, the estimated 2-year incidence of resistance to imatinib mesylate was 80% in blastic phase, 40% to 50% in accelerated phase, and 10% in chronic phase post-interferon-α failure (Kantarjian et al, Blood, 101(2):473-475 (2003). Dasatinib is an ATP-competitive, dual SRC/ABL inhibitor (Lombardo, L. J., et al., J. Med. Chem., 47:6658-6661 (2004)). Notably, Dasatinib has been shown to be several hundred fold more effective in treating CML than Imatinib. On account of the demonstration that patients with complex karyotypes have increased likelihood of being at least partially resistant to Imatinib and Dasatinib, respectively, the inventors of the present invention describe for the first time methods to identify patients who may most benefit from more aggressive dosing regimen monotherapy comprising Dasatinib, or the combination of Dasatinib with other protein tyrosine kinase inhibitors, or other agents.

The present invention is also based, in part, on the discovery that certain individuals treated with N-(2-chloro-6-methylphenyl)-2-(6-(4-(2-hydroxyethyl) piperazin-1-yl)-2-methylpyrimidin-4-ylamino)thiazole-5-carboxamide develop mutations at select amino acid positions within the BCR-ABL kinase domain and that these mutations are associated with at least partial resistance to therapy with N-(2-chloro-6-methylphenyl)-2-(6-(4-(2-hydroxyethyl) piperazin-1-yl)-2-methylpyrimidin-4-ylamino)thiazole-5-carboxamide.

Recognition that these mutations exist in an individual having a BCR-ABL-associated disorder can, among other things, help in determining the responsiveness of individuals to treatment with N-(2-chloro-6-methylphenyl)-2-[[6-[4-(2-hydroxyethyl)-1-piperazinyl]-2-methyl-4-pyrimidinyl] amino]-5-thiazolecarboxamide or a pharmaceutically acceptable salt, hydrate, or solvate thereof, and it can help tailor treatment regimens appropriately.

The structure and use of Dasatinib as an anticancer agent is described in Lombardo, L. J., et al., J. Med. Chem., 47:6658-6661 (2004).

The structure and use of imatinib as an anticancer agent is described in B. J. Druker et al., N. Engl. J. Med. 344, 1031 (2001) and S. G. O'Brien et al., N. Engl. J. Med. 348, 994 (2003).

Wherever the term "Dasatinib" is used herein, it is understood (unless otherwise indicated) that the compound N-(2-chloro-6-methylphenyl)-2-[[6-[4-(2-hydroxyethyl)-1-piperazinyl]-2-methyl-4-pyrimidinyl] amino]-5-thiazolecarboxamide having the following structure (I): 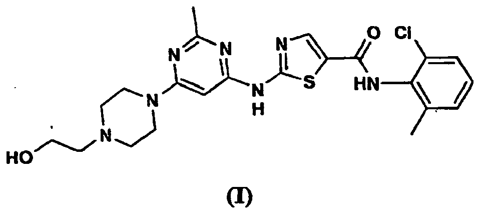 is intended, as well as all pharmaceutically acceptable salts, hydrates, and solvates, thereof. Compound (I) can also be referred to as *N*-(2-chloro-6-methylphenyl)-2-((6-(4-(2-hydroxyethyl)-1-piperazinyl)-2-methyl-4-pyrimidinyl)amino)-1,3-thiazole-5-carboxamide in accordance with IUPAC nomenclature. Use of the term "N-(2-chloro-6-methylphenyl)-2-[[6-[4-(2 hydroxyethyl)-1-piperazinyl]-2-methyl-4-pyrimidinyl]amino]-5-thiazolecarboxamide" encompasses (unless otherwise indicated) solvates (including hydrates) and polymorphic forms of the compound (I) or its salts Pharmaceutical compositions of N-(2-chloro-6-methylphenyl)-2-[[6-[4-(2-hydroxyethyl)-1-piperazinyl]-2-methyl-4-pyrimidinyl]amino]-5-thiazolecarboxamide include all pharmaceutically acceptable compositions comprising N-(2-chloro-6-methylphenyl)-2-[[6-[4-(2-hydroxyethyl)-1-piperazinyl]-2-methyl-4-pyrimidinyl]amino]-5-thiazolecarboxamide and one or more diluents, vehicles and/or excipients. One example of a pharmaceutical composition comprising N-(2-chloro-6-methylphenyl)-2-[[6-[4-(2-hydroxyethyl)-1-piperazinyl]-2-methyl-4-pyrimidinyl] amino]-5-thiazolecarboxamide is SPRYCEL™ (Bristol-Myers Squibb Company). SPRYCEL™ comprises N-(2-chloro-6-methylphenyl)-2-[[6-[4-(2-hydroxyethyl)-1-piperazinyl]-2-methyl-4-pyrimidinyl] amino]-5-thiazolecarboxamide as the active ingredient, also referred to as dasatinib, and as inactive ingredients or excipients, lactose monohydrate, microcrystalline cellulose, croscarmellose sodium, hydroxypropyl cellulose, and magnesium stearate in a tablet comprising hypromellose, titanium dioxide, and polyethylene glycol.

Wherein the term "a farnysyl transferase inhibitor" herein, it is understood (unless otherwise indicated) that the compound have formula (II), (R)-2,3,4,5-tetrahydro-1-(1H-imidazo-4-ylmethyl)-3-(phenylmethyl)-4-(2-thienylsulfonyl)-1H-1,4-benzodiazepine-7-carbonitrile, hydrochloride salt, is an anti-cancer agent. The compound of formula (II) is a cytotoxic FT inhibitor which is known to kill non-proliferating cancer cells preferentially. The compound of formula (II) may further be useful in killing stem cells. 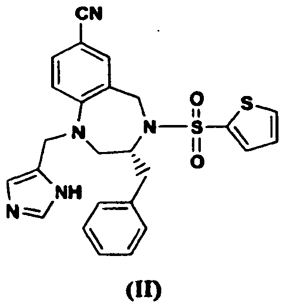 Uses of the compound of formula (II) are also described in WO2004/015130, published February 19,2004.

The terms "combination", and "combination of N-(2-chloro-6-methylphenyl)-2-[[6-[4-(2-hydroxyethyl)-1-piperazinyl]-2-methyl-pyrimidinyl] amino]-5-thiazolecarboxamide" as used herein refers to a combination of N-(2-chloro-6-methylphenyl)-2-[[6-[4-(2-hydroxyethyl)-1-piperazinyl]-2-methyl4-pyrimidinyl]amino]-5-thiazolecarboxamide and imatinib; combination of N-(2-chloro-6-methylphenyl)-2-[[6-[4-(2-hydroxyethyl)-1-piperazinyl]-2-methyl-4-pyrimidinyl]amino]-5-thiazolecarboxamide and a tubulin stabilizing agent (e.g., pacitaxol, epothilone, taxane, etc.); a combination of N-(2-chloro-6-methylphenyl)-2-[[6-[4-(2-hydroxyethyl)-1-piperazinyl]-2-methyl-4-pyrimidinyl]amino]-5-thiazolecarboxamide and a farnysyl transferase inhibitor (e.g., (R)-2,3,4,5-tetrahydro-1-(1H-imidazol-4-ylmethyl)-3-(phenylmethyl)-4-(2-thienylsulfonyl)-1 H-1,4-benzodiazepine-7-carbonitrile); a combination of N-(2-chloro-6-methylphenyl)-2-[[6-[4-(2-hydroxyethyl)-1-piperazinyl]-2-methyl-4-pyrimidinyl]amino]-5-thiazolecarboxamide and another protein tyrosine kinase inhibitor; a combination of N-(2-chloro-6-methylphenyl)2-[[6-[4-(2-hydroxyethyl)-1-piperazinyl]-2-methyl-4-pyrimidinyl]amino]-5-thiazolecarboxamide with AMN-107, PD180970, CGP76030, AP23464, SKI 606, NS-187, AZD0530, and/or ARIAD; and/or any compounds disclosed and referenced in Deininger et al (Blood, 105(7):2640-2653 (2005); but are not limited to IFN, pegylated IFN, homoharringtonine, cytabine, hydroxyurea, farnesyl transferase inhibitors, lonafamib, tipifamib, MEK1 inhibitors, PD98059, RAF-1 inhibitors, BAY43-9006, PI3 kinase inhibitors, LY294002, mTOR inhibitors, rapamycin, cyclin-dependent kinase inhibitors, favopiridol; ; a combination of N-(2-chloro-6-methylphenyl)-2-[[6-[4-(2-hydroxyethyl)-1-piperazinyl]-2-methyl-4-pyrimidinyl]amino]-5-thiazolecarboxamide and ATP non-competitive inhibitors ONO12380; a combination of N-(2-chloro-6-methylphenyl)-2-[[6-[4-(2-hydroxyethyl)-1-piperazinyl]-2-methyl-4-pyrimidinyl]amino]-5-thiazolecarboxamide and Aurora kinase inhibitor VX-680; a combination of N-(2-chloro-6-methylphenyl)-2-[[6-[4-(2-hydroxyethyl)-1-piperazinyl]-2-methyl-4-pyrimidinyl]amino]-5-thiazolecarboxamide and p38 MAP kinase inhibitor High-796; a combination of N-(2-chloro-6-methylphenyl-2-[[6-[4-(2-hydroxyethyl)-1-piperazinyl]-2-methyl-4-pyrimidinyl]amino]-5-thiazolecarboxamide with any number of BCR-ABL inhibitors; a combination of any compounds disclosed and/or referenced in La Rosee et al (Leukemia, 16:1213-1219 (2002)); or pharmaceutically acceptable salts thereof, and optionally in therapeutically effective amounts thereof.

For use herein, a "BCR-ABL inhibitor" refers to any molecule or compound that can partially inhibit BCR-ABL or mutant BCR-ABL activity or expression. These include inhibitors of the Src family kinases such as BCR/ABL, ABL, c-Src, SRC/ABL, and other forms including, but not limited to, JAK, FAK, FPS, CSK, SYK, BTK FGR, FYN, YES, BLK, HCK, LCK AND LYN, as well as other protein tyrosine kinases, including PDGFR, c-kit and Eph receptors. A series of inhibitors, based on the 2-phenylaminopyrimidine class of pharmacophotes, has been identified that have exceptionally high affinity and specificity for Abl (see, e.g., Zimmerman et al., Bloorg, Med. Chem. Lett. 7, 187 (1997)). All of these inhibitors are encompassed within the term a BCR-ABL inhibitor. Imatinib, one of these inhibitors, also known as STI-571 (formerly referred to as Novartis test compound CGP 57148 and also known as Gleevec), has been successfully tested in clinical trail a therapeutic agent for CML. AMN107, is another BCR-ABL kinase inhibitor that was designed to fit into the ATP-binding site of the BCR-ABL protein with higher affinity than imatinib. In addition to being more potent than imatinib (IC50<30 nM) against wild-type BCR-ABL, AMN107 is also significantly active against 32/33 imatinib-resistant BCR-ABL mutants. In preclinical studies, AMN107 demonstrated activity in vitro and in vivo against wild-type and imatinib-resistant BCR-ABL-expressing cells. In phase I/II clinical trials, AMN107 has produced haematological and cytogenetic responses in CML patients, who either did not initially respond to imatinib or developed imatinib resistance (Weisberg et al., British Journal of Cancer (2006) 94, 1765-1769). SKI-606, NS-187, AZD0530, PD180970, CGP76030, and AP23464 are all examples of kinase inhibitors that can be used in the present invention. SKI-606 is a 4-anilino-3-quinolinecarbonitrile inhibitor of Abl that has demonstrated potent antiproliferative activity against CML cell (Golas et al., Cancer Research (2003) 63, 375-381). AZD0530is a dual Abl/Src kinase inhibitor that is in ongoing clinical trials for the treatment of solid tumors and leukemia (Green et al., Preclinical Activity of AZD0530, a novel, oral, potent, and selective inhibitor of the Src family kinases. Poster 3161 presented at the EORTC-NCI-AACR, Geneva Switzerland 28 September 2004). PD180970 is a pyrido[2,3-d]pyrimidine derivative that has been shown to inhibit BCR-ABL and induce apoptosis in BCR-ABL expressing leukemic cells (Rosee et al., Cancer Research (2002) 62, 7149-7153). CGP76030 is dual-specific Src and Abl kinase inhibitor shown to inhibit the growth and survival of cells expressing imatinib-resistant BCR-ABL kinases (Warmuth et al., Blood, (2003) 101(2), 664-672). AP23464 is an ATP-based kinase inhibitor that has been shown to inhibit imatinib-resistant BCR-ABL mutants (O'Hare et al., Clin Cancer Res (2005) 11(19), 6987-6993). NS-187 is a selective dual Bcr-Abl/Lyn tyrosine kinase inhibitor that has been shown to inhibit imatinib-resistant BCR-ABL mutants (Kimura et al., Blood, 106(12):3948-3954 (2005)).

"Protein tyrosine kinase-associated disorders" are those disorders which result from aberrant tyrosine kinase activity, and/or which are alleviated by the inhibition of one or more of these enzymes. Disorders included in the scope of the present invention may include chronic myeloid leukemia, acute lymphoblastic leukemia, Philadelphia chromosome positive acute lymphoblastic leukemia (Ph+ ALL), squamous cell carcinoma, small-cell lung cancer, non-small cell lung cancer, glioma, gastrointestinal cancer, renal cancer, ovarian cancer, liver cancer, colorectal cancer, endometrial cancer, kidney cancer, prostate cancer, thyroid cancer, neuroblastoma, pancreatic cancer, glioblastoma multiforme, cervical cancer, stomach cancer, bladder cancer, hepatoma, breast cancer, colon carcinoma, and head and neck cancer, gastric cancer, germ cell tumor, pediatric sarcoma, sinonasal natural killer, multiple myeloma, acute myelogenous leukemia, chronic lymphocytic leukemia, mastocytosis and any symptom associated with mastocytosis. In addition, disorders include urticaria pigmentosa, mastocytosises such as diffuse cutaneous mastocytosis, solitary mastocytoma in human, as well as dog mastocytoma and some rare subtypes like bullous, erythrodermic and teleangiectatic mastocytosis, mastocytosis with an associated hematological disorder, such as a myeloproliferative or myelodysplastic syndrome, or acute leukemia, myeloproliferative disorder associated with mastocytosis, and mast cell leukemia. Various cancers are also included within the scope of protein tyrosine kinase-associated disorders including (but not limited to) the following: carcinoma, including that of the bladder, breast, colon, kidney, liver, lung, ovary, pancreas, stomach, cervix, thyroid, testis, particularly testicular seminomas, and skin; including squamous cell carcinoma; gastrointestinal stromal tumors ("GIST"); hematopoietic tumors of lymphoid lineage, including leukemia, acute lymphocytic leukemia, acute lymphoblastic leukemia, B-cell lymphoma, T-cell lymphoma, Hodgkins lymphoma, non-Hodgkins lymphoma, hairy cell lymphoma and Burketts lymphoma; hematopoietic tumors of myeloid lineage, including acute and chronic myelogenous leukemias and promyelocytic leukemia; tumors of mesenchymal origin, including fibrosarcoma and rhabdomyoscarcoma; other tumors, including melanoma, seminoma, tetratocarcinoma, neuroblastoma and glioma; tumors of the central and peripheral nervous system, including astrocytoma, neuroblastoma, glioma, and schwannomas; tumors of mesenchymal origin, including fibrosarcoma, rhabdomyoscaroma, and osteosarcoma; and other tumors, including melanoma, xenoderma pigmentosum, keratoactanthoma, seminoma, thyroid follicular cancer, teratocarcinoma, chemotherapy refractory non-seminomatous germ-cell tumors, and Kaposi's sarcoma.

"Protein tyrosine kinase-associated disorders" may also include those disorders which result from BCR-ABL activity, including mutant BCR-ABL activity, and/or which are alleviated by the inhibition of BCR-ABL, including mutant BCR-ABL, expression and/or activity. A reciprocal translocation between chromosomes 9 and 22 produces the oncogenic BCR-ABL fusion protein. The phrase "Protein tyrosine kinase-associated disorders" is inclusive of "mutant BCR-ABL associated disorders" and "BCR-ABL associated disorders".

The term "complex chromosome karyotype" is meant to encompass all non-Philadelphia chromosome abnormalities, karotypes that include chromosomal abnormalities in addition to the presence of a Philadelphia chromosome, in addition to the meaning of this phrase in the art, including the following non-limiting examples disclosed in: Espinoza et al., Cancer Gen. and Cyto., 157:175-7 (2005); Heller et al., Int. J. Oncol., 24(1)127-36 (2004); Giehl et al., Leukemia 19:1192-1197 (2005); and Oudat et al., Arch. Path. Lab. Medicine, 125:437-439 (2001).

The terms "treating", "treatment" and "therapy" as used herein refer to curative therapy, prophylactic therapy, preventative therapy, and mitigating disease therapy.

The phrase "more aggressive dosing regimen" or "increased dosing frequency regimen", as used herein refers to a dosing regimen that necessarily exceeds the basal and/or prescribed dosing regimen of N-(2-chloro-6-methylphenyl)-2-[[6-[4-(2-hydroxyethyl)-1-piperazinyl]-2-methyl-4-pyrimidinyl]amino]-5-thiazolecarboxamide either due to an increased frequency of administration, increased or escalated dose, or the route of administration which may result in an increased bioavailable level of N-(2-chloro-6-methylphenyl)-2-[[6-[4-(2-hydroxyethyl)-1-piperazinyl]-2-methyl-4-pyrimidinyl]amino]-5-thiazolecarboxamide. Non-limiting examples of such dosing regimens may be found by reference in Blood (ASH Annual Meeting Abstracts) 2004, Volume 104: Abstract 20, "Hematologic and Cytogenetic Responses in imatinib-Resistant Accelerated and Blast Phase Chronic Myeloid Leukemia (CML) Patients Treated with the Dual SRC/ABL Kinase Inhibitor N-(2-chloro-6-methylphenyl)-2-[[6-[4-(2-hydroxyethyl)-1-piperazinyl]-2-methyl-4-pyrimidinyl]amino]-5-thiazolecarboxamide: Results from a Phase I Dose Escalation Study.", by Moshe Talpaz, et al; and/or dosing regimens outlined in Deininger et al (Blood, 105(7):2640-2653 (2005).

It is to be understood that this invention is not limited to particular methods, reagents, compounds, compositions, or biological systems, which can, of course, vary. It is also to be understood that the terminology used herein is for the purpose of describing particular aspects only, and is not intended to be limiting. As used in this specification and the appended claims, the singular forms "a", "an", and "the" include plural referents unless the content clearly dictates otherwise. Thus, for example, reference to "a peptide" includes a combination of two or more peptides, and the like.

"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

Treatment regimens can be established based upon the detection of a complex karyotype. For example, the invention encompasses screening cells from an individual who may suffer from, or is suffering from, a disorder that is commonly treated with a protein tyrosine kinase. Such a disorder can include leukemia or disorders associated therewith, or cancers described herein. The cells of an individual are screened, using methods known in the art, for identification of a complex karyotype.

If a complex karyotype is found in the cells from said individual, treatment regimens can be developed appropriately. For example, the presence of a complex karyotype can indicate that said cells are or will become at least partially resistant to commonly used kinase inhibitors, including BCR-ABL inhibitors. For example, a complex karyotype can indicate that the cells in an individual are or are expected to become at least partially resistant to treatment with a kinase inhibitor such as N-(2-chloro-6-methylphenyl)-2-[[6-[4-(2-hydroxyethyl)-1-piperazinyl]-2-methyl-4-pyrimidinyl]amino]-5-thiazolecarboxamide and that administration of higher doses of the same or more aggressive dosing regimens or combination therapy may be warranted. As disclosed herein, in such cases, treatment can include the use of an increased dosing frequency or increased dosage of N-(2-chloro-6-methylphenyl)-2-[[6-[4-(2-hydroxyethyl)-1-piperazinyl]-2-methyl-4-pyrimidinyl]amino]-5-thiazolecarboxamide or a salt, hydrate, or solvate thereof, a combination of N-(2-chloro-6-methylphenyl)-2-[[6-[4-(2-hydroxyethyl)-1-piperazinyl]-2-methyl-4-pyrimidinyl] amino]-5-thiazolecarboxamide or a pharmaceutically acceptable salt, hydrate, or solvate thereof and another kinase inhibitor drug such as imatinib, AMN107, PD180970, GGP76030, AP23464, SKI 606, and/or AZD0530; a combination of N-(2-chloro-6-methylphenyl)-2-[[6-[4-(2-hydroxyethyl)-1-piperazinyl]-2-methyl-4-pyrimidinyl]amino]-5-thiazolecarboxamide and a tubulin stabilizing agent (e.g., pacitaxol, epothilone, taxane, etc.); a combination of N-(2-chloro-6-methylphenyl)-2-[[6-[4-(2-hydroxyethyl)-1-piperazinyl]-2-methyl-4-pyrimidinyl]amino]-5-thiazolecarboxamide and a farnysyl transferase inhibitor, any other combination disclosed herein; and any other combination or dosing regimen comprising N-(2-chloro-6-methylphenyl)-2-[[6-[4-(2-hydroxyethyl)-1-piperazinyl]-2-methyl-4-pyrimidinyl]amino]-5-thiazolecarboxamide disclosed herein. In one aspect, an increased level of N-(2-chloro-6-methylphenyl)-2-[[6-[4-(2-hydroxyethyl)-1-piperazinyl]-2-methyl-4-pyrimidinyl]amino]-5-thiazolecarboxamide would be about 10, 20, 30, 40, 50, 60, 70, 80, 90, or 95% more than the typical N-(2-chloro-6-methylphenyl)-2-[[6-[4-(2-hydroxyethyl)-1-piperazinyl]-2-methyl-4-pyrimidinyl]amino]-5-thiazolecarboxamide dose for a particular indication or for individual (e.g., 50mg, 70mg, 90mg, 120mg BID), or about 1.5x, 2x, 2.5x, 3x, 3.5x, 4x, 4.5x, 5x, 6x, 7x, 8x, 9x, or 10x more N-(2-chloro-6-methylphenyl)-2-[[6-[4-(2-hydroxyethyl)-1-piperazinyl]-2-methyl-4-pyrimidinyl]amino]-5-thiazolecarboxamide than the typical N-(2-chloro-6-methylphenyl)-2-[[6-[4-(2-hydroxyethyl)-1-piperazinyl]-2-methyl-4-pyrimidinyl]amino]-5-thiazolecarboxamide dose for a particular indication or for individual.

A therapeutically effective amount of N-(2-chloro-6-methylphenyl)-2-[[6-[4-(2-hydroxyethyl)-1-piperazinyl]-2-methyl-4-pyrimidinyl] amino]-5-thiazolecarboxamide or a pharmaceutically acceptable salt, hydrate, or solvate thereof can be orally administered as an acid salt of N-(2-chloro-6-methylphenyl)-2-[[6-[4-(2-hydroxyethyl)-1-piperazinyl]-2-methyl-4-pyrimidinyl]amino]-5-thiazolecarboxamide. The actual dosage employed can be varied depending upon the requirements of the patient and the severity of the condition being treated. Determination of the proper dosage for a particular situation is within the skill of the art. The effective amount of N-(2-chloro-6-methylphenyl)-2-[[6-[4-(2-hydroxyethyl)-1-piperazinyl]-2-methyl-4-pyrimidinyl] amino]-5-thiazolecarboxamide or a pharmaceutically acceptable salt, hydrate, or solvate thereof (and Compound I salt) can be determined by one of ordinary skill in the art, and includes exemplary dosage amounts for an adult human of from about 0.05 to about 100 mg/kg of body weight of N-(2-chloro-6-methylphenyl)-2-[[6-[4-(2-hydroxyethyl)-1-piperazinyl]-2-methyl-4-pyrimidinyl] amino]-5-thiazolecarboxamide or a pharmaceutically acceptable salt, hydrate, or solvate thereof, per day, which can be administered in a single dose or in the form of individual divided doses, such as from 1, 2, 3, or 4 times per day. In certain embodiments, N-(2-chloro-6-methylphenyl)-2-[[6-[4-(2-hydroxyethyl)-1-piperazinyl]-2-methyl-4-pyrimidinyl] amino]-5-thiazolecarboxamide or a pharmaceutically acceptable salt, hydrate, or solvate thereof is administered 2 times per day at 70 mg. Alternatively, it can be dosed at, for example, 50, 70, 90, 100, 110, or 120 BID, or 100, 140, or 180 once, twice, or three times daily. It will be understood that the specific dose level and frequency of dosing for any particular subject can be varied and will depend upon a variety of factors including the activity of the specific compound employed, the metabolic stability and length of action of that compound, the species, age, body weight, general health, sex and diet of the subject, the mode and time of administration, rate of excretion, drug combination, and severity of the particular condition. Preferred subjects for treatment include animals, most preferably mammalian species such as humans, and domestic animals such as dogs, cats, and the like, subject to protein tyrosine kinase-associated disorders. The same also applies to Compound 11 or any combination of Compound I and II, or any combination disclosed herein.

A treatment regimen is a course of therapy administered to an individual suffering from a protein kinase associated disorder that can include treatment with one or more kinase inhibitors, as well as other therapies such as radiation and/or other agents (*i.e*., combination therapy). When more than one therapy is administered, the therapies can be administered concurrently or consecutively (for example, more than one kinase inhibitor can be administered together or at different times, on a different schedule). Administration of more than one therapy can be at different times (i.e., consecutively) and still be part of the same treatment regimen. As disclosed herein, for example, cells from an individual suffering from a protein kinase associated disorder can be found to develop at least partial resistance to N-(2-chloro-6-methylphenyl)-2-[[6-[4-(2-hydroxyethyl)-1-piperazinyl]-2-methyl-4-pyrimidinyl]amino]-5-thiazolecarboxamide if a complex karyotype is present. Based upon the present discovery that such cells can be sensitive to combination therapy or a more aggressive dosage or dosing regimen of N-(2-chloro-6-methylphenyl)-2-[[6-[4-(2-hydroxyethyl)-1-piperazinyl]-2-methyl-4-pyrimidinyl] amino]-5-thiazolecarboxamide or a pharmaceutically acceptable salt, hydrate, or solvate thereof, a treatment regimen can be established that includes treatment with the combination either as a monotherapy, or in combination with another kinase inhibitor, or in combination with another agent as disclosed herein. Additionally, the combination can be administered with radiation or other known treatments.

Therefore the present invention includes a method for establishing a treatment regimen for an individual suffering from a protein tyrosine kinase associated disorder or treating an individual suffering from a protein tyrosine kinase disorder comprising determining whether a biological sample obtained from an individual has a complex karyotype, and administering to the subject an appropriate treatment regimen based on whether a complex karyotype is present. The determination can be made by any method known in the art, for example, by screening said sample of cells for the presence of a complex karyotype or by obtaining information from a secondary source that the individual has a complex karyotype.

Treatment regimens can also be established based upon the presence of one or more mutant BCR-ABL kinases disclosed herein. For example, the invention encompasses screening cells from an individual who may suffer from, or is suffering from, a disorder that is commonly treated with a kinase inhibitor. Such a disorder can include myeloid leukemia or disorders associated therewith, or cancers described herein. The cells of an individual are screened, using methods known in the art, for identification of a mutation in a BCR-ABL kinase. Mutations of interest are those that result in BCR-ABL kinase being constitutively activated. Specific mutations include, for example, N49S, N53S, C100R, S126P, E138G, N 146S, I242T, K271R, E292V, L324Q, V338M, M351A, M458T. Other mutations include, for example, E279K, F359C, F359I, L364I, L387M, F486S, D233H, T243S, M244V, G249D, G250E, G251S, Q252H, Y253F, Y253H, E255K, E255V, V256L, Y257F, Y257R, F259S, K262E, D263G, K264R, S265R, V268A, V270A, T272A, Y274C, Y274R, D276N, T277P, M278K, E279K, E282G, F283S, A288T, A288V, M290T, K291R, E292G, I293T, P296S, L298M, L298P, V299L, Q300R, G303E, V304A, V304D, C305S, C305Y, T306A, F311L, I314V, T3151, T315A, E316G, F317L, F317I, M318T, Y320C, Y320H, G321E, D325H, Y326C, L327P, R328K, E329V, Q333L, A337V, V339G, L342E, M343V, M343T, A344T, A344V, I347V, A350T, M351T, E352A, E352K, E355G, K357E, N358D, N358S, F359V, F359C, F359I, I360K, I360T, L364H, L364I, E373K, N374D, K378R, V379I, A380T, A380V, D381G, F382L, L387M, M388L, T389S, T392A, T394A, A395G, H396K, H396R, A399G, P402T, T406A, S417Y, F486S or any combination thereof, *i.e*., M244V, G250E, Q252H, Q252R, Y253F, Y253H, E255K, E255V, T315I, F317L, M351T, E355G, F359V, H396R, F486S and any combination thereof; M244V, E279K, F359C, F359I, L364I, L387M, F486S and any combination thereof; and L248R, Q252H, E255K, V299L, T315I, F317V, F317L, F317S and any combination thereof.

If an activating BCR-ABL kinase mutation is found in the cells from said individual, treatment regimens can be developed appropriately. For example, an identified mutation can indicate that said cells are or will become at least partially resistant to commonly used kinase inhibitors. For example, a N49S, N53S, C100R, S126P, E138G, N146S, I242T, K271R, E292V, L324Q, V338M, M351A, M458T mutation can indicate that the cells in an individual are or are expected to become at least partially resistant to treatment with a kinase inhibitor such as N-(2-chloro-6-methylphenyl)-2-[[6-[4-(2-hydroxyethyl)-1-piperazinyl]-2-methyl-4-pyrimidinyl]amino]-5-thiazolecarboxamide. As disclosed herein, in such cases, treatment can include the use of an increased dosing frequency or increased dosage of N-(2-chloro-6-methylphenyl)-2-[[6-[4-(2-hydroxyethyl)-1-piperazinyl]-2-methyl-4-pyrimidinyl]amino]-5-thiazolecarboxamide or a salt, hydrate, or solvate thereof, a combination of N-(2-chloro-6-methylphenyl)-2-[[6-[4-(2-hydroxyethyl)-1-piperazinyl]-2-methyl-4-pyrimidinyl] amino]-5-thiazolecarboxamide or a pharmaceutically acceptable salt, hydrate, or solvate thereof and another kinase inhibitor drug such as imatinib, AMN107, PD180970, GGP76030, AP23464, SKI 606, and/or AZD0530; a combination of N-(2-chloro-6-methylphenyl)-2-[[6-[4-(2-hydroxyethyl)-1-piperazinyl]-2-methyl-4-pyrimidinyl]amino]-5-thiazolecarboxamide and a tubulin stabilizing agent (e.g., pacitaxol, epothilone, taxane, etc.); a combination of N-(2-chloro-6-methylphenyl)-2-[[6-[4-(2-hydroxyethyl)-1-piperazinyl]-2-methyl-4-pyrimidinyl]amino]-5-thiazolecarboxamide and a farnysyl transferase inhibitor, any other combination disclosed herein; and any other combination or dosing regimen comprising N-(2-chloro-6-methylphenyl)-2-[[6-[4-(2-hydroxyethyl)-1-piperazinyl]-2-methyl-4-pyrimidinyl]amino]-5-thiazolecarboxamide disclosed herein. In one aspect, an increased level of N-(2-chloro-6-methylphenyl)-2-[[6-[4-(2-hydroxyethyl)-1-piperazinyl]-2-methyl-4-pyrimidinyl]amino]-5-thiazolecarboxamide would be about 10, 20, 30, 40, 50, 60, 70, 80, 90, or 95% more than the typical N-(2-chloro-6-methylphenyl)-2-[[6-[4-(2-hydroxyethyl)-1-piperazinyl]-2-methyl-4-pyrimidinyl]amino]-5-thiazolecarboxamide dose for a particular indication or for individual, or about 1.5x, 2x, 2.5x, 3x, 3.5x, 4x, 4.5x, 5x, 6x, 7x, 8x, 9x, or 10x more N-(2-chloro-6-methylphenyl)-2-[[6-[4-(2-hydroxyethyl)-1-piperazinyl]-2-methyl-4-pyrimidinyl]amino]-5-thiazolecarboxamide than the typical N-(2-chloro-6-methylphenyl)-2-[[6-[4-(2-hydroxyethyl)-1-piperazinyl]-2-methyl-4-pyrimidinyl]amino]-5-thiazolecarboxamide dose for a particular indication or for individual.

Additionally, dosage regimens can be further adapted based upon the presence of additional amino acid mutation in a BCR-ABL kinase and/or the presence of a complex karyotype. As described herein, a mutation in E279K, F359C, F359I, L3641, L387M, F486S, D233H, T243S, M244V, G249D, G250E, G251S, Q252H, Y253F, Y253H, E255K, E255V, V256L, Y257F, Y257R, F259S, K262E, D263G, K264R, S265R, V268A, V270A, T272A, Y274C, Y274R, D276N, T277P, M278K, E279K, E282G, F283S, A288T, A288V, M290T, K291R, E292G, 1293T, P296S, L298M, L298P, V299L, Q300R, G303E, V304A, V304D, C305S, C305Y, T306A, F311L, I314V, T315I, E316G, F317L, M318T, Y320C, Y320H, G321E, D325H, Y326C, L327P, R328K, E329V, Q333L, A337V, V339G, L342E, M343V, M343T, A344T, A344V, 1347V, A350T, M351T, E352A, E352K, E355G, K357E, N358D, N358S, F359V, F359C, F359I, I360K, I360T, L364H, L364I, E373K, N374D, K378R, V379I, A380T, A380V, D381G, F382L, L387M, M388L, T389S, T392A, T394A, A395G, H396K, H396R, A399G, P402T, T406A, S417Y, F486S, or any combination thereof can indicate that the BCR-ABL kinase has developed at least partial resistance to therapy with a protein kinase inhibitor such as imitinab.

In practicing the many aspects of the invention herein, biological samples can be selected from many sources such as tissue biopsy (including cell sample or cells cultured therefrom; biopsy of bone marrow or solid tissue, for example cells from a solid tumor), blood, blood cells (red blood cells or white blood cells), serum, plasma, lymph, ascetic fluid, cystic fluid, urine, sputum, stool, saliva, bronchial aspirate, CSF or hair. Cells from a sample can be used, or a lysate of a cell sample can be used. In certain embodiments, the biological sample is a tissue biopsy cell sample or cells cultured therefrom, for example, cells removed from a solid tumor or a lysate of the cell sample. In certain embodiments, the biological sample comprises blood cells.

Pharmaceutical compositions for use in the present invention can include compositions comprising one or a combination of BCR-ABL inhibitors in an effective amount to achieve the intended purpose. The determination of an effective dose of a pharmaceutical composition of the invention is well within the capability of those skilled in the art. A therapeutically effective dose refers to that amount of active ingredient which ameliorates the symptoms or condition. Therapeutic efficacy and toxicity can be determined by standard pharmaceutical procedures in cell cultures or experimental animals, for example the ED50 (the dose therapeutically effective in 50% of the population) and LD50 (the dose lethal to 50% of the population).

Dosage regimens involving N-(2-chloro-6-methylphenyl)-2-[[6-[4-(2-hydroxyethyl)-1-piperazinyl]-2-methyl-4-pyrimidinyl]amino]-5-thiazolecarboxamide useful in practicing the present invention are described in US Serial No 10/395,503, filed March 24, 2003; and Blood (ASH Annual Meeting Abstracts) 2004, Volume 104: Abstract 20, "Hematologic and Cytogenetic Responses in imatinib-Resistant Accelerated and Blast Phase Chronic Myeloid Leukemia (CML) Patients Treated with the Dual SRC/ABL Kinase Inhibitor N-(2-chloro-6-methylphenyl)-2-[[6-[4-(2-hydroxyethyl)-1-piperazinyl]-2-methyl-4-pyrimidinyl]amino]-5-thiazolecarboxamide: Results from a Phase I Dose Escalation Study.", by Moshe Talpaz, et al.

A "therapeutically effective amount" of an inhibitor of BCR-ABL can be a function of whether a complex karyotype is present. A therapeutically relevant dose of N-(2-chloro-6-methylphenyl)-2-[[6-[4-(2-hydroxyethyl)-1-piperazinyl]-2-methyl-4-pyrimidinyl]amino]-5-thiazolecarboxamide for patients having a complex karyotype, for example, could range anywhere from 1 to 14 fold or more higher than the typical dose. Accordingly, therapeutically relevant doses of N-(2-chloro-6-methylphenyl)-2-[[6-[4-(2-hydroxyethyl)-1-piperazinyl]-2-methyl-4-pyrimidinyl]amino]-5-thiazolecarboxamide for any of the BCR-ABL-associated or protein tyrosine kinase associated disorder in which a complex karyotype is present can be, for example, about 1, 2, 3, 4, 5, 6, 7, 8, 9, 10, 11, 12, 13, 14, 15, 16, 17, 18, 19, 20, 25, 30, 35, 40, 50, 60, 70, 80, 90, 100, 125, 150, 175, 200, 225, 250, or 300 folder higher than the prescribed or standard dose. Alternatively, therapeutically relevant doses of N-(2-chloro-6-methylphenyl)-2-[[6-[4-(2-hydroxyethyl)-1-piperazinyl]-2-methyl-4-pyrimidinyl]amino]-5-thiazolecarboxamide can be, for example, about 0.9x, 0.8x, 0.7x, 0.6x, 0.5x, 0.4x, 0.3x, 0.2x, 0.1x, 0.09x, 0.08x, 0.07x, 0.06x, 0.05x, 0.04x, 0.03x, 0.02x, or 0.01x of the prescribed dose.

According to O'hare et al. (Cancer Res., 65(11):4500-5 (2005)), the M244V mutant had a fold change of "1.3" in the GST-Abl kinase assay, a fold change of "1.1" in the autophosphorylation assay, and a fold change of "2" in the cellular proliferation assay; the G250E mutant had a fold change of "0.5" in the GST-Abl kinase assay, a fold change of "3" in the autophosphorylation assay, and a fold change of "2" in the cellular proliferation assay; the Q252H mutant had a fold change of "4" in the cellular proliferation assay; the Y253F mutant had a fold change of "0.6" in the GST-Abl kinase assay, a fold change of "4" in the autophosphorylation assay, and a fold change of "4" in the cellular proliferation assay; the Y253H mutant had a fold change of "3" in the GST-Abl kinase assay, a fold change of "2" in the autophosphorylation assay, and a fold change of "2" in the cellular proliferation assay; the E255K mutant had a fold change of "0.3" in the GST-Abl kinase assay, a fold change of "2" in the autophosphorylation assay, and a fold change of "7" in the cellular proliferation assay; the F317L mutant had a fold change of "1.5" in the GST-Abl kinase assay, a fold change of "1.4" in the autophosphorylation assay, and a fold change of "9" in the cellular proliferation assay; the M351T mutant had a fold change of "0.2" in the GST-Abl kinase assay, a fold change of "2" in the autophosphorylation assay, and a fold change of "1.4" in the cellular proliferation assay; the F359V mutant had a fold change of "0.8" in the GST-Abl kinase assay, a fold change of "2" in the autophosphorylation assay, and a fold change of "3" in the cellular proliferation assay; the H396R mutant had a fold change of "1.3" in the GST-Abl kinase assay, a fold change of "3" in the autophosphorylation assay, and a fold change of "2" in the cellular proliferation assay.

For patients harboring the T315I mutation, administration of higher doses of N-(2-chloro-6-methylphenyl)-2-[[6-[4-(2-hydroxyethyl)-1-piperazinyl]-2-methyl-4-pyrimidinyl]amino]-5-thiazolecarboxamide, or combinations of N-(2-chloro-6-methylphenyl)-2-[[6-[4-(2-hydroxyethyl)-1-piperazinyl]-2-methyl-4-pyrimidinyl]amino]-5-thiazolecarboxamide and imatinib; a combination of N-(2-chloro-6-methylphenyl)-2-[[6-[4-(2-hydroxyethyl)-1-piperazinyl]-2-methyl-4-pyrimidinyl]amino]-5-thiazolecarboxamide and a tubulin stabilizing agent (e.g., pacitaxol, epothilone, taxane, etc.); a combination of N-(2-chloro-6-methylphenyl)-2-([6-[4-(2-hydroxyethyl)-1-piperazinyl]-2-methyl-4-pyrimidinyl]amino]-5-thiazolecarboxamide and a farnysyl transferase inhibitor, a combination of N-(2-chloro-6-methylphenyl)-2-[[6-[4-(2-hydroxyethyl)-1-piperazinyl]-2-methyl-4-pyrimidinyl]amino]-5-thiazolecarboxamide and another protein tyrosine kinase inhibitor, any other combination discloses herein; an increased dosing frequency regimen of N-(2-chloro-6-methylphenyl)-2-[[6-[4-(2-hydroxyethyl)-1-piperazinyl]-2-methyl-4-pyrimidinyl]amino]-5-thiazolecarboxamide; and any other combination or dosing regimen comprising N-(2-chloro-6-methylphenyl)-2-[[6-[4-(2-hydroxyethyl)-1-piperazinyl]-2-methyl-4-pyrimidinyl]amino]-5-thiazolecarboxamide disclosed herein, may be warranted. Alternatively, combinations of N-(2-chloro-6-methylphenyl)-2-[[6-[4-(2-hydroxyethyl)-1-piperazinyl]-2-methyl-4-pyrimidinyl]amino]-5-thiazolecarboxamide with a T315I inhibitor may also be warranted.

The present invention provides methods of determining responsiveness of an individual having a protein tyrosine kinase-associated disorder to a certain treatment regimen and describes methods of treating an individual having a protein tyrosine kinase-associated disorders.

Disorders included in the scope of the present invention include, for example, leukemias, including, for example, chronic myeloid leukemia (CML), acute lymphoblastic leukemia, and Philadelphia chromosome positive acute lymphoblastic leukemia (Ph+ ALL), squamous cell carcinoma, small-cell lung cancer, non-small cell lung cancer, glioma, gastrointestinal cancer, renal cancer, ovarian cancer, liver cancer, colorectal cancer, endometrial cancer, kidney cancer, prostate cancer, thyroid cancer, neuroblastoma, pancreatic cancer, glioblastoma multiforme, cervical cancer, stomach cancer, bladder cancer, hepatoma, breast cancer, colon carcinoma, and head and neck cancer, gastric cancer, germ cell tumor, pediatric sarcoma, sinonasal natural killer, multiple myeloma, acute myelogenous leukemia, chronic lymphocytic leukemia, mastocytosis and any symptom associated with mastocytosis. In addition, disorders include urticaria pigmentosa, mastocytosises such as diffuse cutaneous mastocytosis, solitary mastocytoma in human, as well as dog mastocytoma and some rare subtypes like bullous, erythrodermic and teleangiectatic mastocytosis, mastocytosis with an associated hematological disorder, such as a myeloproliferative or myelodysplastic syndrome, or acute leukemia, myeloproliferative disorder associated with mastocytosis, and mast cell leukemia. Various additional cancers are also included within the scope of protein tyrosine kinase-associated disorders including, for example, the following: carcinoma, including that of the bladder, breast, colon, kidney, liver, lung, ovary, pancreas, stomach, cervix, thyroid, testis, particularly testicular seminomas, and skin; including squamous cell carcinoma; gastrointestinal stromal tumors ("GIST"); hematopoietic tumors of lymphoid lineage, including leukemia, acute lymphocytic leukemia, acute lymphoblastic leukemia, B-cell lymphoma. T-cell lymphoma, Hodgkins lymphoma, non-Hodgkins lymphoma, hairy cell lymphoma and Burketts lymphoma; hematopoietic tumors of myeloid lineage, including acute and chronic myelogenous leukemias and promyelocytic leukemia; tumors of mesenchymal origin, including fibrosarcoma and rhabdomyoscarcoma; other tumors, including melanoma, seminoma, tetratocarcinoma, neuroblastoma and glioma; tumors of the central and peripheral nervous system, including astrocytoma, neuroblastoma, glioma, and schwannomas; tumors of mesenchymal origin, including fibrosarcoma, rhabdomyoscaroma, and osteosarcoma; and other tumors, including melanoma, xenoderma pigmentosum, keratoactanthoma, seminoma, thyroid follicular cancer, teratocarcinoma, chemotherapy refractory non-seminomatous germ-cell tumors, and Kaposi's sarcoma. In certain preferred embodiments, the disorder is leukemia, breast cancer, prostate cancer, lung cancer, colon cancer, melanoma, or solid tumors. In certain preferred embodiments, the leukemia is chronic myeloid leukemia (CML), Ph+ ALL, AML, imatinib-resistant CML, imatinib-intolerant CML, accelerated CML, lymphoid blast phase CML.

A "solid tumor" includes, for example, sarcoma, melanoma, colon carcinoma, breast carcinoma, prostate carcinoma, or other solid tumor cancer.

The terms "cancer", "cancerous", or "malignant" refer to or describe the physiological condition in mammals that is typically characterized by unregulated cell growth. Examples of cancer include, for example, leukemia, lymphoma, blastoma, carcinoma and sarcoma. More particular examples of such cancers include chronic myeloid leukemia, acute lymphoblastic leukemia, Philadelphia chromosome positive acute lymphoblastic leukemia (Ph+ ALL), squamous cell carcinoma, small-cell lung cancer, non-small cell lung cancer, glioma, gastrointestinal cancer, renal cancer, ovarian cancer, liver cancer, colorectal cancer, endometrial cancer, kidney cancer, prostate cancer, thyroid cancer, neuroblastoma, pancreatic cancer, glioblastoma multiforme, cervical cancer, stomach cancer, bladder cancer, hepatoma, breast cancer, colon carcinoma, and head and neck cancer, gastric cancer, germ cell tumor, pediatric sarcoma, sinonasal natural killer, multiple myeloma, acute myelogenous leukemia (AML), and chronic lymphocytic leukemia (CML).

"Leukemia" refers to progressive, malignant diseases of the blood-forming organs and is generally characterized by a distorted proliferation and development of leukocytes and their precursors in the blood and bone marrow. Leukemia is generally clinically classified on the basis of (1) the duration and character of the disease--acute or chronic; (2) the type of cell involved; myeloid (myelogenous), lymphoid (lymphogenous), or monocytic; and (3) the increase or non-increase in the number of abnormal cells in the blood--leukemic or aleukemic (subleukemic). Leukemia includes, for example, acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia. In certain aspects, the present invention provides treatment for chronic myeloid leukemia, acute lymphoblastic leukemia, and/or Philadelphia chromosome positive acute lymphoblastic leukemia (Ph+ ALL).

### Results of Dasatinib Dose Escalation Clinical Trial

A total of 44 patients with advanced CML or Ph+ ALL with imatinib resistance/intolerance were enrolled into a dose escalation clinical for dasatinib (Table 1). Eleven patients had AP, 23 had MBC, and 10 had LBC or Ph+ ALL. 64% (28/44) of patients had received prior treatment with chemotherapy (excluding hydroxyurea), including 23% (10/44) who had undergone a prior bone marrow or stem cell transplant. Of the 44 patients, 91% (40/44) were resistant to imatinib, and 61% (27/44) were on doses of greater than 600 mg per day. Nine percent (4/44) were intolerant of imatinib, due to LFT abnormalities, rash or bone pain. 61% (27/44) had mutations in *BCR-ABL* detected prior to the start of dasatinib.

The most significant adverse event was myelosuppression. As expected in this patient population, a significant fraction of patients had grade 3 or 4 myelosuppression at the time of study entry (Supplemental Table 2). Nonetheless, Grade 3 or 4 neutropenia occurred on treatment in 9/11 (82%), 22/23 (96%) and 8/10 (80%) patients with AP, MBC, and LBC/Ph+ ALL, respectively (Table 2a). Similarly grade 3/4 thrombocytopenia occurred in 9/11 (82%), 19/23 (83%), and 7/10 (70%) in AP, MBC and LBC/Ph+ ALL, respectively. Myelosuppression was generally reversible, managed by dose interruption or reduction and was no longer an issue in patients who recovered Ph negative hematopoiesis.

Grade 3/4 non-hematologic adverse events occurred in 20% (9/44) of patients and included pleural or pericardial effusion, dyspnea, tumor lysis syndrome and rectal hemorrhage (Table 2b). These events were managed with supportive care and, in some cases, dose interruption and reduction. Additional grade 1-2 adverse events included diarrhea, rash, flushing and headache. Toxicities did not appear to be dose-related since similar adverse events occurred in all dose cohorts, but such conclusions are limited by the fact that some patients were dose escalated. Importantly, imatinib-intolerant patients were able to tolerate dasatinib and no patient was removed from the study due to a drug-related toxicity. A maximum tolerated dose was not defined.

Patients with AP, MBC, LBC and Ph+ ALL were treated for a median (range) of 5 (1.3-12.9), 5 (0.2-12), and 3 (0.5-7.7) months, respectively. The number of patients in each dasatinib dose cohort was 1 (35 mg), 8 (50 mg), 17 (70 mg), 11 (90 mg) and 7 (120 mg), although some patients underwent dose escalation after 4 weeks. Major HR was achieved in 70% (31/44) of the total patient population, with CHR in 45% (20/44) of patients (Table 3). 57% (25/44) of patients achieved a CyR. A CCyR was achieved in 25% (11/44) of patients and a major CyR (CCyR plus PCyR) was achieved in 43% (19/44) of patients. Responses were observed in all dose cohorts.

In AP patients, 9/11 (81%) achieved a major HR, 6 (55%) of which were confirmed after 4 weeks. Major cytogenetic responses occurred in 3/11 (27%) AP patients. In MBC patients, 14/23 (61%) achieved a major HR, 7 of which (30%) were confirmed after 4 weeks. Cytogenetic responses were observed in 12/23 (52%) of MBC patients. The major HR rate in LBC/Ph+ ALL was 8/10 (80%) of which 5 (50%) were confirmed after 4 weeks. LBC/Ph+ALL patients had a MCyR rate of 8/10 (80%) and overall cytogenetic response rate of 9/10 (90%).

Response duration varied between AP, MBC and LBC/Ph+ ALL patients, with relapses occurring in all three groups (Fig 1). In the AP cohort, 9/11 (81%) of patients who achieved a major HR remain on study for a median of 8 months (range 2-13 months). A larger fraction of MBC patients have relapsed, but 6 of 23 (26%) remain on the study with follow-up ranging 5 - 12 months, including three who continue to have a CCyR at 10, 11 and 12 months each. As with AP patients, MBC patients who achieve major HR have done well with a median follow-up of 5 months (range 1-12 months). In contrast, all but one of the LBC/Ph+ ALL patients have relapsed, despite the high major HR rate, with a median follow-up of 4 months (range 1-8 months).

### Molecular Correlates of Resistance to Dasatinib

Nine of the 44 patients (20%) failed to meet the criteria for major or minor hematologic response. To gain insight into molecular determinants of non-response to dasatinib, BCR-ABL kinase inhibition, as determined by phosphorylation of the substrate CrkI, was measured in these patients. As expected, CRKL phosphorylation was substantially reduced at 4 hours in a responding patient (AP-7) with no detectable *BCR-ABL* mutation (Fig 2a). Two of the non-responder patients (AP-3 and AP-8) had the T315I *BCR-ABL* mutation, which confers resistance to imatinib and dasatinib in vitro. Dasatinib had no effect on Crkl phosphorylation in these T315I patients (Fig 2a), despite adequate serum levels (Fig 2b). Of note, SRC kinase activity, as measured using a phosphorylation-specific antibody that detects active SRC, was inhibited comparably in the wild-type and non-responder T315I patient (Fig 2a), suggesting that SRC kinase inhibition was insufficient to induce clinical response in these patients.

The mechanism of non-response in the non-T315I patients is less clear. While all but one had other imatinib resistance mutations, these are unlikely to be responsible for treatment failure because Crk1 phosphorylation was inhibited in all of them on whom samples were available (Fig 2c). Furthermore, chronic phase patients with these same pretreatment mutations have responded to dasatinib (Talpaz et al, submitted). Upon closer examination of the peripheral blood blast counts during the initial 2 weeks of therapy, it is apparent that dasatinib had anti-leukemic activity in some of these non-responders, but was insufficient to achieve a hematologic response (Fig 2d). In one patient with an E255K mutation (ALL-4), T315I is still the likely explanation of treatment failure because disease progression occurred after a transient fall in blood counts, followed by relapse with a subclone bearing only the T315I mutation.

Since T315I can only account for a subset of non-responders, we considered the potential role of additional non-Ph chromosomal abnormalities. Overall; 25/43 patients had complex karyotypes, 17 of whom responded (68%). 18/43 patients had simple Ph karyotypes, 17 of whom responded (94%) (p=0.049). Remarkably, all but one of the non-responders had complex karyotypes, the single exception being a T315I patient.

### DISCUSSION

The major limitation of imatinib as treatment for advanced phase CML is the rapid acquisition of resistance, most often due to the outgrowth of CML subclones harboring mutations in the kinase domain of *BCR-ABL* that interfere with imatinib binding. Here we show that dasatinib has single agent activity in imatinib-resistant advanced phase CML, including those with a broad range of imatinib-resistant *BCR-ABL* mutations. These significant response rates confirm the prediction that imatinib resistance is driven by reactivation of BCR-ABL activity (7). As in the early imatinib trials in advanced phase CML, response duration varied significantly between AP patients and those in blast crisis. With dasatinib, AP responses are durable with a maximal follow-up of 13 months, whereas all but one patient with lymphoid blast crisis or Ph+ ALL relapsed within 6 months. Myeloid BC responders also relapse, but 3 of the 8 patients who achieved CHR have had durable responses for 10 months or longer and remain on dasatinib. Based on these findings, phase II studies of dasatinib in patients with imatinib-resistant advanced phase CML are currently underway.

The major complication associated with dasatinib in advanced phase CML was severe (grade 4) myelosuppression in about 70% of patients, which is higher than that reported previously with imatinib in phase I/II studies (30-50%) (2, 4). While this difference may not prove significant in larger studies, there are several reasons why dasatinib could be more myelosuppressive than imatinib. Dasatinib is several hundred times more potent and, unlike imatinib, is not a substrate for p-glycoprotein; therefore, dasatinib may reach higher concentrations in early hematopoietic progenitor and stem cell populations. Additionally, dasatinib could lead to more rapid clearance of Ph-chromosome-containing cells, thereby unmasking cytopenias in a high proportion of patients. Dasatinib does not appear to be myelosuppressive against normal hematopoietic cells since patients who achieved cytogenetic remissions often recovered normal blood counts, including several prior allograft recipients who rapidly reverted to donor hematopoiesis after initiating dasatinib therapy. Furthermore, myelosuppression has not been a complication of dasatinib treatment in patients with solid tumors (25).

Clinically significant edema syndromes occurred in some patients, but appear clinically distinct from imatinib-related edema. For example, non-malignant pleural effusions developed in 10 patients, often in the absence of other edema, whereas the common imatinib-related side effect of periorbital edema was less frequent. Indeed several patients had resolution of generalized edema upon switching from imatinib to dasatinib at study entry. Other common imatinib-associated side effects such as muscle cramps and nausea were rarely observed. It is also notable that prolonged treatment with a pan-SRC kinase inhibitor did not cause clinically significant immune dysfunction, as might be predicted from mouse knockout models (18).

The correlation of *BCR-ABL* genotype with clinical response to dasatinib mirrors predictions from preclinical findings, in that the T315I mutation confers primary resistance. In addition, T315I is likely to be responsible for a large fraction of acquired resistance based on preliminary analysis of patients in this study who relapsed (N. Shah, unpublished findings). Additional mechanisms clearly play a role since several BC patients with presumably dasatinib-sensitive *BCR-ABL* mutations failed to respond. Pharmacodynamic studies in these patients demonstrated inhibition of BCR-ABL kinase activity on day one of treatment, confirming that these mutations are indeed sensitive to dasatinib at a biochemical level. The fact that these patients failed to respond clinically raises new questions about other molecular determinants of resistance, including the possibility of BCR-ABL independent CML, as has recently been described in one patient (19). All these patients had complex karyotypes; therefore, secondary genetic abnormalities seem likely to play a role. Precedent comes from recent studies of glioblastoma patients with *VIII EGFR* mutations, showing that secondary mutations in the *PTEN* tumor suppressor gene confer primary resistance to EGFR inhibitors (20).

These initial clinical findings with dasatinib in CML could provide insight into a broader strategy for developing kinase inhibitor cancer therapy. Acquired resistance is a growing problem with other cancers treated with kinase inhibitors, such as lung cancer and GIST, all of which share a common mechanism of resistance through mutation of the target kinase (21-24). Dasatinib offers a compelling solution to imatinib resistance in CML based on the molecular details underlying its binding to the BCR-ABL kinase domain. Unlike imatinib which binds exclusively to the inactive BCR-ABL conformation, dasatinib binding is conformation-tolerant and is relatively unaffected by imatinib-resistance mutations. A general theme might be that combinations of kinase inhibitors, which bind to distinct conformations, could be used sequentially or in combination to delay the emergence of resistance. One significant weakness of both dasatinib and imatinib is the failure of both of these compounds to inhibit the T315I BCR-ABL mutant. Therefore, effective long-term control of advanced CML is likely to require a third agent with activity against this so called "gatekeeper" mutation. However, the efficacy of dasatinib even in a genetically complex cancer such as blast crisis CML reinforces the therapeutic potential of targeting critical genetic lesions.

### Karyotype Detection Method

The invention provides methods of screening a biological sample from an individual suffering from a BCR-ABL- or protein tyrosine kinase-associated disorder for the presence of a complex karyotype, as well as methods for treating individuals who are identified as having a complex karyotype.

Methods of determining whether a patient has a complex karyotype are known in the art, particularly in the cytogenetics arts, including, but not limited to chromosome plating, staining, FISH, gimsa staining, chromosome staining using fluorescent dyes or probes, etc. Standard molecular biology techniques are contemplated for determining whether a complex karyotype is present in the cells of a given individual. Many kits are available for karyotyping, including those designed for peripheral blood lymphocyte karyotyping including those from Biological Industries (Israel), Catalog No. 01-198-1 using the methods outlined with the kit, for example.

The solid substrate is typically glass or a polymer, the most commonly used polymers being cellulose, polyacrylamide, nylon, polystyrene, polyvinyl chloride or polypropylene. The solid supports may be in the form of tubes, beads, discs or microplates, or any other surface suitable for conducting an immunoassay. The binding processes are well-known in the art and generally consist of cross-linking covalently binding or physically adsorbing the molecule to the insoluble carrier.

Additionally, the invention provides assays for the detection of a complex karyotype in a biological sample, such as cell preparations, and the like. A number of methods for amplifying and/or detecting the presence of a complex karyotype are well known in the art and can be employed in the practice of this aspect of the invention.

The invention also provides assays for detecting the presence of a mutant BCR-ABL kinase protein in a biological sample. The results of such an assay may be important in establishing a treatment regimen for a patient, either alone or in conjunction with the identification of a complex karyotype, since certain BCR-ABL mutations confer resistance to protein tyrosine kinase inhibitors. Methods for detecting a mutant BCR-ABL kinase protein are also well known and include, for example, immunoprecipitation, immunohistochemical analysis, Western Blot analysis, molecular binding assays, ELISA, ELIFA and the like. For example, in one embodiment, a method of detecting the presence of a mutant BCR-ABL kinase protein in a biological sample comprises first contacting the sample with a BCR-ABL antibody, a mutant BCR-ABL kinase-reactive fragment thereof, or a recombinant protein containing an antigen binding region of a mutant BCR-ABL kinase antibody; and then detecting the binding of mutant BCR-ABL kinase protein in the sample thereto.

### Polypeptides, Polynucleotides, and Antibodies

The present invention provides isolated novel BCR-ABL nucleotides and their encoded proteins having mutations at certain amino acids that can render an individual at least partially resistant to therapy with N-(2-chloro-6-methylphenyl)-2-[[6-[4-(2-hydroxyethyl)-1-piperazinyl]-2-methyl-4-pyrimidinyl] amino]-5-thiazolecarboxamide or a pharmaceutically acceptable salt of hydrate thereof. At least one of the mutations is a N49S, N53S, C100R, S126P, E138G, N146S, I242T, K271R, E292V, L324Q, V338M, M351A, M458T mutation.

The single letter amino acid sequence of wild-type human BCR-ABL protein shown is known in the art and provided as SEQ ID NO:2. The nucleic acid sequence of BCR-ABL is encoded by nucleotides 1 to 3681 of SEQ ID NO:1.

For purposes of shorthand designation of the mutant variants described herein, it is noted that numbers refer to the amino acid residue position along the amino acid sequence of the BCR-ABL polypeptide as provided as SEQ ID NO:2. For example, L324 refers to the amino acid leucine at position 324. Amino acid substitutions at a particular position are written as the wild type amino acid, position number, and amino acid substituted therein, in that order. For example, L324Q refers to a substitution of glutamine for leucine at position 324. Amino acid identification uses the single-letter alphabet of amino acids, as shown in Table 1 below.

**Table 1**

| | | | | | | |
|---|---|---|---|---|---|---|
| Asp | D | Aspartic acid | | Ile | I | Isoleucine |
| Thr | T | Threonine | | Leu | L | Leucine |
| Ser | S | Serine | | Tyr | Y | Tyrosine |
| Glu | E | Glutamic acid | | Phe | F | Phenylalanine |
| Pro | P | Proline | | His | H | Histidine |
| Gly | G | Glycine | | Lys | K | Lysine |
| Ala | A | Alanine | | Arg | R | Arginine |
| Cys | C | Cysteine | | Trp | W | Tryptophan |
| Val | V | Valine | | Gln | Q | Glutamine |
| Met | M | Methionine | | Asn | N | Asparagine |

Accordingly the present invention provides isolated novel BCR-ABL polypeptides comprising the amino acid sequence set forth in SEQ ID NO:2 or having substantial identity to the amino acid sequence set forth in SEQ ID NO:2 and having at least one mutation selected from the group consisting of: N49S, N53S, C100R, S126P, E138G, N146S, I242T, K271R, E292V, L324Q, V338M, M351A, M458T mutation, and fragments thereof. The present invention also provides polypeptides having at least a N49S, N53S, C100R, S126P, E138G, N146S, I242T, K271R, E292V, L324Q, V338M, M351A, M458T mutation and one or more of the following mutations or any combination thereof: E279K, F359C, F359I, L364I, L387M, F486S, D233H, T243S, M244V, G249D, G250E, G251S, Q252H, Y253F, Y253H, E255K, E255V, V256L, Y257F, Y257R, F259S, K262E, D263G, K264R, S265R, V268A, V270A, T272A, Y274C, Y274R, D276N, T277P, M278K, E279K, E282G, F283S, A288T, A288V, M290T, K291R, E292G, I293T, P296S, L298M, L298P, V299L, Q300R, G303E, V304A, V304D, C305S, C305Y, T306A, F311L, I314V, T315I, E316G, F317L, M318T, Y320C, Y320H, G321E, D325H, Y326C, L327P, R328K, E329V, Q333L, A337V, V339G, L342E, M343V, M343T, A344T, A344V, I347V, A350T, M351T, E352A, E352K, E355G, K357E, N358D, N358S, F359V, F359C, F359I, I360K, I360T, L364H, L364I, E373K, N374D, K378R, V379I, A380T, A380V, D381G, F382L, L387M, M388L, T389S, T392A, T394A, A395G, H396K, H396R, A399G, P402T, T406A, S417Y, or F486S, including for example, M244V, G250E, Q252H, Q252R, Y253F, Y253H, E255K, E255V, T3151, F317L, M351T, E355G, F359V, H396R, F486S; M244V, E279K, F359C, F3591, L3641, L387M, F486S and any combination thereof; and L248R, Q252H, E255K, V299L, T3151, F317V, F317L, F317S and any combination thereof.

The present invention also describes conservatively modified variants of SEQ ID NO:2 having at least a N49S, N53S, C100R, S126P, E138G, N146S, I242T, K271R, E292V, L324Q, V338M, M351A, and/or M458T mutation, and fragments thereof.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence with respect to the expression product, but not with respect to actual probe sequences. As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles described herein.

The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, *e.g*., Creighton, Proteins (1984)).

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

As used herein, the term "polynucleotide" means a polymeric form of nucleotides of at least about 10 bases or base pairs in length, either ribonucleotides or deoxynucleotides or a modified form of either type of nucleotide, and is meant to include single and double stranded forms of DNA.

As used herein, a polynucleotide is said to be "isolated" when it is substantially separated from contaminant polynucleotides that correspond or are complementary to genes other than, the Bcr-Abl gene or mutants thereof. As used herein, a polypeptide is said to be "isolated" when it is substantially separated from contaminant polypeptide that correspond to polypeptides other than the BCR-ABL peptide or mutant polypeptides or fragments thereof. A skilled artisan can readily employ polynucleotide or polypeptide isolation procedures well known in the art to obtain said isolated polynucleotides and/or polypeptides.

As used herein "substantial identity" to a specified sequence refers to 80% identity or greater, *i.e*., 81%, 82%, 83%, 84%, 85%, 86%, 87%, 88%, 89%, 90%, 91%, 91%, 93%, 94%, 95%, 96%, 97%, 98%, o99%, 99.5% or 99.9% identity to the specified sequence.

In the context of amino acid sequence comparisons, the term "identity" is used to identify and express the percentage of amino acid residues at the same relative positions that are the same. Also in this context, the term "homology" is used to identify and express the percentage of amino acid residues at the same relative positions that are either identical or are similar, using the conserved amino acid criteria of BLAST analysis, as is generally understood in the art. For example, identity and homology values can be generated by WU-BLAST-2 (Altschul et al., Methods in Enzymology, 266: 460-480 (1996): http://blast.wustl/edu/blast/README.html).

"Percent (%) amino acid sequence identity" with respect to the sequences identified herein is defined as the percentage of amino acid residues in a candidate sequence that are identical with the amino acid residues in the BCR-ABL sequence, after aligning the sequences and introducing gaps, if necessary, to achieve the maximum percent sequence identity. Alignment for purposes of determining percent amino acid sequence identity can be achieved in various ways that are within the skill in the art can determine appropriate parameters for measuring alignment, including assigning algorithms needed to achieve maximal alignment over the full-length sequences being compared. For purposes herein, percent ammo acid identity values can also be obtained using the sequence comparison computer program, ALIGN-2, the source code of which has been filed with user documentation in the US Copyright Office, Washington, D.C., 20559, registered under the US Copyright Registration No. TXU510087. The ALIGN-2 program is publicly available through Genentech, Inc., South San Francisco, Calif. All sequence comparison parameters are set by the ALIGN-2 program and do not vary.

The polynucleotides described herein are useful for a variety of purposes, including, for example, their use in the detection of the gene(s), mRNA(s), or fragments thereof; as reagents for the diagnosis and/or prognosis of BCR-ABL associated disorders, including cancers; as coding sequences capable of directing the expression of their encoded polypeptides; and as tools for modulating or inhibiting the function of the encoded protein.

Further specific embodiments of this aspect include primers and primer pairs, which allow the specific amplification of the polynucleotides described herein or of any specific parts thereof, and probes that selectively or specifically hybridize to nucleic acid molecules described herein or to any part thereof. Probes can be labeled with a detectable marker, such as, for example, a radioisotope, fluorescent compound, bioluminescent compound, a chemiluminescent compound, metal chelator or enzyme. Such probes and primers can be used to detect the presence of a polynucleotide described herein in a sample and as a means for detecting a cell expressing a protein described herein.

As used herein, the terms "hybridize", "hybridizing", "hybridizes" and the like, used in the context of polynucleotides, are meant to refer to conventional hybridization conditions, preferably such as hybridization in 50% formamide/6xSSC/0.1% SDS/100 µg/ml ssDNA, in which temperatures for hybridization are above 37°C and temperatures for washing in 0.1xSSC/0.1% SDS are above 55°C, and most preferably to stringent hybridization conditions.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature that can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", are known to those of skill in the art and as defined herein, can be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1 % bovine serum albumin/0.1% Ficoll/0.1% polyvinylpyrrolidone/50 mM sodium phosphate buffer at pH 6.5 with 750 mM sodium chloride, 75 mM sodium citrate at 42°C; or (3) employ 50% formamide, 5xSSC (0.75 M NaCl, 0.075 M sodium citrate), 50 mM sodium phosphate (pH 6.8), 0.1% sodium pyrophosphate, 5xDenhardt's solution, sonicated salmon sperm DNA (50 µg/ml), 0.1% SDS, and 10% dextran sulfate at 42°C, with washes at 42°C in 0.2xSSC (sodium chloride/sodium. citrate) and 50% formamide at 55°C, followed by a high-stringency wash consisting of 0.1xSSC containing EDTA at 55°C.

"Moderately stringent conditions" can be identified as described by Sambrook et al., 1989, Molecular Cloning: A Laboratory Manual, New York: Cold Spring Harbor Press, and include the use of washing solution and hybridization conditions (e.g., temperature, ionic strength and %SDS) less stringent than those described above. A non-limiting example of moderately stringent conditions is overnight incubation at 37°C in a solution comprising: 20% formamide, 5xSSC (150 mM NaCl, 15 mM trisodium citrate), 50 mM sodium phosphate (pH 7.6), 5x Denhardt's solution, 10% dextran sulfate, and 20 mg/mL denatured sheared salmon sperm DNA, followed by washing the filters in 1xSSC at about 37-50°C. The skilled artisan will recognize how to adjust the temperature, ionic strength, etc. as necessary to accommodate factors such as probe length and the like.

The invention also describes recombinant DNA or RNA molecules comprising a polynucleotide described herein, including, for example, phages, plasmids, phagemids, cosmids, YACs, BACs, as well as various viral and non-viral vectors well known in the art, and cells transformed or transfected with such recombinant DNA or RNA molecules. As used herein, a recombinant DNA or RNA molecule is a DNA or RNA molecule that has been subjected to molecular manipulation in vitro. Methods for generating such molecules are well known (see, for example, Sambrook et al, 1989, supra).

The invention further describes a host-vector system comprising a recombinant DNA molecule containing a polynucleotide described herein within a suitable prokaryotic or eukaryotic host cell. Examples of suitable eukaryotic host cells include a yeast cell, a plant cell, or an animal cell, such as a mammalian cell or an insect cell (e.g., a baculovirus-infectible cell such as an Sf9 cell). Examples of suitable mammalian cells include various cancer cell lines, other transfectable or transducible cell lines, including those mammalian cells routinely used for the expression of recombinant proteins (e.g., COS, CHO, 293, 293T cells and the like). More particularly, a polynucleotide encoding a mutant BCR-ABL described herein can be used to generate proteins or fragments thereof using any number of host vector systems routinely used and widely known in the art. Cell lines comprising the BCR-ABL polypeptides and BCR-ABL polynucleotides described herein are provided herein.

Proteins encoded by the genes described herein, or by fragments thereof, have a variety of uses, including, for example, generating antibodies and in methods for identifying ligands and other agents (*e.g*. small molecules such as 2-phenylpyrimidines) and cellular constituents that bind to a gene product. Antibodies raised against a BCR-ABL mutant protein or fragment thereof are useful in diagnostic and prognostic assays, imaging methodologies (including, particularly, cancer imaging), and therapeutic methods in the management of human cancers characterized by expression of a protein described herein, including, for example, cancer of the lymphoid lineages. Various immunological assays useful for the detection of proteins described herein are contemplated, including, for example, various types of radioimmunoassays, enzyme-linked immunosorbent assays (ELISA), enzyme-linked immunofluorescent assays (ELIFA), immunocytochemical methods, and the like. Such antibodies can be labeled and used as immunological imaging reagents capable of detecting leukemia cells (e.g., in radioscintigraphic imaging methods).

A wide range of host vector systems suitable for the expression of mutant proteins or fragments thereof are available, see for example, Sambrook et al., 1989, supra; Current Protocols in Molecular Biology, 1995, supra). Vectors for mammalian expression include, for example, pcDNA 3.1 myc-His-tag (Invitrogen) and the retroviral vector pSR.alpha.tkneo (Muller et al., 1991, MCB 11:1785). Using these expression vectors, the polypeptides described herein can be preferably expressed in cell lines, including for example CHO COS, 293, 293T, rat-1, 3T3 etc. The host vector systems described herein are useful for the production of a mutant protein or fragment thereof. Such host-vector systems can be employed to study the functional properties of the proteins.

The present invention describes antibodies that can specifically bind with the polypeptides described herein. The term "antibody" is used in the broadest sense and specifically covers monoclonal antibodies, polyclonal antibodies, antibody compositions with polyepitopic specificity, bispecific antibodies, diabodies, chimeric, single-chain, and humanized antibodies, as well as antibody fragments (*e.g*., Fab, F(ab')₂, and Fv), so long as they exhibit the desired biological activity. Antibodies can be labeled for use in biological assays (*e.g*., radioisotope labels, fluorescent labels) to aid in detection of the antibody.

Antibodies that bind to mutant polypeptides can be prepared using, for example, intact polypeptides or fragments containing small peptides of interest, which can be prepared recombinantly for use as the immunizing antigen. The polypeptide or oligopeptide used to immunize an animal can be derived from the transition of RNA or synthesized chemically, and can be conjugated to a carrier protein, if desired. Commonly used carriers that are chemically coupled to peptides include, for example, bovine serum albumin (BSA), keyhole limpet hemocyanin (KLH), and thyroglobulin. The coupled peptide is then used to immunize the animal (e.g, a mouse, a rat, or a rabbit).

The term "antigenic determinant" refers to that portion of a molecule that makes contact with a particular antibody (i.e., an epitope). When a protein or fragment of a protein is used to immunize a host animal, numerous regions of the protein can induce the production of antibodies which bind specifically to a given region or three-dimensional structure on the protein; each of these regions or structures is referred to as an antigenic determinant. An antigenic determinant can compete with the intact antigen (i.e., the immunogen used to elicit the immune response) for binding to an antibody.

The phrase "specifically binds to" refers to a binding reaction which is determinative of the presence of a target in the presence of a heterogeneous population of other biologics. Thus, under designated assay conditions, the specified binding region bind preferentially to a particular target and do not bind in a significant amount to other components present in a test sample. Specific binding to a target under such conditions can require a binding moiety that is selected for its specificity for a particular target. A variety of assay formats can be used to select binding regions that are specifically reactive with a particular analyte. Typically a specific or selective reaction will be at least twice background signal or noise and more typically more than 10 times background. For purposes of the present invention, compounds, for example small molecules, can be considered for their ability to specifically bind to mutants described herein.

"Imatinib-resistant BCR-ABL mutation" refers to a specific mutation in the amino acid sequence of BCR-ABL that confers upon cells that express said mutation resistance to treatment with imatinib. As discussed herein such mutations can include mutations at the T315I position of BCR-ABL. Additional mutations that may render a BCR-ABL protein at least partially imatinib resistant can include, for example, E279K, F359C, F359I, L364I, L387M, F486S, D233H, T243S, M244V, G249D, G250E, G251S, Q252H, Y253F, Y253H, E255K, E255V, V256L, Y257F, Y257R, F259S, K262E, D263G, K264R, S265R, V268A, V270A, T272A, Y274C, Y274R, D276N, T277P, M278K, E279K, E282G, F283S, A288T, A288V, M290T, K291R, E292G, I293T, P296S, L298M, L298P, V299L, Q300R, G303E, V304A, V304D, C305S, C305Y, T306A, F311L, I314V, T315I, E316G, F317L, M318T, Y320C, Y320H, G321E, D325H, Y326C, L327P, R328K, E329V, Q333L, A337V, V339G, L342E, M343V, M343T, A344T, A344V, 1347V, A350T, M351T, E352A, E352K, E355G, K357E, N358D, N358S, F359V, F359C, F359I, I360K, I360T, L364H, L364I, E373K, N374D, K378R, V379I, A380T, A380V, D381G, F382L, L387M, M388L, T389S, T392A, T394A, A395G, H396K, H396R, A399G, P402T, T406A, S417Y, and F486S, in addition to the mutations described herein including N49S, N53S, C100R, S126P, E138G, N146S, I242T, K271R, E292V, L324Q, V338M, M351A, M458T.

"N-(2-chloro-6-methylphenyl)-2-[[6-[4-(2-hydroxyethyl)-1-piperazinyl]-2-methyl-4-pyrimidinyl]amino]-5-thiazolecarboxamide-resistant BCR-ABL mutation" refers to a specific mutation in the amino acid sequence of BCR-ABL that confers upon cells that express said mutation resistance to treatment with N-(2-chloro-6-methylphenyl)-2-[[6-[4-(2-hydroxyethyl)-1-piperazinyl]-2-methyl-4-pyrimidinyl]amino]-5-thiazolecarboxamide. As discussed herein such mutations can include the N49S, N53S, C100R, S126P, E138G, N146S, I242T, K271R, E292V, L324Q, V338M, M351A, M458T mutations. Additional mutations that render a BCR-ABL protein at least partially N-(2-chloro-6-methylphenyl)-2-[[6-[4-(2-hydroxyethyl)-1-piperazinyl]-2-methyl-4-pyrimidinyl]amino]-5-thiazolecarboxamide resistant include, for example, T315I.

"Imatinib-resistant CML" refers to a CML in which the cells involved in CML are resistant to treatment with imatinib. Generally it is a result of a mutation in BCR-ABL.

"Imatinib-intolerant CML" refers to a CML in which the individual having the CML is intolerant to treatment with imatinib, *i.e*., the toxic and/or detrimental side effects of imatinib outweigh any therapeutically beneficial effects.

### BCR-ABL Mutant Detection Methods

The invention describes methods of screening a biological sample from an individual for the presence of at least one mutation in the BCR-ABL kinase sequence, as well as methods for identifying a cell that expresses mutant BCR-ABL kinase.

Methods of identifying the amino acid and nucleic acid sequence of a wild-type or mutant BCR-ABL polynucleotide or BCR-ABL polypeptide are known in the art. Standard molecular biology techniques are contemplated for precisely determining a BCR-ABL mutation in the cells of a given individual.

Antibodies that immunospecifically bind to a mutant BCR-ABL kinase can be used in identifying one or more of the BCR-ABL mutants described herein. Contemplated herein are antibodies that specifically bind to a mutant BCR-ABL kinase described herein and that do not bind (or bind weakly) to wild type BCR-ABL protein or polypeptides. Anti-mutant BCR-ABL kinase antibodies include, for example, monoclonal and polyclonal antibodies as well as fragments containing the antigen binding domain and/or one or more complementarity determining regions of these antibodies.

For some applications, it may be desirable to generate antibodies which specifically react with a particular mutant BCR-ABL kinase protein and/or an epitope within a particular structural domain. For example, antibodies useful for diagnostic purposes can be those which react with an epitope in a mutated region of the BCR-ABL protein as expressed in cancer cells. For example, antibodies that bind specifically to a F317I and/or T315A mutant BCR-ABL kinase. Such antibodies can be generated by using the mutant BCR-ABL kinase protein described herein, or using peptides derived from various domains thereof, as an immunogen.

Mutant BCR-ABL kinase antibodies described herein can be particularly useful in cancer (e.g., chronic myeloid leukemia, acute lymphoblastic leukemia, Philadelphia chromosome positive acute lymphoblastic leukemia (Ph+ ALL, GIST)) therapeutic strategies, diagnostic and prognostic assays, and imaging methodologies. Similarly, such antibodies can be useful in the diagnosis, and/or prognosis of other cancers, to the extent such mutant BCR-ABL kinase is also expressed or overexpressed in other types of cancer. The invention describes various immunological assays useful for the detection and quantification of mutant BCR-ABL kinase proteins and polypeptides. Such assays generally comprise one or more mutant BCR-ABL kinase antibodies capable of recognizing and binding a mutant BCR-ABL kinase protein, as appropriate, and can be performed within various immunological assay formats well known in the art, including, for example, various types of radioimmunoassays, enzyme-linked immunosorbent assays (ELISA), enzyme-linked immunofluorescent assays (ELIFA), and the like. In addition, immunological imaging methods capable of detecting cancer cells are also described by the invention including, for example, imaging methods using labeled mutant BCR-ABL kinase antibodies. Such assays can be used clinically in the detection, monitoring, and prognosis of cancers.

Accordingly, the present invention describes methods of assaying for the presence of a mutant BCR-ABL polypeptide described herein. By way of example only, in certain embodiments, an antibody raised against the fragment, or other binding moiety capable of specifically binding to the target analyte, is immobilised onto a solid substrate to form a first complex and a biological test sample from a patient is brought into contact with the bound molecule. After a suitable period of incubation, for a period of time sufficient to allow formation of an antibody-secondary complex, a second antibody labelled with a reporter molecule capable of producing a detectable signal is then added and incubated, allowing sufficient time for the formation of a tertiary complex. Any unreacted material is washed away, and the presence of the tertiary complex is determined by observation of a signal produced by the reporter molecule. The results may either be qualitative, by simple observation of the visible signal or may be quantitated by comparison with a control sample containing known amounts of hapten. Variations of this assay include a simultaneous assay, in which both sample and labelled antibody are added simultaneously to the bound antibody, or a reverse assay in which the labelled antibody and sample to be tested are first combined, incubated and then added simultaneously to the bound antibody. These techniques are well known to those skilled in the art, and the possibility of variations will be readily apparent.

By "reporter molecule", as used in the present specification, is meant a molecule which, by its chemical nature, produces an analytically identifiable signal which allows the detection of antigen-bound antibody. Detection may be either qualitative or quantitative. The most commonly used reporter molecule in this type of assay are either enzymes, fluorophores or radionuclide containing molecules (i.e. radioisotopes).

The solid substrate is typically glass or a polymer, the most commonly used polymers being cellulose, polyacrylamide, nylon, polystyrene, polyvinyl chloride or polypropylene. The solid supports may be in the form of tubes, beads, discs or microplates, or any other surface suitable for conducting an immunoassay. The binding processes are well-known in the art and generally consist of cross-linking covalently binding or physically adsorbing the molecule to the insoluble carrier.

The expression profiles of mutant BCR-ABL kinases can be used as diagnostic markers for disease states. The status of mutant BCR-ABL kinase gene products in patient samples can be analyzed by a variety protocols that are well known in the art including the following non-limiting types of assays: PCR-free genotyping methods, Single-step homogeneous methods, Homogeneous detection with fluorescence polarization, Pyrosequencing, "Tag" based DNA chip system, Bead-based methods, fluorescent dye chemistry, Mass spectrometry based genotyping assays, TaqMan genotype assays, Invader genotype assays, microfluidic genotype assays, immunohistochemical analysis, the variety of Northern blotting techniques including in situ hybridization, RT-PCR analysis (for example on laser capture micro-dissected samples), western blot analysis, tissue array analysis, and any other methods known in the art or described elsewhere herein.

Specifically encompassed by the present invention are the following, non-limiting genotyping methods: Landegren, U., Nilsson, M. & Kwok, P. Genome Res 8, 769-776 (1998); Kwok, P., Pharmacogenomics 1, 95-100 (2000); Gut, I., Hum Mutat 17, 475-492 (2001); Whitcombe, D., Newton, C. & Little, S., Curr Opin Biotechnol 9, 602-608 (1998); Tillib, S. & Mirzabekov, A., Curr Opin Biotechnol 12, 53-58 (2001); Winzeler, E. et al., Science 281, 1194-1197 (1998); Lyamichev, V. et al., Nat Biotechnol 17, 292-296 (1999); Hall, J. et al., Proc Natl Acad Sci U S A 97, 8272-8277 (2000); Mein, C. et al., Genome Res 10, 333-343 (2000); Ohnishi, Y. et al., J Hum Genet 46, 471-477 (2001); Nilsson, M. et al., Science 265, 2085-2088 (1994); Baner, J., Nilsson, M., Mendel-Hartvig, M. & Landegren, U., Nucleic Acids Res 26, 5073-5078 (1998); Baner, J. et al., Curr Opin Biotechnol 12, 11-15 (2001); Hatch, A., Sano, T., Misasi, J. & Smith, C., Genet Anal 15, 35-40 (1999); Lizardi, P. et al., Nat Genet 19, 225-232 (1998); Zhong, X., Lizardi, P., Huang, X., Bray-Ward, P. & Ward, D., Proc Natl Acad Sci U S A 98, 3940-3945 (2001); Faruqi, F. et al. BMC Genomics 2, 4 (2001); Livak, K., Gnet Anal 14, 143-149 (1999); Marras, S., Kramer, F. & Tyagi, S., Genet Anal 14, 151-156 (1999); Ranade, K. et al., Genome Res 11, 1262-1268 (2001); Myakishev, M., Khripin, Y., Hu, S. & Hamer, D., Genome Re 11, 163-169 (2001); Beaudet, L., Bedard, J., Breton, B., Mercuri, R. & Budarf, M., Genome Res 11, 600-608 (2001); Chen, X., Levine, L. & PY, K., Genome Res 9, 492-498 (1999); Gibson, N. et al., Clin Chem 43, 1336-1341 (1997); Latif, S., Bauer-Sardina, I., Ranade, K., Livak, K. & PY, K., Genome Res 11, 436-440 (2001); Hsu, T., Law, S., Duan, S., Neri, B. & Kwok, P., Clin Chem 47, 1373-1377 (2001); Alderborn, A., Kristofferson, A. & Hammerling, U., Genome Res 10, 1249-1258 (2000); Ronaghi, M., Uhlen, M. & Nyren, P., Science 281, 363, 365 (1998); Ronaghi, M., Genome Res 11, 3-11 (2001); Pease, A. et al., Proc Natl Acad Sci U S A 91, 5022-5026 (1994); Southern, E., Maskos, U. & Elder, J., Genomics 13, 1008-1017 (1993); Wang, D. et al., Science 280, 1077-1082 (1998); Brown, P. & Botstein, D., Nat Genet 21, 33-37 (1999); Cargill, M. et al. Nat Genet 22, 231-238 (1999); Dong, S. et al., Genome Res 11, 1418-1424 (2001); Halushka, M. et al., Nat Genet 22, 239-247 (1999); Hacia, J., Nat Genet 21, 42-47 (1999); Lipshutz, R., Fodor, S., Gingeras, T. & Lockhart, D., Nat Genet 21, 20-24 (1999); Sapolsky, R. et al., Genet Anal 14, 187-192 (1999); Tsuchihashi, Z. & Brown, P., J Virol 68, 5863 (1994); Herschlag, D., J Biol Chem 270, 20871-20874 (1995); Head, S. et al., Nucleic Acids Res 25, 5065-5071 (1997); Nikiforov, T. et al., Nucleic Acids Res 22, 4167-4175 (1994); Syvanen, A. et al., Genomics 12, 590-595 (1992); Shumaker, J., Metspalu, A. & Caskey, C., Hum Mutat 7, 346-354 (1996); Lindroos, K., Liljedahl, U., Raitio, M. & Syvanen, A., Nucleic Acids Res 29, E69-9 (2001); Lindblad-Toh, K. ct al., Nat Genet 24, 381-386 (2000); Pastinen, T. et al., Genome Res 10, 1031-1042 (2000); Fan, J. et al., Genome Res 10, 853-860 (2000); Hirschhorn, J. ct al., Proc Natl Acad Sci U S A 97, 12164-12169 (2000); Bouchie, A., Nat Biotechnol 19, 704 (2001); Hensel, M. et al., Science 269, 400-403 (1995); Shoemaker, D., Lashkari, D., Morris, D., Mittmann, M. & Davis, R. Nat Genet 14, 450-456 (1996); Gerry, N. et al., J Mol Biol 292, 251-262 (1999); Ladner, D. et al., Lab Invest 81, 1079-1086 (2001); Iannone, M. et al., Cytometry 39, 131-140 (2000); Fulton, R., McDade, R., Smith, P., Kienker, L. & Kettman, J. J., Clin Chem 43, 1749-1756 (1997); Armstrong, B., Stewart, M. & Mazumder, A., Cytometry 40, 102-108 (2000); Cai, H. et al., Genomics 69, 395 (2000); Chen, J. et al., Genome Res 10, 549-557 (2000); Ye, F. et al. Hum Mutat 17, 305-316 (2001); Michael, K., Taylor, L., Schultz, S. & Walt, D., Anal Chem 70, 1242-1248 (1998); Steemers, F., Ferguson, J. & Walt, D., Nat Biotechnol 18, 91-94 (2000); Chan, W. & Nie, S., Science 281, 2016-2018 (1998); Han, M., Gao, X., Su, J. & Nie, S., Nat Biotechnol 19, 631-635 (2001); Griffin, T. & Smith, L., Trends Biotechnol 18, 77-84 (2000); Jackson, P., Scholl, P. & Groopman, J., Mol Med Today 6, 271-276 (2000); Haff, L. & Smimov, I., Genome Res 7, 378-388 (1997); Ross, P., Hall, L., Smirnov, I. & Haff, L., Nat Biotechnol 16, 1347-1351 (1998); Bray, M., Boerwinkle, E. & Doris, P. Hum Mutat 17, 296-304 (2001); Sauer, S. et al., Nucleic Acids Res 28, E13 (2000); Sauer, S. et al., Nucleic Acids Res 28, E100 (2000); Sun, X., Ding, H., Hung, K. & Guo, B., Nucleic Acids Res 28, E68 (2000); Tang, K. et al., Proc Natl Acad Sci U S A 91, 10016-10020 (1999); Li, J. et al., Electrophoresis 20, 1258-1265 (1999); Little, D., Braun, A., O'Donnell, M. & Koster, H., Nat Med 3, 1413-1416 (1997); Little, D. et al. Anal Chem 69, 4540-4546 (1997); Griffin, T., Tang, W. & Smith, L., Nat Biotechnol 15, 1368-1372 (1997); Ross, P., Lee, K. & Belgrader, P., Anal Chem 69, 4197-4202 (1997); Jiang-Baucom, P., Girard, J., Butler, J. & Belgrader, P., Anal Chem 69, 4894-4898 (1997); Griffin, T., Hall, J., Prudent, J. & Smith, L., Proc Natl Acad Sci U S A 96, 6301-6306 (1999); Kokoris, M. et al., Mol Diagn 5, 329-340 (2000); Jurinke, C., van den Boom, D., Cantor, C. & Koster, H. (2001); and/or Taranenko, N. et al., Genet Anal 13, 87-94 (1996).

Probes and primers can be designed so as to be specific to such mutation analysis and can be derived from the wild type BCR-ABL sequence, segments and complementary sequences thereof.

Additionally, the invention describes assays for the detection of mutant BCR-ABL kinase polynucleotides in a biological sample, such as cell preparations, and the like. A number of methods for amplifying and/or detecting the presence of mutant BCR-ABL kinase polynucleotides are well known in the art and can be employed in the practice of this aspect of the invention.

In certain embodiments, a method for detecting a mutant BCR-ABL kinase mRNA in a biological sample comprises producing cDNA from the sample by reverse transcription using at least one primer; amplifying the cDNA so produced using mutant BCR-ABL kinase polynucleotides as sense and antisense primers to amplify mutants BCR-ABL kinase cDNAs therein; and detecting the presence of the amplified mutant BCR-ABL kinase cDNA. Any number of appropriate sense and antisense probe combinations can be designed from the nucleotide sequences provided for a mutant BCR-ABL kinase and used for this purpose.

The invention also describes assays for detecting the presence of a mutant BCR-ABL kinase protein in a biological sample. Methods for detecting a mutant BCR-ABL kinase protein are also well known and include, for example, immunoprecipitation, immunohistochemical analysis, Western Blot analysis, molecular binding assays, ELISA, ELIFA and the like. For example, in one embodiment, a method of detecting the presence of a mutant BCR-ABL kinase protein in a biological sample comprises first contacting the sample with a BCR-ABL antibody, a mutant BCR-ABL kinase-reactive fragment thereof, or a recombinant protein containing an antigen binding region of a mutant BCR-ABL kinase antibody; and then detecting the binding of mutant BCR-ABL kinase protein in the sample thereto.

Methods for identifying a cell that expresses mutant BCR-ABL kinase arc also described. In one embodiment, an assay for identifying a cell that expresses a mutant BCR-ABL kinase gene comprises detecting the presence of mutant BCR-ABL mRNA in the cell. Methods for the detection of particular mRNAs in cells are well known and include, for example, hybridization assays using complementary DNA probes (such as in situ hybridization using labeled mutant BCR-ABL kinase riboprobes, Northern blot and related techniques) and various nucleic acid amplification assays (such as RT-PCR using complementary primers specific for a mutant BCR-ABL kinase, and other amplification type detection methods, such as, for example, branched DNA, SISBA, TMA and the like).

The detection methods described herein also include methods for identifying amino acid positions within the BCR-ABL polypeptide that may confer at least partial resistance to a tyrosine kinase inhibitor. The methods can comprise the steps of creating a co-crystal of the polypeptide with the BCR-ABL inhibitor, and identifying the amino acid positions of the polypeptide that either contact, bond, interface, or interact with the BCR-ABL inhibitor. Methods of creating crystal structures are known in the art and can include, for example, the use of X-ray crystallography to determine the crystal structure (See, for example, Tokarski et al., Cancer Res (2006), 66(11), 5790-5797) In certain embodiments, the contact or interface amino acids will be at positions 248, 299, 315, and/or 317. In certain embodiments, the contact or interface amino acids will be at positions 244, 248, 255, 290, 299, 313, 315, 316, 317, 318, 320, 321 and/or 380.

### Kits

For use in the diagnostic and therapeutic applications described or suggested above, kits are also provided by the invention. Such kits can, for example, comprise a carrier means being compartmentalized to receive in close confinement one or more container means such as vials, tubes, and the like, each of the container means comprising one of the separate elements to be used in the method. For example, one of the container means can comprise a karyotype detection kit for performing karyotype analysis.

The kit of the invention will typically comprise the container described above and one or more other containers comprising materials desirable from a commercial and user standpoint, including buffers, diluents, filters, needles, syringes, and package inserts with instructions for use. A label can be present on the container to indicate that the composition is used for a specific therapy or non-therapeutic application, and can also indicate directions for either in vivo or in vitro use, such as those described above.

Kits useful in practicing therapeutic methods disclosed herein can also contain a compound that is capable of inhibiting a BCR-ABL kinase and/or mutant BCR-ABL kinases. Specifically contemplated by the invention is a kit comprising a combination of N-(2-chloro-6-methylphenyl)-2-[[6-[4-(2-hydroxyethyl)-1-piperazinyl]-2-methyl-4-pyrimidinyl]amino]-5-thiazolecarboxamide or salt, hydrate, or solvate thereof, and a tubulin stabilizing agent (e.g., pacitaxol, epothilone, taxane, etc.); a combination of N-(2-chloro-6-methylphenyl)-2-[[6-[4-(2-hydroxyethyl)-1-piperazinyl]-2-methyl-4-pyrimidinyl]amino]-5-thiazolecarboxamide or salt, hydrate, or solvate thereof, and a farnysyl transferase inhibitor; a combination of N-(2-chloro-6-methylphenyl)-2-[[6-[4-(2-hydroxyethyl)-1-piperazinyl]-2-methyl-4-pyrimidinyl]amino]-5-thiazolecarboxamide or salt, hydrate, or solvate thereof, and another protein tyrosine kinase inhibitor, such as, imatinib, AMN107, PD180970, GGP76030, AP23464, SKI 606, NS-187, and/or AZD0530; an increased dose and/or dosing frequency regimen of N-(2-chloro-6-methylphenyl)-2-[[6-[4-(2-hydroxyethyl)-1-piperazinyl]-2-methyl-4-pyrimidinyl]amino]-5-thiazolecarboxamide or salt, hydrate, or solvate thereof, relative a treatment regimen suitable for such other forms of such BCR-ABL kinase (e.g., wild-type); and any other combination or dosing regimen comprising N-(2-chloro-6-methylphenyl)-2-[[6-[4-(2-hydroxyethyl)-1-piperazinyl]-2-methyl-4-pyrimidinyl]amino]-5-thiazolecarboxamide or salt, hydrate, or solvate thereof disclosed herein, useful in treating mammals suffering from a BCR-ABL associated disorder, including mutant BCR-ABL associated disorder. For example, kits useful in identifying a mutant BCR-ABL kinase in a mammalian patient (e.g., a human) suffering from a cancer that is completely or partially resistant to, or has developed complete or partial resistance to, N-(2-chloro-6-methylphenyl)-2-[[6-[4-(2-hydroxyethyl)-1-piperazinyl]-2-methyl-4-pyrimidinyl]amino]-5-thiazolecarboxamide or salt, hydrate, or solvate thereof, imatinib, or another protein tyrosine kinase inhibitor and where said kits also comprise a therapeutically effective amount of the combination or increased dose or dosing regimen, are contemplated herein.

In addition, the kits can include instructional materials containing directions (i.e., protocols) for the practice of the methods of this invention. While the instructional materials typically comprise written or printed materials they are not limited to such. Any medium capable of storing such instructions and communicating them to an end user is contemplated by this invention. Such media include, but are not limited to electronic storage media (e.g., magnetic discs, tapes, cartridges, chips, and the like), optical media (e.g., CD ROM), and the like. Such media can include addresses to internet sites that provide such instructional materials.

The kit can also comprise, for example, a means for obtaining a biological sample from an individual. Means for obtaining biological samples from individuals are well known in the art, e.g., catheters, syringes, and the like, and are not discussed herein in detail.

For use in the diagnostic and therapeutic applications described or suggested above, kits are also provided by the invention. Such kits can, for example, comprise a carrier means being compartmentalized to receive in close confinement one or more container means such as vials, tubes, and the like, each of the container means comprising one of the separate elements to be used in the method. For example, one of the container means can comprise a probe that is or can be detectably labeled. Such probe can be an antibody or polynucleotide specific for a mutant BCR-ABL kinase protein or a mutant BCR-ABL kinase gene or message, respectively. Where the kit utilizes nucleic acid hybridization to detect the target nucleic acid, the kit can also have containers containing nucleotide(s) for amplification of the target nucleic acid sequence and/or a container comprising a reporter-means, such as a biotin-binding protein, such as avidin or streptavidin, bound to a reporter molecule, such as an enzymatic, florescent, or radioisotope label.

The kit of the invention will typically comprise the container described above and one or more other containers comprising materials desirable from a commercial and user standpoint, including buffers, diluents, filters, needles, syringes, and package inserts with instructions for use. A label can be present on the container to indicate that the composition is used for a specific therapy or non-therapeutic application, and can also indicate directions for cither in vivo or in vitro use, such as those described above.

Kits useful in practicing therapeutic methods disclosed herein can also contain a compound that is capable of inhibiting a mutant BCR-ABL kinase. Specifically contemplated by the invention is a kit comprising a combination of N-(2-chloro-6-methylphenyl)-2-[[6-[4-(2-hydroxyethyl)-1-piperazinyl]-2-methyl-4-pyrimidinyl]amino]-5-thiazolecarboxamide or salt, hydrate, or solvate thereof, and a tubulin stabilizing agent (e.g., pacitaxol, epothilone, taxane, etc.); a combination of N-(2-chloro-6-methylphenyl)-2-[[6-[4-(2-hydroxyethyl)-1-piperazinyl]-2-methyl-4-pyrimidinyl]amino]-5-thiazolecarboxamide or salt, hydrate, or solvate thereof, and a farnysyl transferase inhibitor; a combination of N-(2-chloro-6-methylphenyl)-2-[[6-[4-(2-hydroxyethyl)-1-piperazinyl]-2-methyl-4-pyrimidinyl]amino]-5-thiazolecarboxamide or salt, hydrate, or solvate thereof, and another protein tyrosine kinase inhibitor, such as, imatinib, AMN107, PD180970, GGP76030, AP23464, SKI 606, NS-187, and/or AZD0530; an increased dose and/or dosing frequency regimen of N-(2-chloro-6-methylphenyl)-2-[[6-[4-(2-hydroxyethyl)-1-piperazinyl]-2-methyl-4-pyrimidinyl]amino]-5-thiazolecarboxamide or salt, hydrate, or solvate thereof, relative a treatment regimen suitable for such other forms of such BCR-ABL kinase (e.g., wild-type); and any other combination or dosing regimen comprising N-(2-chloro-6-methylphenyl)-2-[[6-[4-(2-hydroxyethyl)-1-piperazinyl]-2-methyl-4-pyrimidinyl]amino]-5-thiazolecarboxamide or salt, hydrate, or solvate thereof disclosed herein, useful in treating mammals suffering from a BCR-ABL associated disorder, including mutant BCR-ABL associated disorder. For example, kits useful in identifying a mutant BCR-ABL kinase in a mammalian patient (e.g., a human) suffering from a cancer that is completely or partially resistant to, or has developed complete or partial resistance to, N-(2-chloro-6-methylphenyl)-2-[[6-[4-(2-hydroxyethyl)-1-piperazinyl]-2-methyl-4-pyrimidinyl]amino]-5-thiazolecarboxamide or salt, hydrate, or solvate thereof, imatinib, or another protein tyrosine kinase inhibitor and where said kits also comprise a therapeutically effective amount of the combination or increased dose or dosing regimen, are contemplated herein.

In addition, the kits can include instructional materials containing directions (i.e., protocols) for the practice of the methods of this invention. While the instructional materials typically comprise written or printed materials they are not limited to such. Any medium capable of storing such instructions and communicating them to an end user is contemplated by this invention. Such media include, but are not limited to electronic storage media (e.g., magnetic discs, tapes, cartridges, chips, and the like), optical media (e.g., CD ROM), and the like. Such media can include addresses to internet sites that provide such instructional materials.

The kit can also comprise, for example, a means for obtaining a biological sample from an individual. Means for obtaining biological samples from individuals are well known in the art, *e.g*., catheters, syringes, and the like, and are not discussed herein in detail.

The present invention as defined in the claims is not to be limited in scope by the embodiments disclosed herein, which are intended as single illustrations of individual aspects of the invention, and any that are functionally equivalent are within the scope of the invention. Various modifications to the models and methods of the invention, in addition to those described herein, will become apparent to those skilled in the art from the foregoing description and teachings, and are similarly intended to fall within the scope of the invention as defined in the claims..

The following representative examples contain important additional information, exemplification and guidance which can be adapted to the practice of this invention in its various embodiments and the equivalents thereof. These examples are intended to help illustrate the invention, and are not intended to, nor should they be construed to, limit its scope.

### REFERENCES

1. Sawyers CL. Chronic myeloid leukemia. N Engl J Med 1999;340(17):1330-40.
2. Druker BJ, Sawyers CL, Kantarjian H, et al. Activity of a specific inhibitor of the BCR-ABL tyrosine kinase in the blast crisis of chronic myeloid leukemia and acute lymphoblastic leukemia with the Philadelphia chromosome. N Engl J Med 2001;344(14):1038-42.
3. Ottmann OG, Druker BJ, Sawyers CL, et al. A phase 2 study of imatinib in patients with relapsed or refractory Philadelphia chromosome-positive acute lymphoid leukemias. Blood 2002;100(6):1965-71.
4. Sawyers CL, Hochhaus A, Feldman E, et al. Imatinib induces hematologic and cytogenetic responses in patients with chronic myelogenous leukemia in myeloid blast crisis: results of a phase II study. Blood 2002;99(10):3530-9.
5. Talpaz M, Silver RT, Druker BJ, et al. Imatinib induces durable hematologic and cytogenetic responses in patients with accelerated phase chronic myeloid leukemia: results of a phase 2 study. Blood 2002;99(6):1928-37.
6. Silver RT, Talpaz M, Sawyers CL, et al. Four years follow-up of 1027 patients with late chronic phase (L-CP), accelerated phase (AP), or blast crisis (BC) chronic myeloid leukemia (CML) treated with imatinib in three large Phase II trials. Blood 2004;104(11a): 10a (Abstract 23).
7. Gorre ME, Mohammed M, Ellwood K, et al. Clinical resistance to STI-571 cancer therapy caused by BCR-ABL gene mutation or amplification. Science 2001;293(5531):876-80.
8. Donato NJ, Wu JY, Stapley J, et al. BCR-ABL independence and LYN kinase overexpression in chronic myelogenous leukemia cells selected for resistance to STI571. Blood 2003;101(2):690-8.
9. Shah NP, Nicoll JM, Nagar B, et al. Multiple BCR-ABL kinase domain mutations confer polyclonal resistance to the tyrosine kinase inhibitor imatinib (STI571) in chronic phase and blast crisis chronic myeloid leukemia. Cancer Cell 2002;2(2):117-25.
10. Branford S, Rudzki Z, Walsh S, et al. High frequency of point mutations clustered within the adenosine triphosphate-binding region of BCR/ABL in patients with chronic myeloid leukemia or Ph-positive acute lymphoblastic leukemia who develop imatinib (STI571) resistance. Blood 2002;99(9):3472-5.
11. Shah NP, Tran C, Lee FY, Chen P, Norris D, Sawyers CL. Overriding imatinib resistance with a novel ABL kinase inhibitor. Science 2004;305(5682):399-401.
12. O'Hare T, Walters DK, Stoffregen EP, et al. In vitro activity of Bcr-Abl inhibitors AMN107 and BMS-354825 against clinically relevant imatinib-resistant Abl kinase domain mutants. Cancer Res 2005;65(11):4500-5.
13. Lombardo LJ, Lee FY, Chen P, et al. Discovery of N-(2-chloro-6-methyl-phenyl)-2-(6-(4-(2-hydroxyethyl)-piperazin-1-yl)-2-methylpyrimidin-4-ylamino)thiazole-5-carboxamide (BMS-354825), a dual Src/Abl kinase inhibitor with potent antitumor activity in preclinical assays. J Med Chem 2004;47(27):6658-61.
14. Burgess M, Shah N, Skaggs B, Lee F, Sawyers C. Comparative analysis of two BCR-ABL small molecule inhibitors reveals overlapping but distinct mechanisms of resistance. Blood 2004;104(11):160a (Abstract 552).
15. Schindler T, Bornmann W, Pellicena P, Miller WT, Clarkson B, Kuriyan J. Structural mechanism for STI-571 inhibition of abelson tyrosine kinase. Science 2000;289(5486):1938-42.
16. Tokarski J, Newitt J, Lee F, et al. The crystal structure of Abl kinase with BMS-354825, a dual SRC/ABL kinase inhibitor. Blood 2004;104(11):160a (Abstract 553).
17. Cancer Therapy Evaluation Program. Common Terminology Criteria for Adverse Events. Version 3.0: National Cancer Institute. December 12, 2003.
18. Lowell CA. Src-family kinases: rheostats of immune cell signaling. Mol Immunol 2004;41(6-7):631-43.
19. Yamamoto M, Kakihana K, Kurosu T, Murakami N, Miura O. Clonal evolution with inv(11)(p15q22) and NUP98/DDX10 fusion gene in imatinib-resistant chronic myelogenous leukemia. Cancer Genet Cytogenet 2005;157(2):104-8.
20. Mellinghoff IK, Wang MY, Vivanco I, et al. Molecular determinants of the response of glioblastomas to EGFR kinase inhibitors. N Engl J Med 2005;353(19):2012-
21. Pao W, Miller VA, Politi KA, et al. Acquired resistance of lung adenocarcinomas to gefitinib or erlotinib is associated with a second mutation in the EGFR kinase domain. PLoS Med 2005;2(3):e73.
22. Kobayashi S, Boggon TJ, Dayaram T, et al. EGFR mutation and resistance of non-small-cell lung cancer to gefitinib. N Engl J Med 2005;352(8):786-92.
23. Antonescu CR, Besmer P, Guo T, et al. Acquired resistance to imatinib in gastrointestinal stromal tumor occurs through secondary gene mutation. Clin Cancer Res 2005;11(11):4182-90.
24. Wardelmann E, Thomas N, Merkelbach-Bruse S, et al. Acquired resistance to imatinib in gastrointestinal stromal tumours caused by multiple KIT mutations. Lancet Oncol 2005;6(4):249-51.
25. T. R J. Evans, J. A. Morgan, A. D. van den Abbeele, et. al. Phase I dose-escalation study of the SRC and multi-kinase inhibitor BMS-354825 in patients (pts) with GIST and other solid tumors J Clin Oncol (Meeting Abstracts) 2005 23: 3034.

### EXAMPLES

### EXAMPLE 1 - METHODS USED IN THE DOSE ESCALATION CLINICAL TRIAL TO EVALUATE DASATINIB IN PATIENTS WITH ADVANCED CML OR PH+ALL

### Study patients

Patients aged ≥14 years with AP, BC, or Ph+ ALL with primary or acquired resistance or intolerance to imatinib therapy (after receiving at least 400 mg per day) were eligible. Patients were classified as having BC if they had ≥30% blasts in peripheral blood or bone marrow or extramedullary infiltrates of leukemic cells (other than the spleen or liver). Patients were classified as having AP if they did not fulfill either criterion for BC, but did meet any of the following criteria: ≥15% to <30% blasts in peripheral blood or bone marrow; ≥20% basophils in peripheral blood or bone marrow; ≥30% blasts plus promyelocytes (but <30% blasts) in peripheral blood or bone marrow; or a platelet count <100,000 cells/mm³ unrelated to therapy. Ph+ ALL patients had ≥30% lymphoblasts in peripheral blood or bone marrow without prior evidence of chronic phase CML. All patients gave written informed consent according to institutional regulations, prior to participation in the study.

Primary hematologic resistance to imatinib was defined as either failure to return to chronic phase after 3 months of imatinib treatment or disease progression within 3 months from the start of imatinib treatment Acquired hematologic resistance was defined as disease progression after obtaining a hematologic response (defined below and in Supplemental Table 1) sustained for 3 months. Patients were considered intolerant of imatinib if they had discontinued imatinib therapy due to non-hematologic toxicity of any grade.

### Study design

This pilot open-label, dose escalation study was designed to test the safety and anti-leukemic activity of dasatinib in imatinib-resistant or -intolerant patients with Ph+ CML orPh+ ALL. The starting dose of 35 mg taken orally, twice daily (BID) was chosen based on safety data available from a phase I study in chronic phase CML patients which was conducted simultaneously. Subsequent cohorts were treated with 50 mg, 70 mg, 90 mg or 120 mg BID. Intrapatient dose escalation was permitted. One treatment cycle was defined as 4 weeks (28 days). Dose modifications were made based on hematologic and non-hematologic adverse events. The study protocol was approved by the Institutional Review Boards at UCLA and MD Anderson Cancer Center.

### Assessment of safety and toxicity

Patients were assessed by physical examination, performance status, vital signs and 12-lead ECG at baseline. Adverse events (hematologic and non-hematologic) were evaluated throughout the study and graded according to the National Cancer Institute Common Terminology Criteria for Adverse Events (NCI-CTCAE) Version 3.0 (17). Complete blood counts and serum chemistries were performed twice weekly for the first 3 months and then every 2 weeks for 3 months, and every 6 weeks for the remainder of the study.

### Assessment of response

Hematologic responses (HR) were scored as major, minor or non-response (Supplemental Table 1). Major hematologic response (major HR) includes two subgroups, called no evidence of leukemia (NEL) and complete HR (CHR), based on whether full recovery of peripheral blood counts was achieved. CHR was defined as: bone marrow lasts ≤ 5% and white blood cell (WBC) count ≤ institutional upper limits of normal (ULN); absolute neutrophil count (ANC) ≥1000 cells/mm³; platelets ≥100,000 cells/mm³; no blasts or promyelocytes in peripheral blood; <5% myelocytes plus metamyelocytes in peripheral blood; and basophils < 20%. NEL was defined as: bone marrow blasts ≤5%, no blasts or promyelocytes in peripheral blood, WBC ≤ institutional ULN, peripheral blood basophils < 20%, ANC ≥ 500/mm³ < 1000/mm³, and platelets ≥ 20,000/mm³ < 100,000/mm³. Minor HR was defined as: blasts >5% and <15% in the bone marrow or peripheral blood, <30% blasts plus promyelocytes in the bone marrow and peripheral blood, basophils ≤ 20% and no extramedullary disease other than spleen or liver. Responses were called confirmed if they persisted for 4 weeks.

Bone marrow morphology and cytogenetics were assessed every 3 months for determination of cytogenetic response (CyR). Extent of CyR was defined on the basis of percent Ph+ cells in metaphase in the bone marrow: complete CyR (CCyR), 0%; partial CyR (PCyR), 1-35%; minor CyR, 36-65%; minimal CyR, 66-95%; no CyR, 96-100%.

Any patient with a major or minor HR that subsequently failed to meet these criteria over a 2 week period was considered to have progressive disease. Patients with AP were considered to have progressed if they developed BC. Patients with BC or Ph+ ALL were considered to have progressed if they had an increase in percent blasts in peripheral blood or bone marrow despite at least 4 weeks of treatment.

### Analysis of mutational status

Blood samples were prospectively collected for *BCR-A.BL* mutation analysis prior to the first treatment. Additionally, mutational status was re-assessed in some patients at the time of disease progression. All baseline mutational analyses were conducted as previously described and performed at a central laboratory (9). Karyotypes of each patient were also determined.

### Pharmacokinetics/Pharmacodynamics

Plasma pharmacokinetics (PK) were determined on Days 1 and 8 of the first treatment cycle and Day 1 of the second treatment cycle. Pharmacodynamic (PD) evaluation was conducted by analysis of phosphorylation of CRKL or SRC in peripheral blood samples (7) obtained at baseline, then at 4, 8 and 24 hours post-dose on day 1.

### EXAMPLE 2 - METHOD OF ASSESSING WHETHER A PATIENT HAS A COMPLEX CHROMOSOME KARYOTYPE

A number of methods are known in the art for analyzing chromosomes karyotypes. The following, non-limiting, methods are encompassed by the present invention: the methods described in Aoun et al., Can. Gen. and Cyto. 154:138-143 (2004); Braziel et al., Blood 100(2):435-41 (2002); Espinoza et al., Cancer Gen. and Cyto., 157:175-7 (2005); Heller et al., Int. J. Oncol., 24(1)127-36 (2004); Giehl et al., Leukemia 19:1192-1197 (2005); Oudat et al., Arch. Path. Lab: Medicine, 125:437-439 (2001); Speicher et al. Nat Genet. 12(4):368-75 (1996); and Schröck et al, Science; 273 (5274):494 (1996); the entire contents of each of these references are hereby incorporated by reference in their entirety herein.

One non-limiting example in how such karyotyping analysis may be performed in provided. Briefly, patient blood samples are cultured for 24-48 hours at 37°C in CHANG MEDIUM (Irvine Scientific, Irvine, CA), and then conventional karyotyping using Giemsa or Wright staining methods for metaphasic chromosomes are performed, and then the chromosomes analyzed.

Another non-limiting example is provided. Briefly, about 1 ml of blood can be cultured in 5 ml of RPMI 1640 media containing 0.25 % w/v ampicillin, 0.01 1 % w/v streptomycin, 20 % v/v fetal bovine serum, 100 ml of phytohaemagglutinin and 300 ml of concanavalin A. The mixture is then incubated at 37°C for 96 hrs (Seabright 1971; Meevatee 1988).

### Peripheral blood lymphocytes and chromosome preparation

An amount of 100 ml of colcemid solution (0.2 mg/ml) is added to the above mixture, which is then further incubated at room temperature (30±1°C) for 30 mins. The mixture is centrifuged at 1200 rpm for 5 mins.

The supernatant is removed and then 5 ml of phosphate buffer saline (PBS) was added to the cells. The mixture is centrifuged at 1200 rpm for 5 mins. The supernatant is removed and then 5 ml of hypotonic solution (0.075 M KCl) was added to swell up and lyse the cells. The mixture is centrifuged at 1200 rpm for 5 mins. The cells are fixed with 5 ml of Carnoy fixative [methanol/acetic acid (3:1)] and centrifuged at 1200 rpm for 5 mins. The fixing step is repeated twice. The resulting cells are kept at 4±1°C for chromosome banding and staining (Ida and Kyo 1980; Meevatee 1988).

### Preparation of chromosomal G-banding and staining

About one milliliter of Carnoy fixative is added to the cells. The mixture is dropped on a clean slide and air dried. The slide is warmed at 90°C and then dipped in 0.25% w/v trypsin solution for 15 seconds, washed with PBS and air dried. The slide is immersed in 10 % w/v Giemsa in Sorenson's phosphate buffer solution (pH 7) for 40 mins and followed by rinsing with distilled water (Uwa and Ojima 1981).

### Chromosomal analysis

The slides of the chromosomes are examined using a light microscope (Zeiss Axioskop, Germany) at a magnification of 1,000 X with the Matrox inspector version 2.1 Program. Metaphases of the chromosomes are classified, photographed and counted. Representative metaphases are printed on high contrast paper and the karyotypes are arranged according to chromosomal morphology, centromere, size and arm (Meevatee 1988).

### Example 3 - Exemplary Methods for Detecting BCR-ABL Kinase Mutations

N-(2-chloro-6-methylphenyl)-2-[[6-[4-(2-hydroxyethyl)-1-piperazinyl]-2-methyl-4-pyrimidinyl]amino]-5-thiazolecarboxamide and imatinib are two potent BCR-ABL kinase inhibitors that are effective in treating CML and solid tumors. Provided herein are exemplary combination therapies and dosing regimens that will be useful in treating cancers which are resistant to protein tyrosine kinase inhibitor agents, such as imatinib and other kinase inhibitors, and specifically including cancers involving one or more mutations in BCR-ABL kinase.

A significant aspect of this combination therapy is the detection of the mutations in BCR-ABL kinase. If a mutant BCR-ABL kinase of the present invention is present in a patient, it indicates an individual can be selected for combination therapy, or more aggressive dosing regimens (e.g., higher and/or more frequent doses), or a combination of aggressive dosing regimen and combination therapy. Furthermore, if a specific BCR-ABL kinase mutant is detected, the amount of either or both inhibitors can be increased or decreased in order to enhance the therapeutic effect of the regimen.

There are several methods that can be used to detect a mutant BCR-ABL kinase in cancer patients, particularly CML patients. They include methods for detecting BCR-ABL kinase polynucleotides and BCR-ABL kinase proteins, as well as methods for identifying cells that express BCR-ABL kinase. Detection of certain mutant BCR-ABL kinasse in a patient would be diagnostic that such patients either are or will become at least partially resistant to imatinib therapy or N-(2-chloro-6-methylphenyl)-2-[[6-[4-(2-hydroxyethyl)-1-piperazinyl]-2-methyl-4-pyrimidinyl]amino]-5-thiazolecarboxamide therapy. As discussed in detail below, the status of BCR-ABL kinase gene products in patient samples can be analyzed by a variety of protocols well know in the art including, for example, immunohistochemical analysis, the variety of Northern blotting techniques including in situ hybridization, RT-PCR analysis (for example on laser capture micro-dissected samples), western blot analysis, tissue array analysis, microarray analysis, genotyping methods, and mass-spectroscopic methods.

Methods of identifying the nucleic acid and the amino acid of a mutant BCR-ABL kinase are known in the art.

One experimental strategy is to use PCR to amplify a region of the BCR-ABL transcript using primers specific to BCR and ABL, and sequence the PCR fragment directly, or subclone this product and sequence several independent clones in both directions. This strategy allows one to quantify fluctuations in different clones from the same patient over time. Typical methodologies are for such protocols are provided below.

Blood samples can be obtained from patients enrolled in clinical trials in the treatment of CML. RNA is then extracted using TriAgent or TriAzol. cDNA synthesis is performed using MMTV reverse transcriptase. Polymerase chain reaction (PCR) is performed to amplify the cDNA, using primers CM10 (5'-GAAGCTTCTCCCTGACATCCGT-3') (SEQ ID NO: 3) and 3' Abl Long KD (5'-CCCCACGGACGCCTTGTTTCCCCAG -3') (SEQ ID NO: 4). The resultant fragment is then ligated into pBluescript II KS+digested with Eco RV. Bacterial transformants are plated on media containing ampicillin and X- gal. Ten white colonies per cDNA, for example, are inoculated into media and miniprep DNA is isolated. Sequencing of each clone is then performed using M13 universal forward (CGCCAGGGTTTTCCCAGTCACGAC; SEQ ID NO:5) and M13 reverse (AGCGGATAACAATTTCACACAGGA; SEQ ID NO:6) primers. A mutation can be considered present if it was detected on both strands of at least two independent clones per patient.

Alternatively, antibodies that immunospecifically bind to mutant BCR-ABL kinase can be used to detect the presence of a mutant BCR-ABL kinase in a sample. First, mutant BCR-ABL kinase can be generated by site directed mutagenesis. Cell lines expressing these mutant BCR-ABL kinase isoforms will then be created. Next, antibodies against mutant BCR-ABL kinase isoforms will be produced. Expression of BCR-ABL kinase and its mutant isoforms will be documented by Western blot analysis.

Specifically, site directed mutagenesis can be used to create the BCR-ABL kinase mutations (QuickChange Kit, Stratagene, La Jolla, CA) and all mutations will be confirmed by bidirectional sequencing (O'Farrell, A. M., et al., Blood, 101:3597-3605 (2003)). Retroviral transduction is performed and Ba/F3 cell lines stably expressing mutant BCR-ABL kinase isoforms are generated by double selection for G418 resistance and IL-3 independent growth (Yee, K. W., et al., Blood, 100:2941-2949 (2002); Yee, K. W., et al., Blood, 104:4202-4209 (2004); Tse, K. F., et al., Leukemia, 14:1766-1776 (2000); Schittenhelm, M. M., et al., manuscript submitted (2005)). Transient transfections of CHO-Kl chinese hamster cell lines with BCR-ABL kinase wild type ("WT") or mutant isoforms are performed using a lipofection-assay (LipofectAMINE-kit purchased from Gibco-Invitrogen, Carlsbad, CA). Cells are treated with N-(2-chloro-6-methylphenyl)-2-[[6-[4-(2-hydroxyethyl)-1-piperazinyl]-2-methyl-4-pyrimidinyl]amino]-5-thiazolecarboxa,mide 24 hours after transfection (Heinrich, M. C., et aL, Journal of Clinical Oncology, 21:4342-4349 (2003)). Alternatively, cells can be treated with any of the combinations outlined herein, or using increased levels of N-(2-chloro-6-methylphenyl)-2-[[6-[4-(2-hydroxyethyl)-1-piperazinyl]-2-methyl-4-pyrimidinyl]amino]-5-thiazolecarboxamide.

An anti- BCR-ABL kinase rabbit polyclonal antibody, an anti-STAT3 mouse monoclonal antibody (both Santa Cruz Biotechnology, Santa Cruz, CA), an anti-AKT (polyclonal) rabbit antibody (Cell Signaling Technology, Beverly MA) and an anti-MAP kinase 1/2 (Erk 1/2) rabbit monoclonal antibody (Upstate Biotechnology, Lake Placid, NY) can be used at a 1:5000 to 1:2000 dilution. Anti-phosphotyrosine BCR-ABL antibodies (Tyr568/570 and Tyr703), an anti-phosphothreonine/tyrosine MAP kinase (Thr202/Tyr204) antibody, an anti-phosphothreonine (Thr308) and an anti-phosphoserine (Ser473) AKT antibody, an anti-phosphotyrosine (Tyr705) STAT3 antibody and an unspecific anti-phosphotyrosine antibody (clone pY20) are used at dilutions of 1:100 to 1:2000 (all Cell Signaling Technology, Beverly MA). Peroxidase conjugated goat anti-mouse antibody and goat anti-rabbit antibody will be used at 1:5000 and 1:10,000 dilutions respectively (BioRad; Hercules, CA). Protein A/G PLUS-Agarose immunoprecipitation reagent can be purchased from Santa Cruz Biotechnology (Santa Cruz, CA). imatinib, N-(2-chloro-6-methylphenyl)-2-[[6-[4-(2-hydroxyethyl)-1-piperazinyl]-2-methyl-4-pyrimidinyl]amino]-5-thiazolecarboxamide, paclia tubulin stabilizing agent (e.g., pacitaxol, epothilone, taxane, etc.), and another agents useful in combination with N-(2-chloro-6-methylphenyl)-2-[[6-[4-(2-hydroxyethyl)-1-piperazinyl]-2-methyl-4-pyrimidinyl]amino]-5-thiazolecarboxamide, are dissolved in DMSO to create 10 mM stock solutions and be stored at - 20° C.

Western blot assays can be conducted as follows. ~5 x 10⁷ cells are exposed to varying concentrations of N-(2-chloro-6-methylphenyl)-2-[[6-[4-(2-hydroxyethyl)-1-piperazinyl]-2-methyl-4-pyrimidinyl]amino]-5-thiazolecarboxamide and cultured for 90 minutes at 37°C in a 5% CO₂ atmosphere. Cell pellets are lysed with 100-150 µL of protein lysis buffer (50 mM Tris, 150 mM NaCl, 1% NP-40, 0.25% deoxycholate with added inhibitors aprotinin, AEBSF, leupeptin, pepstatin, sodium orthovanadate, and sodium pyruvate). 500-2000 microgram of protein from cell lysates are used for immunoprecipitation experiments and 75-200 microgram of protein from cell lysates are used for whole cell protein analysis by western immunoblot assays as previously described in Hoatlin, M. E., et al., Blood, 91:1418-1425 (1998).

In certain contexts, it may be desirable to amplify a specific region in BCR-ABL kinase such as one of the functional domains discussed herein. For example, the region corresponding to the ATP binding pocket and the activation loop domain of BCR-ABL is critical to the selectivity of imatinib and is the region known to harbor the most imatinib-resistant and protein tyrosine kinase inhibitor mutations. Sequencing of this region can most efficiently reveal the patients' CML clinical profile, and hence the appropriate combination therapy and/or dosing regimen.

However, in the Briefly, RNA is extracted from purified peripheral blood or bone marrow cells with TriReagent-LS (Molecular Research Center, Inc., Cincinnati, Ohio). Total RNA is subjected to RT-PCR by using the same protocol and primers as described supra. PCR products are cloned into the pCR2.1 TA cloning vector (Invitrogen, Carlsbad, Calif.). Both strands of the are sequenced with the 5' M13 reverse and M13 forward primer for the fragment, on an ABI prism 377 automated DNA sequencer (PE Applied Biosystems, Foster City, Calif). Sequence analysis is then performed using the ClustalW alignment algorithm). Any detected mutation is then confirmed by analysis of genomic DNA. Briefly, genomic DNA is extracted from purified bone marrow or peripheral blood cells with the QiaAMP Blood Mini Kit (Qiagen, Inc., Valencia, Calif.) using primers specific to intron sequences that flank both sides of the location of the mutation of interest. PCR products are cloned and sequenced.

Additional methods of detecting mutant BCR-ABL kinases is disclosed in O'Hare et al. (Cancer Research, 65(11):4500-5 (2005),

### Example 4 - Exemplary method of Assessing the potential of the Combination Therapy

The combination of N-(2-chloro-6-methylphenyl)-2-[[6-[4-(2-hydroxyethyl)-1-piperazinyl]-2-methyl-4-pyrimidinyl]amino]-5-thiazolecarboxamide and imatinib can be studied in mouse models of imatinib-resistant or protein tyrosine kinase inhibitor resistant, BCR-ABL -dependent disease. A series of such pharmacodynamic experiments will help to determine the optimal dosing regimen for different mutant BCR-ABL isoforms in vivo. Pharmacodynamic experiments are well known in the art and one skilled in the art would readily appreciate that such experiments can be modified to alter existing conditions, as applicable. Briefly, severe combined immuno-deficient mice are injected intravenously with Ba/F3 cells expressing different BCR-ABL wild-type or mutant isoforms as well as the firefly luciferase gene. Untreated mice harboring Ba/F3 cells expressing nonmutant or imatinib-resistant BCR-ABL mutants are expected to develop aggressive disease, with massive liver and splenic infiltration, typically resulting in death. To assess the ability of combination therapy, or a modified dosing regimen, to inhibit BCR-ABL in vivo, BCR-ABL kinase activity in splenocyte lysates prepared at various time points after administration of a different single dose of 0, 0.5, 1, 5, and 10 micromoles per liter of N-(2-chloro-6-methylphenyl)-2-[[6-[4-(2-hydroxyethyl)-1-piperazinyl]-2-methyl-4-pyrimidinyl]amino]-5-thiazolecarboxamide and imatinib combination by oral gavage will be assessed. Phosphorylation of the adapter protein CRKL, a known BCR-ABL substrate (T. Oda et al., J. Biol. Chem. 269, 22925 (1994)), will be monitored to gauge the efficacy of the combination therapy. On the basis of a series of such pharmacodynamic experiments, an proper dose of the combination will be chosen for efficacy studies. Then, mice will be documented by bioluminescence imaging before and after dosing. On the basis of a series of such pharmacodynamic experiments, the optimal dosing regimen and/or combination therapy can be identified. Mice are dosed with combination or vehicle alone by gavage for 2 weeks, beginning 3 days after injection of Ba/F3 cells, and disease burden is then assessed by bioluminescence imaging. All vehicle-treated mice are expected to develop progressive disease. In contrast, combination-treated mice harboring nonmutant BCR-ABL or the clinically common imatinib-resistant and protein tyrosine kinase inhibitor resistant mutations described herein are expected to develop less or no progressive disease. It is also expected that different optimal dosing regimens will be identified for different BCR-ABL isoforms. Such dosing difference can be taken into consideration in the treatment of patients with a known BCR-ABL mutation(s).

### Example 5 - Exemplary Method of Assessing the Safety and Efficacy of Protein Tyrosine Kinase Combination Therapy and/or Modified Dosing Regimens

Previous findings have shown that both N-(2-chloro-6-methylphenyl)-2-[[6-[4-(2-hydroxyethyl)-1-piperazinyl]-2-methyl-4-pyrimidinyl]amino]-5-thiazolecarboxamide and imatinib are highly selective for leukemic versus normal hematopoietic cells (B. J. Druker et al., Nature Med. 2. 561 (1996) and N. P. Shah et al., Science 305. 399 (2004)). Such high selectivity demonstrates the high safety and efficacy of these inhibitors, and the expected efficacy of their combination. To assess the efficacy of the combination on human bone marrow progenitors, the compounds are tested in vitro in colony-forming-unit (CEU) assays. A series of concentrations of N-(2-chloro-6-methylphenyl)-2-[[6-[4-(2-hydroxyethyl)-1-piperazinyl]-2-methyl-4-pyrimidinyl]amino]-5-thiazolecarboxamide and imatinib combination, or other combinations disclosed herein, are applied to bone marrow progenitors isolated from healthy volunteers and from CML patients with either imatinib-sensitive (nonmutant BCR-ABL) or imatinib-resistant disease. Furthermore, the blast-forming unit-erythroid (BFU-E) and CFU-granulocyte-monocyte (GM) colonies from CML patient marrow samples will be analyzed by polymerase chain reaction (PCR) analysis in order to detect the sensitivity of selection for growth of rare normal progenitors present in these leukemic marrow samples. Briefly, bone marrow is harvested from clinical subjects. Viable frozen Ficoll-Hypaque-purified mononuclear cells are thawed and grown overnight in Iscove's Media supplemented with 10% Fetal calf serum, 1-glutamine, pen-strep, and stem cell factor (100 ug/ml) at a density of 5x10⁵/ml. After 24 hours, viable cells are quantitated and plated in Methocult media (Cell Signal Technologies, Beverly, MA) at 1x10⁴ and 1x10⁵ cells per plate in the presence of 5 nM N-(2-chloro-6-methylphenyl)-2-[[6-[4-(2-hydroxyethyl)-1-piperazinyl]-2-methyl-4-pyrimidinyl]amino]-5-thiazolecarboxamide or vehicle. Experiments are performed in triplicate. On day 11, erythroid blast-forming unit (BFU-E) and granulocyte-macrophage colony forming units (CFU-GM) will be quantitated. On day 14, colonies will be isolated with a pipet tip, and RNA will be isolated using a Qiagen Rneasy kit. A primer complementary to the region of ABL approximately 200 nucleotides downstream of the BCR-ABL mRNA (5'-CGGCATTGCGGGACACAGGCCCATGGTACC; SEQ ID NO:7) junction is annealed to purified RNA. cDNA is synthesized using mouse Moloney leukemia virus (MMLV) reverse transcriptase, and subjecting to 40 cycles of PCR using either a BCR (5'-TGACCAACTCGTGTGTGAAACT; SEQ ID NO:8) or ABL type Ia 5' primer (GGGGAATTCGCCACCATGTTGGAGATCTGCCTGA; SEQ ID NO:9) as a control for the quality of RNA.

### Example 6 - Exemplary Methods for Measuring of BCR-ABL Kinase Activity Via the Phosphotyrosine Content of Crkl

The ability of a combination therapy or more aggressive dosing regimen of the present invention to effectively overcome N-(2-chloro-6-methylphenyl)-2-[[6-[4-(2-hydroxyethyl)-1-piperazinyl]-2-methyl-4-pyrimidinyl]amino]-5-thiazolecarboxamide or imatinib resistance, to inhibit BCR-ABL activity, or to inhibit BCR-ABL mutant activity, the phosphotyrosine content of Crkl, an adaptor protein which is specifically and constitutively phosphorylated by BCR-ABL in CML cells can be used (see, e.g. J. ten Hoeve et al., Blood 84, 1731 (1994); T. Oda et al., J. Biol. Chem. 269, 22925 (1994); and G. L. Nichols et al., Blood 84, 2912 (1994)). The phosphotyrosine content of Crkl has been shown to be reproducibly and quantitatively measured in clinical specimens. Crkl binds BCR-ABL directly and plays a functional role in BCR-ABL transformation by linking the kinase signal to downstream effector pathways (see, e.g. K. Senechal et al., J. Bio. Chem.. 271, 23255 (1996)). When phosphorylated, Crkl migrates with altered mobility in SDS-PAGE gels and can be quantified using densitometry. Sawyers et al have shown that Crkl phosphorylation in primary CML patient cells was inhibited in a dose-dependent manner when exposed to STI-571 and correlated with dephosphorylation of BCR-ABL. Likewise, we have also shown that Crkl phosphorylation was inhibited in a dose- dependent manner when exposed to N-(2-chloro-6-methylphenyl)-2-[[6-[4-(2-hydroxyethyl)-1-piperazinyl]-2-methyl-4-pyrimidinyl]amino]-5-thiazolecarboxamide (data not shown). Thus, such a Crkl assay will allows for an assessment of the enzymatic activity of BCR-ABL protein in a reproducible, quantitative fashion and be a useful means of assessing the ability of a combination therapy or more aggressive dosing regimen of the present invention to effectively overcome imatinib resistance, to inhibit BCR-ABL activity, or to inhibit BCR-ABL mutant activity.

Briefly, cells are lysed in 1% Triton X-100 buffer with protease and phosphatase inhibitors (see, e.g. A. Goga et al., Cell 82, 981 (1995)). Equal amounts of protein, as determined by the BioRad DC protein assay (Bio-RadLaboratories, Hercules, Calif.), are separated by SDS-PAGE, transferred to nitrocellulose and immunoblotted with phosphotyrosine antibody (4G10, Upstate Biotechnologies, Lake Placid, N.Y.), Abl antibody (pex5, (see, e.g. A. Goga et al., Cell 82, 981 (1995)), β-actin antibody (Sigma Chemicals, St. Louis, Mo.) or Crkl antiserum (Santa Cruz Biotechnology, Santa Cruz, CA). Immunoreactive bands are visualized by ECL (Amersham Pharmacia Biotech, Piscataway, N.J.). Several exposures are obtained to ensure linear range of signal intensity. Optimal exposures are quantified by densitometry using ImageQuant software (Molecular Dynamics, Sunnyvale, Calif.)).

### Example 7 - Methods for Examining Amplification of the BCR-ABL Gene in Mammalian Cells

An additional method of assessing the ability of a combination therapy or more aggressive dosing regimen of the present invention to effectively overcome imatinib resistance, to inhibit BCR-ABL activity, or to inhibit BCR-ABL mutant activity is provided. Specifically, dual- color fluorescence in situ hybridization (FISH) experiments can be performed to determine if BCR-ABL gene amplification is effectively diminished. The latter is based upon the appreciation in the art that BCR-ABL amplification is observed in imatinib-resistant and protein tyrosine kinase inhibitor resistant patients. Briefly, interphase and metaphase cells are prepared (see, e.g. E. Abruzzese et al, Cancer Genet. Cytogenet. 105, 164 (1998)) and examined using Locus Specific Identifier (LSI) BCR-ABL dual color translocation probe (Lysis, Inc., Downers Grove, Ill.)). Cytogenetic and FISH characterization of metaphase spreads can be observed to assess if an inverted duplicate Ph-chromosome with interstitial amplification of the BCR-ABL fusion gene is present

Alternatively, quantitative PCR analysis of genomic DNA obtained from patients can be used to assess if BCR-ABL gene amplification is present. Briefly, genomic DNA can be extracted from purified bone marrow or peripheral blood cells with the QiaAMP Blood Mini Kit (Qiagen, Inc., Valencia, Calif.). 10 ng of total genomic DNA is subjected to real-time PCR analysis with the iCycler iQ system (Bio-Rad Laboratories, Hercules, Calif.). A 361-bp cDNA fragment including ABL exon 3 is amplified using two primers (5'-GCAGAGTCAGAATCCTTCAG-3' (SEQ ID NO:10) and 5'- TTTGTAAAAGGCTGCCCGGC- 3' (SEQ ID NO:11)) which are specific for intron sequences 5' and 3' of ABL exon 3, respectively. A 472-bp cDNA fragment of glyceraldehyde-3- phosphate dehydrogenase (GAPDH) is amplified using two primers (5'- TTCACCACCATGGAGAAGGC-3' (SEQ ID NO: 12) and 5'-CAGGAAATGAGCTTGACAAA-3' (SEQ ID NO: 13)) which are specific for sequences in exon 5 and exon 8 of GAPDH, respectively. Fold increase in ABL copy number can be determined by calculating the difference between threshold cycle numbers of ABL and GAPDH for each sample (DCt). A control can be used as a reference sample, DCt from each sample can be subtracted from DCt of control to determine D(DCt). Fold increase is then calculated as 2^{-D(DCt)}.

### Example 8 - Art Accepted Methods for Measuring Enzymological and Biological Properties of BCR-ABL Mutant.

A variety of assays for measuring the enzymological properties of protein kinases such as Abl are known in the art, for example those described in Konopka et al., Mol Cell Biol. November 1985; 5(11):3116-23; Davis et aL, Mol Cell Biol., January 1985;5(1):204-13; and Konopka et al., Cell. Jul. 1, 1984;37(3):1035-42 the contents of which are incorporated herein by reference. Using such assays the skilled artisan can measure the enzymological properties of mutant BCR-ABL protein kinases and to assess the ability of a combination therapy or more aggressive dosing regimen of the present invention to effectively overcome imatinib resistance, to inhibit BCR-ABL activity, or to inhibit BCR-ABL mutant activity.

A variety of bioassays for measuring the transforming activities of protein kinase such as Abl are know in the art, for example those described in Lugo et al., Science. Mar. 2, 1990;247(4946) :1079-82; Lugo et al., Mol Cell Biol. March1989;9(3):1263-70; Klucher et al., Blood. May 15, 1998;91(10):3927-34; Renshaw et al., Mol Cell Biol. March 1995; 15(3):1286-93; Sitard et al., Blood. Mar. 15, 1994;83(6):1575- 85; Laneuville et al., Cancer Res. Mar. 1, 1994;54(5):1360-6; Laneuville et al., Blood. Oct. 1, 1992;80(7):1788-97; Mandanas et al., Leukemia. August 1992;6(8):796-800; and Laneuville et al., Oncogene. February 1991; 6(2):275-82 the contents of which are incorporated herein by reference. Using such assays the skilled artisan can measure the phenotype of mutant BCR- Abl protein kinases.

Additional methods are disclosed in O'Hare et al. (Cancer Research, 65(11):4500-5 (2005), which is hereby incorporated by reference in its entirety.

**TABLE 1**

| **Baseline characteristics** | | | | |
|---|---|---|---|---|
| | AP-CML n=11 | MBC-CML n=23 | LBC-CML/ Ph+ ALL n=10 | Total N=44 |
| Male - n (%) | 3 (27) | 14 (61) | 9 (90) | 26 (59) |
| Female-n (%) | 8(73) | 9 (39) | 1 (10) | 18 (41) |
| Age -yrs | | | | |
| Median | 63 | 53 | 50 | 53 |
| Range | 40-73 | 30-70 | 15-73 | 15-73 |
| White cell count - cells/mm3 | | | | |
| Median | 20.6 | 20.5 | 11.6 | 19.6 |
| Range | 1.3-107.7 | 0.3-117.3 | 1.2-197.6 | 0.3-197.6 |
| Platelet count - cells/mm3 | | | | |
| Median | 279 | 39 | 40.5 | 42.5 |
| Range | 4-1710 | 7-1057 | 22-375 | 4-1710 |
| Disease history Duration of disease - months | | | | |
| Median | 67 | 44 | 26 | 46 |
| Range | 22-139 | 5-216 | 9-70 | 5-216 |
| Prior CHR with imatinib-n(%) | 8(73) | 15(65) | 9 (90) | 32 (73) |
| Prior CyR on imatinib-n(%) | 4 (36) | 5 (22) | 3 (30) | 12 (27) |
| Treatment history | | | | |
| Prior BMT - n (%) | 0 (0) | 5 (22) | 5 (50) | 10 (23) |
| Prior chemotherapy - n (%) | 4 (36) | 15 (65) | 9 (90) | 28 (64) |
| Imatinib resistant - n (%) | 9 (82) | 22 (96) | 9 (90) | 40 (91) |
| Imatinib intolerant - n (%) | 2 (18) | 1 (4) | 1(10) | 4 (9) |
| Maximum prior imatinib dose - n (%) | | | | |
| 400-600 mg/day | 4 (36) | 10(43) | 3 (30) | 17 (39) |
| >600 mg/day | 7 (64) | 13 (57) | 7 (70) | 27 (61) |
| BCR-ABL mutation status - n (%) | 8 (73) | 13 (57) | 6 (60) | 27 (61) |

**TABLE 2A**

| **On-Study Myelosuppression** | | | | | | |
|---|---|---|---|---|---|---|
| | **Grade 3** | | | **Grade 4** | | |
| | **AP** | **MBC** | **LBC/Ph+ALL** | **AP-CML** | **MBC** | **LBC/Ph+ALL** |
| | **(N=11)** | **(N=23)** | **(N=10)** | **(N=11)** | **(N=23)** | **(N=10)** |
| ANCϕ | 3 (27) | 2 (9) | 2 (20) | 6 (55) | 20 (87) | 6 (60) |
| Platelets* | 3 (27) | 2 (9) | 0 | 6(55) | 17 (74) | 7 (70) |

| | | | | | | |
|---|---|---|---|---|---|---|
| ϕ Pretreatment grade 3/4 neutropenia occurred in 18% AP, 30% MBC and 20% LBC/Ph+ ALL *Pre-treatment grade 3/4 thrombocytopenia occurred in 27% AP, 60% MBC and 70% of LBC/Ph+ ALL | | | | | | |

**TABLE 2B**

| **Significant Non-hematologic adverse events*** | | | | | | |
|---|---|---|---|---|---|---|
| | **Grade 1-4** | | | **Grade 3-4** | | |
| | **AP** | **MBC** | **LBC/ALL** | **AP** | **MBC** | **LBC/ALL** |
| | **(N=11)** | **(N=23)** | **(N=10)** | **(N=11)** | **(N=23)** | **(N=10)** |
| Diarrhea | 5 (45) | 5 (22) | 2 (20) | - | 1 (4) | - |
| Vomiting | 1 (9) | 2 (9) | 1 (10) | - | - | - |
| Nausea | 1 (9) | 4 (17) | 1 (10) | - | - | - |
| Rectal hemorrhage | - | 2 (9) | - | - | 2 (9) | - |
| Pleural effusion | - | 8 (35) | 2 (20) | - | 3(13) | - |
| Peripheral edema | 3 (27) | 5 (22) | 1 (10) | - | - | - |
| Periorbital edema | 1 (9) | 2 (9) | 1 (10) | - | - | - |
| Pericardial effusion | - | 3 (13) | - | - | 2 (9) | - |
| Generalized edema | 1 (9) | - | 1 (10) | - | - | |
| Dyspnea/Pulmonary edema | 3 (27) | 2 (9) | 1 (10) | - | 2 (9) | - |
| Rash | 5 (45) | 2 (9) | 1 (10) | - | - | - |
| Flushing | 3 (27) | 1 (4) | - | - | - | - |
| Headache | 3 (27) | 1 (4) | - | - | - | - |
| Tumor lysis syndrome | - | 2 (9) | - | - | 2 (9) | - |

| | | | | | | |
|---|---|---|---|---|---|---|
| *Laboratory Abnormalities are presented in Supplemental Table 3 | | | | | | |

**TABLE 3**

| **Treatment response** | | | | |
|---|---|---|---|---|
| | AP CML | MBC-CML | LBC-CML/ ALL | Total |
| | n=11 | n=23 | n=10 | N=44 |
| Hematologic response | | | | |
| Major HR | 9 (81) | 14 (61) | 8 (80) | 31 (70) |
| CHR | 5 (45) | 8 (35) | 7 (70) | 20 (45) |
| NEL | 4 (16) | 6 (26) | 1 (10) | 11 (25) |
| Minor HR | - | 4 (17) | - | 4 (9) |
| Cytogenetic response | | | | |
| Overall CyR | 4 (36) | 12 (52) | 9 (90) | 2 (57) |
| Major CyR | 3 (27) | 8 (35) | 8 (80) | 19 (43) |
| CCyR | 2 (18) | 6 (26) | 3 (30) | 11 (25) |
| PCyR | 1 (9) | 2 (9) | 5 (50) | 8 (18) |
| Minor CyR | - | 2 (9) | 1 (10) | 3 (7) |
| Minimal CyR | 1(9) | 2(9) | - | 3 (7) |

| | | | | |
|---|---|---|---|---|
| Major HR is defined as bone marrow blasts <5%, and has two subgroups: CHR and NEL. CHR = complete hematologic response (<5% blasts in bone marrow and return of peripheral blood to normal parameters); NEL = no evidence of leukemia (same as CHR, but without hematopoietic recovery of the peripheral blood parameters). CyR = cytogenetic response; CCyR = complete CyR (0% Ph+); PCyR = partial CyR (1-35% Ph+). Overall CyR = CCyR + PCyR + minor CyR + minimal CyR. One LBC/Ph+ALL patient had a PCyR with 3/30 Ph+ metaphases after 4 weeks dasatinib but had 91% blasts in the bone marrow. | | | | |

### SUPPLEMENTAL TABLE 1

### Hematologic Response Criteria

**I. Major Hematologic Response (Major HR)**
   **a) Complete Hematologic Response (CHR)**
      1) WBC ≤ institutional ULN
      2) ANC ≥ 1000/mm³
      3) Platelets ≥ 100,000/mm³
      4) No blasts or promyelocytes in peripheral blood
      5) Bone marrow blasts ≤ 5%
      6) < 5% myelocytes plus metamyelocytes in peripheral blood
      7) Basophils in peripheral blood < 20%
      8) No extramedullary involvement (including no hepatomegaly or splenomegaly)
   **b) No Evidence of Leukemia (NEL)**
      1) WBC ≤ institutional ULN
      2) No blasts or promyelocytes in peripheral blood
      3) Bone marrow blasts ≤ 5%
      4) < 5% myelocytes plus metamyelocytes in peripheral blood
      5) Basophils in peripheral blood < 20%
      6) No extramedullary involvement (including no hepatomegaly or splenomegaly)
      7) At least one of the following: (i) 20,000/mm³ ≤ Platelets <100,000/mm³; (ii) 500/mm³≤ ANC <1000/mm³
**II. Minor Hematologic Response (Minor HR)**
   1) < 15% blasts in bone marrow and in peripheral blood
   2) <30% blasts plus promyelocytes in bone marrow and <30% blasts plus promyelocytes in peripheral blood
   3) <20% basophils in peripheral blood
   4) No extramedullary involvement other than spleen and liver

**SUPPLEMENTAL TABLE 2**

| **Baseline Hematologic Parameters Prior to Dasatinib Treatment** | | | | | | |
|---|---|---|---|---|---|---|
| | **Grade 3** | | | **Grade 4** | | |
| | **AP** | **MBC** | **LBC/Ph+ ALL** | **AP-CML** | **MBC** | **LBC/Ph+ALL** |
| | **(N=11)** | **(N=23)** | **(N=10)** | **(N=11)** | **(N=23)** | **(N=10)** |
| ANC | 1 (9) | 3 (13) | 1 (10) | 1 (9) | 4 (17) | 1 (10) |
| Platelets | 1 (9) | 7 (30) | 5 (50) | 2 (18) | 7 (30) | 2 (20) |
| Hgb | 0 | 6 (26) | 0 | 0 | 0 | 0 |

**SUPPLEMENTAL TABLE 3**

| **Laboratory Abnormalities Regardless of Relationship to Treatment** | | | | | | |
|---|---|---|---|---|---|---|
| | **Grade 1-4** | | | **Grade 3-4** | | |
| | **AP** | **MBC** | **LBC/ALL** | **AP** | **MBC** | **LBC/ALL** |
| | **(N=11)** | **N=(23)** | **(N=10)** | **(N=11)** | **(N=23)** | **(N=10)** |
| Elevated AST | 7 (64) | 13 (57) | 9 (90) | | 2 (7) | |
| Elevated ALT | 8 (73) | 13 (57) | 7 (70) | 1 (9) | 2 (7) | |
| Elevated Total bilirubin | 2 (18) | 10 (43) | 5 (50) | | 4 (17) | |
| Elevated creatinine | 2 (18) | 12 (52) | 6 (60) | | | |
| Hypocalcemia | 8 (73) | 21 (91) | 7 (70) | 2 (18) | 6 (26) | 3 (30) |
| Hypomagnesemia | 5 (45) | 26 (70) | 3 (30) | | 1 (4) | |

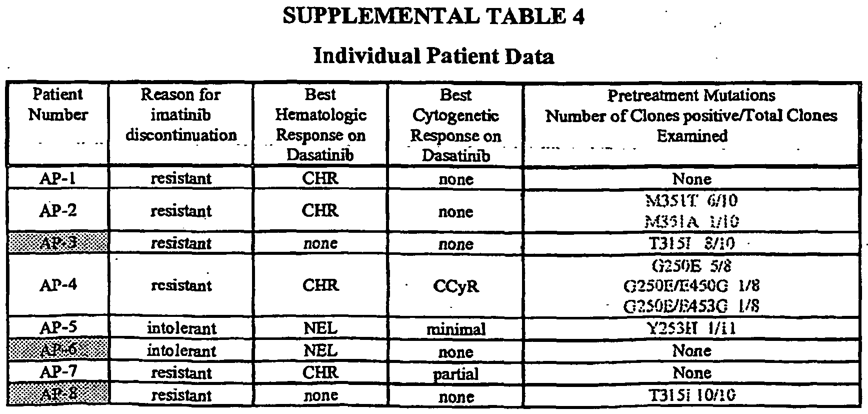

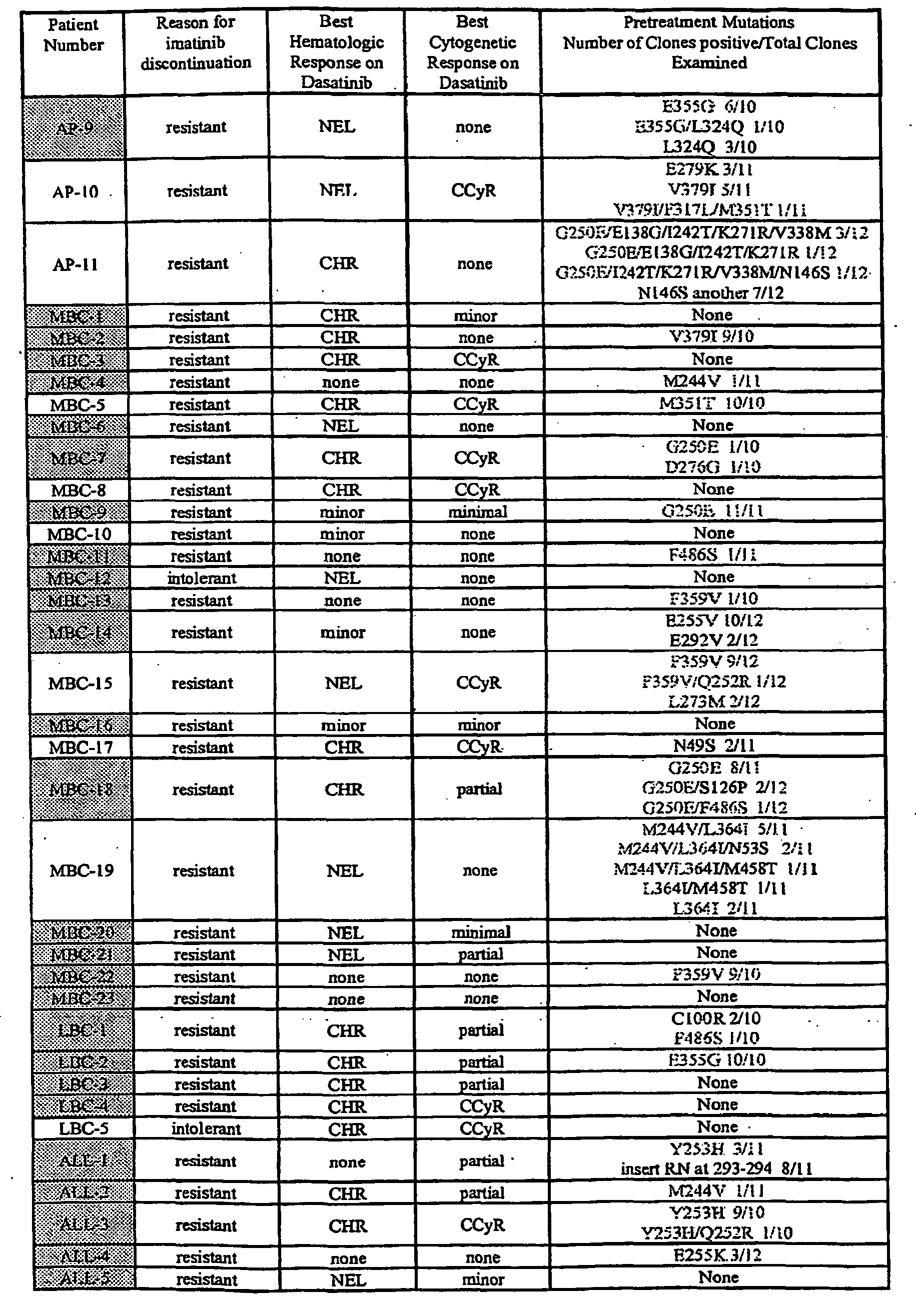

Mutations are reported if present in at least 2/10 clones in the same patient. Mutations previously reported in the literature to confer resistance to imatinib were reported if at least one clone was detected.

## Claims

1. A method for determining the responsiveness of an individual with a BCR-ABL associated disorder to treatment with N-(2-chloro-6-methylphenyl)-2-[[6-[4-(2-hydroxyethyl)-1-piperazinyl]-2-methyl-4-pyrimidinyl]amino]-5-thiazolecarboxamide, or a pharmaceutically acceptable salt, solvate or hydrate thereof, comprising:
screening a biological sample from said individual for the presence of a complex karyotype; wherein the presence of said karyotype is indicative of the individual being at least partially resistant to N-(2-chloro-6-methylphenyl)-2-[[6-[4-(2-hydroxyethyl)-1-piperazinyl]-2-methyl-4-pyrimidinyl]amino]-5-thiazolecarboxamide or a pharmaceutically acceptable salt, solvate or hydrate thereof, therapy,
wherein a complex karyotype is a karyotype that includes chromosomal abnormalities in addition to the presence of a Philadelphia chromosome.

2. A method of determining a treatment regimen for an individual suffering from a BCR-ABL-associated disorder comprising:
determining whether a biological sample obtained from the individual has a complex karyotype, wherein the presence of said karyotype is indicative of the patient being at least partially resistant to N-(2-chloro-6-methylphenyl)-2-[[6-[4-(2-hydroxyethyl)-1-piperazinyl]-2-methyl-4-pyrimidinyl] amino]-5-thiazolecarboxamide, or a pharmaceutically acceptable salt, solvate or hydrate thereof, therapy; and
recommending the administering a therapeutically effective amount of N-(2-chloro-6-methylphenyl)-2-[[6-[4-(2-hydroxyethyl)-1-piperazinyl]-2-methyl-4-pyrimidinyl] amino]-5-thiazolecarboxamide, or a pharmaceutically acceptable salt, solvate or hydrate thereof, to the individual,
wherein a complex karyotype is a karyotype that includes chromosomal abnormalities in addition to the presence of a Philadelphia chromosome.

3. The method of claim 2, wherein the thiazolecarboxamide, or a pharmaceutically acceptable salt, solvate or hydrate thereof, is administered at a dosage of greater than 70 mg twice daily if it is determined that the biological sample has a complex karyotype.

4. The method of claim 3, wherein the thiazolecarboxamide, or a pharmaceutically acceptable salt, solvate or hydrate thereof, is administered in combination with a second therapy to treat the protein tyrosine kinase associated disorder in the individual.

5. The method of claim 4, wherein the second therapy is a tubulin stabilizing agent, a farnysyl transferase inhibitor, a Rab-GGTase inhibitor, a BCR-ABL T315I inhibitor, a second protein tyrosine kinase inhibitor, or a combination thereof.

6. The use of a kit in determining a treatment strategy for an individual with a BCR-ABL-associated disorder, the kit comprising
a means for determining whether a biological sample obtained from said individual has a complex karyotype,
wherein said treatment strategy comprises administration of a therapeutically effective amount of N-(2-chloro-6-methylphenyl)-2-[[6-[4-(2-hydroxyethyl)-1-piperazinyl]-2-methyl-4-pyrimidinyl]amino]-5-thiazolecarboxamide, or a pharmaceutically acceptable salt, solvate or hydrate thereof,
wherein a complex karyotype is a karyotype that includes chromosomal abnormalities in addition to the presence of a Philadelphia chromosome.

7. A kit comprising:
a means for determining whether a biological sample obtained from said individual has a complex karyotype; and
a therapeutically effective amount of N-(2-chloro-6-methylphenyl)-2-[[6-[4-(2-hydroxyethyl)-1-piperazinyl]-2-methyl-4-pyrimidinyl]amino]-5-thiazolecarboxamide, or a pharmaceutically acceptable salt, solvate or hydrate thereof,
wherein a complex karyotype is a karyotype that includes chromosomal abnormalities in addition to the presence of a Philadelphia chromosome.

## Patentansprüche

1. Verfahren zum Bestimmen des Ansprechens eines Individuums mit einer BCR-ABL-assoziierten Erkrankung auf Behandlung mit N-(2-Chlor-6-methylphenyl)-2-[[6-[4-(2-hydroxyethyl)-1-piperazinyl]-2-methyl-4-pyrimidinyl]amino]-5-thiazolcarboxamid, oder einem pharmazeutisch verträglichen Salz, Solvat oder Hydrat davon, umfassend:
Screenen einer biologischen Probe von dem Individuum auf das Vorhandensein eines komplexen Karyotyps; wobei das Vorhandensein des Karyotyps ein Anzeichen dafür ist, dass das Individuum zumindest teilweise resistent gegen eine Therapie mit N-(2-Chlor-6-methylphenyl)-2-[[6-[4-(2-hydroxyethyl)-1-piperazinyl]-2-methyl-4-pyrimidinyl]amino]-5-thiazolcarboxamid, oder einem pharmazeutisch verträglichen Salz, Solvat oder Hydrat davon, ist,
wobei ein komplexer Karyotyp ein Karyotyp ist, der chromosomale Abnormalitäten zusätzlich zu dem Vorhandensein eines Philadelphia-Chromosoms einschließt.

2. Verfahren zum Bestimmen eines Behandlungsregimes für ein Individuum, leidend an einer BCR-ABL-assoziierten Erkrankung, umfassend:
Bestimmen, ob eine biologische Probe, erhalten von dem Individuum, einen komplexen Karyotyp hat, wobei das Vorhandensein des Karyotyps ein Anzeichen dafür ist, dass der Patient zumindest teilweise resistent gegen eine Therapie mit N-(2-Chlor-6-methylphenyl)-2-[[6-[4-(2-hydroxyethyl)-1-piperazinyl]-2-methyl-4-pyrimidinyl]amino]-5-thiazolcarboxamid, oder einem pharmazeutisch verträglichen Salz, Solvat oder Hydrat davon, ist; und
Empfehlen des Verabreichens einer therapeutisch wirksamen Menge von N-(2-Chlor-6-methylphenyl)-2-[[6-[4-(2-hydroxyethyl)-1-piperazinyl]-2-methyl-4-pyrimidinyl]amino]-5-thiazolcarboxamid, oder einem pharmazeutisch verträglichem Salz, Solvat oder Hydrat davon, an das Individuum,
wobei ein komplexer Karyotyp ein Karyotyp ist, der chromosomale Abnormalitäten zusätzlich zu dem Vorhandensein eines Philadelphia-Chromosoms einschließt.

3. Verfahren nach Anspruch 2, wobei das Thiazolcarboxamid, oder ein pharmazeutisch verträgliches Salz, Solvat oder Hydrat davon, mit einer Dosierung von mehr als 70 mg zweimal täglich verabreicht wird, wenn festgestellt wird, dass die biologische Probe einen komplexen Karyotyp hat.

4. Verfahren nach Anspruch 3, wobei das Thiazolcarboxamid, oder ein pharmazeutisch verträgliches Salz, Solvat oder Hydrat davon, in Kombination mit einer zweiten Therapie verabreicht wird, um die Protein-Tyrosin-Kinase-assoziierte Erkrankung bei dem Individuum zu behandeln.

5. Verfahren nach Anspruch 4, wobei die zweite Therapie ein Tubulin stabilisierendes Mittel, ein Farnesyl-Transferase-Inhibitor, ein Rab-GGTase-Inhibitor, ein BCR-ABL-T315I-Inhibitor, ein zweiter Protein-Tyrosin-Kinase-Inhibitor oder eine Kombination davon ist.

6. Verwendung eines Kits beim Bestimmen einer Behandlungsstrategie für ein Individuum mit einer BCR-ABL-assoziierten Erkrankung, wobei der Kit umfasst
ein Mittel zum Bestimmen, ob eine biologische Probe, erhalten von dem Individuum, einen komplexen Karyotyp hat;
wobei die Behandlungsstrategie Verabreichung einer therapeutisch wirksamen Menge von N-(2-Chlor-6-methylphenyl)-2-[[6-[4-(2-hydroxyethyl)-1-piperazinyl]-2-methyl-4-pyrimidinyl]amino]-5-thiazolcarboxamid, oder einem pharmazeutisch verträglichen Salz, Solvat oder Hydrat davon, umfasst,
wobei ein komplexer Karyotyp ein Karyotyp ist, der chromosomale Abnormalitäten zusätzlich zu dem Vorhandensein eines Philadelphia-Chromosoms einschließt.

7. Kit, umfassend:
ein Mittel zum Bestimmen, ob eine biologische Probe, erhalten von dem Individuum, einen komplexen Karyotyp hat; und
eine therapeutisch wirksame Menge von N-(2-Chlor-6-methylphenyl)-2-[[6-[4-(2-hydroxyethyl)-1-piperazinyl]-2-methyl-4-pyrimidinyl]amino]-5-thiazolcarboxamid, oder einem pharmazeutisch verträglichem Salz, Solvat oder Hydrat davon;
wobei ein komplexer Karyotyp ein Karyotyp ist, der chromosomale Abnormalitäten zusätzlich zu dem Vorhandensein eines Philadelphia-Chromosoms einschließt.

## Revendications

1. Méthode de détermination de la sensibilité d'un individu atteint d'un trouble associé à la BCR-ABL, au traitement avec du N-(2-chloro-6-méthylphényl)-2-[[6-[4-(2-hydroxyéthyl)-1-pipérazinyl]-2-méthyl-4-pyrimidinyl]amino]-5-thiazolecarboxamide ou un sel pharmaceutiquement acceptable, solvate ou hydrate de celui-ci, comprenant:
analyser un échantillon biologique dudit individu pour la présence d'un caryotype complexe, où la présence dudit caryotype est indicative du fait que l'individu est au moins partiellement résistant à une thérapie avec du N-(2-chloro-6-méthylphényl)-2-[[6-[4-(2-hydroxyéthyl)-1-pipérazinyl]-2-méthyl-4-pyrimidinyl]amino]-5-thiazolecarboxamide ou un sel pharmaceutiquement acceptable, solvate ou hydrate de celui-ci,
où un caryotype complexe est un caryotype qui comprend des anomalies chromosomiques en plus de la présence d'un chromosome de Philadelphie.

2. Méthode de détermination d'un plan de traitement pour un individu souffrant d'un trouble associé à la BCR-ABL, comprenant:
déterminer si un échantillon biologique obtenu de l'individu a un caryotype complexe, où la présence dudit caryotype est indicative du fait que le patient est au moins partiellement résistant à une thérapie avec du N-(2-chloro-6-méthylphényl)-2-[[6-[4-(2-hydroxyéthyl)-1-pipérazinyl]-2-méthyl-4-pyrimidinyl]amino]-5-thiazolecarboxamide ou un sel pharmaceutiquement acceptable, solvate ou hydrate de celui-ci; et
recommander l'administration d'une quantité thérapeutiquement efficace de N-(2-chloro-6-méthylphényl)-2-[[6-[4-(2-hydroxyéthyl)-1-pipérazinyl]-2-méthyl-4-pyrimidinyl]amino]-5-thiazolecarboxamide ou d'un sel pharmaceutiquement acceptable, solvate ou hydrate de celui-ci à l'individu,
où un caryotype complexe est un caryotype qui comprend des anomalies chromosomiques en plus de la présence d'un chromosome de Philadelphie.

3. Méthode selon la revendication 2, dans laquelle le thiazolecarboxamide ou un sel pharmaceutiquement acceptable, solvate ou hydrate de celui-ci, est administré selon une posologie supérieure à 70 mg deux fois par jour s'il est déterminé que l'échantillon biologique a un caryotype complexe.

4. Méthode selon la revendication 3, dans laquelle le thiazolecarboxamide ou un sel pharmaceutiquement acceptable, solvate ou hydrate de celui-ci, est administré en combinaison avec une deuxième thérapie pour traiter le trouble associé à une protéine tyrosine kinase chez l'individu.

5. Méthode selon la revendication 4, dans laquelle la deuxième thérapie est un agent stabilisant sur la tubuline, un inhibiteur de farnésyl transférase, un inhibiteur de Rab-GGTase, un inhibiteur de BCR-ABL T3151, un deuxième inhibiteur de protéine tyrosine kinase ou une combinaison de ceux-ci.

6. Utilisation d'un kit pour déterminer une stratégie de traitement chez un individu atteint d'un trouble associé à la BCR-ABL, le kit comprenant:
un moyen pour déterminer si un échantillon biologique obtenu dudit individu a un caryotype complexe,
où ladite stratégie de traitement comprend l'administration d'une quantité thérapeutiquement efficace de N-(2-chloro-6-méthylphényl)-2-[[6-[4-(2-hydroxyéthyl)-1-pipérazinyl]-2-méthyl-4-pyrimidinyl]amino]-5-thiazolecarboxamide ou d'un sel pharmaceutiquement acceptable, solvate ou hydrate de celui-ci,
où un caryotype complexe est un caryotype qui comprend des anomalies chromosomiques en plus de la présence d'un chromosome de Philadelphie.

7. Kit comprenant:
un moyen pour déterminer si un échantillon biologique obtenu dudit individu a un caryotype complexe; et
une quantité thérapeutiquement efficace de N-(2-chloro-6-méthylphényl)-2-[[6-[4-(2-hydroxyéthyl)-1-pipérazinyl]-2-méthyl-4-pyrimidinyl]amino]-5-thiazolecarboxamide ou d'un sel pharmaceutiquement acceptable, solvate ou hydrate de celui-ci,
où un caryotype complexe est un caryotype qui comprend des anomalies chromosomiques en plus de la présence d'un chromosome de Philadelphie.
